# EUROPÄISCHE PATENTSCHRIFT

(11) **EP 1 809 653 B1**
(45) Veröffentlichungstag und Bekanntmachung des Hinweises auf die Patenterteilung: **20.01.2010**
(21) Anmeldenummer: 05790976.4
(22) Anmeldetag: 08.10.2005
(51) Int. Cl.: C07K 7/06, A61K 38/08, A61P 31/04

(54) **SUBSTITUIERTE NONADEPSIPEPTIDE**
SUBSTITUTED NONADEPSIPEPTIDES
NONADEPSIPEPTIDES SUBSTITUES

(30) Priorität: 20.10.2004 DE 102004051025
(43) Veröffentlichungstag der Anmeldung: 25.07.2007
(73) Patentinhaber: AiCuris GmbH & Co. KG, 42117 Wuppertal (DE)
(72) Erfinder: VON NUSSBAUM, Franz, 40219 Düsseldorf (DE); BRUNNER, Nina, 45279 Essen (DE); ENDERMANN, Rainer, 42113 Wuppertal (DE); FÜRSTNER, Chantal, 45478 Mühlheim/Ruhr (DE); HARTMANN, Elke, 42111 Wuppertal (DE); RAGOT, Jacques, 40625 Düsseldorf (DE); SCHIFFER, Guido, 42111 Wuppertal (DE); SCHUHMACHER, Joachim, 42113 Wuppertal (DE); SVENSTRUP, Niels, 42553 Velbert (DE); TELSER, Joachim, 51061 Köln (DE); ANLAUF, Sonja, 42115 Wuppertal (DE); BRÜNING, Michael-Alexander, 13469 Berlin (DE)
(74) Vertreter: Weller, Wolfgang
(86) Internationale Anmeldenummer: PCT/EP2005/010857
(87) Internationale Veröffentlichungsnummer: WO 2006/042654

(56) Entgegenhaltungen:
- WO-A-2004/099239
- TYMIAK A A ET AL: "STRUCTURE DETERMINATION OF LYSOBACTIN A MACROCYCLIC PEPTIDE LACTONE ANTIBIOTIC" JOURNAL OF ORGANIC CHEMISTRY, AMERICAN CHEMICAL SOCIETY. EASTON, US, Bd. 54, Nr. 5, 1989, Seiten 1149-1157, XP002289533 ISSN: 0022-3263 in der Anmeldung erwähnt
- EGNER B J ET AL: "Monitoring the solid phase synthesis of analogues of lysobactin and the katanosins using in situ MALDI-TOF MS" TETRAHEDRON, ELSEVIER SCIENCE PUBLISHERS, AMSTERDAM, NL, Bd. 53, Nr. 41, 13. Oktober 1997 (1997-10-13), Seiten 14021-14030, XP004618631 ISSN: 0040-4020

## Beschreibung

Die Erfindung betrifft Nonadepsipeptide der Formel (I) und Verfahren zu ihrer Herstellung sowie ihre Verwendung zur Herstellung von Arzneimitteln zur Behandlung und/oder Prophylaxe von Krankheiten, insbesondere bakteriellen Infektionskrankheiten.

Die bakterielle Zellwand wird durch eine Reihe von Enzymen synthetisiert (Zellwandbiosynthese) und ist essentiell für das Überleben bzw. die Vermehrung von Mikroorganismen. Die Struktur dieses Makromoleküls ebenso wie die an ihrer Synthese beteiligten Proteine sind innerhalb der Bakterien stark konserviert. Aufgrund ihrer essentiellen Natur und Einheitlichkeit ist die Zellwandbiosynthese ein idealer Angriffspunkt für neue Antibiotika (D.W.Green, The bacterial cell wall as a source of antibacterial targets, Expert Opin. Ther. Targets, 2002, 6, 1-19).

Vancomycin und Penicilline sind Inhibitoren der bakteriellen Zellwandbiosynthese und stellen erfolgreiche Beispiele für die antibiotische Potenz dieses Wirkprinzips dar. Sie werden seit mehreren Jahrzehnten in der Klinik zur Behandlung von bakteriellen Infektionen, vor allem mit Grampositiven Erregern eingesetzt. Durch das wachsende Auftreten von resistenten Keimen, z.B. Methicillin-resistenten Staphylokokken, Penicillin-resistenten Pneumokokken und Vancomycinresistenten Enterokokken (F. Baquero, Gram-positive resistance: challenge for the development of new antibiotics, J. Antimicrob. Chemother., 1997, 39, Suppl A: 1-6; A.P. Johnson, D.M. Livermore, G.S. Tillotson, Antimicrobial susceptibility of Gram-positive bacteria: what's current, what's anticipated ?, J. Hosp. Infect., 2001, (49), Suppl A: 3-11) sowie jüngst auch erstmals Vancomycinresistenten Staphylokokken (B. Goldrick, First reported case of VRSA in the United States, Am. J. Nurs., 2002, 102, 17) verlieren diese Substanzen zunehmend ihre therapeutische Wirksamkeit.

Die vorliegende Erfindung beschreibt eine neue Klasse von Zellwandbiosynthese-Inhibitoren ohne Kreuzresistenzen zu bekannten Antibiotika-Klassen.

Der Naturstoff Lysobactin und einige Derivate sind als antibakteriell wirksam beschrieben in US 4,754,018. Die Isolierung und antibakterielle Wirksamkeit von Lysobactin ist auch in EP-A-196 042 und JP 01132600 beschrieben. WO04/099239 beschreibt Derivate des Lysobactins mit antibakterieller Wirksamkeit.

Die antibakterielle Wirkung von Lysobactin und Katanosin A wird weiterhin beschrieben in O'Sullivan, J. et al., J. Antibiot. 1988, 41, 1740 - 1744*,* Bonner, D. P. et al., J. Antibiot. 1988, 41, 1745 - 1751, Shoji, J. et al., J. Antibiot. 1988, 41, 713 - 718 und Tymiak, A. A. et al., J. Org. Chem. 1989, 54, 1149 - 1157.

Eine Aufgabe der vorliegenden Erfindung ist es, alternative Verbindungen mit vergleichbarer oder verbesserter antibakterieller Wirkung, besserer Löslichkeit und besserer Verträglichkeit, z.B. geringerer Nephrotoxizität, zur Behandlung von bakteriellen Erkrankungen bei Menschen und Tieren zur Verfügung zu stellen.

Gegenstand der Erfindung sind Verbindungen der Formel 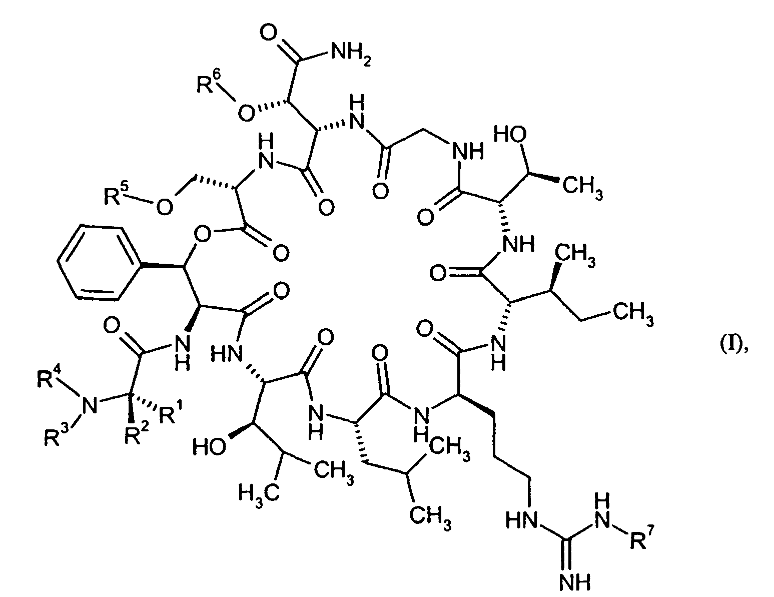 in welcher
- R¹: Wasserstoff, C₁-C₆-Alkyl, C₂-C₆-Alkenyl, C₃-C₆-Cycloalkyl oder C₆-C₁₀-Aryl bedeutet,
wobei Alkyl, Alkenyl, Cycloalkyl und Aryl substituiert sein können mit 0, 1, 2 oder 3 Substituenten unabhängig voneinander ausgewählt aus der Gruppe bestehend aus Halogen, Hydroxy, Amino, Cyano, Trimethylsilyl, C₁-C₆-Alkyl, C₁-C₆-Alkoxy, Benzyloxy, C₃-C₆-Cycloalkyl, C₆-C₁₀-Aryl, 5- bis 7-gliedriges Heterocyclyl, 5- bis 10-gliedriges Heteroaryl, C₁-C₆-Alkylamino, C₆-C₁₀-Arylamino, C₁-C₆-Alkylcarbonylamino, C₆-C₁₀-Arylcarbonylamino, C₁-C₆-Alkylcarbonyl, C₁-C₆-Alkoxycarbonyl, C₆-C₁₀-Arylcarbonyl und Benzyloxycarbonylamino,
worin Cycloalkyl, Aryl, Heterocyclyl und Heteroaryl ihrerseits substituiert sein können mit 0, 1, 2 oder 3 Substituenten unabhängig voneinander ausgewählt aus der Gruppe bestehend aus Halogen, Hydroxy, Amino, Cyano, Nitro, Trifluormethyl; C₁-C₆-Alkyl, C₁-C₆-Alkoxy, Phenyl und 5- bis 7-gliedriges Heterocyclyl,
- R²: Wasserstoff oder C₁-C₄-Alkyl bedeutet,
- R³: C₁-C₆-Alkyl, C₃-C₆-Cycloalkyl, 5- bis 7-gliedriges Heterocyclyl, C₆-C₁₀-Aryl, 5- oder 6-gliedriges Heteroaryl, C₁-C₆-Alkylcarbonyl, C₁-C₆-Alkoxycarbonyl, C₃-C₆-Cycloalkylcarbonyl, 5- bis 7-gliedriges Heterocyclylcarbonyl, C₆-C₁₀-Arylcarbonyl, 5- oder 6-gliedriges Heteroarylcarbonyl oder C₁-C₆-Alkylaminocarbonyl bedeutet,
wobei Alkyl, Cycloalkyl, Heterocyclyl, Aryl, Heteroaryl, Alkoxycarbonyl, Cycloalkylcarbonyl, Heterocyclylcarbonyl, Arylcarbonyl, Heteroarylcarbonyl und Alkylaminocarbonyl substituiert sein können mit 0, 1, 2 oder 3 Substituenten unabhängig voneinander ausgewählt aus der Gruppe bestehend aus Halogen, Hydroxy, Amino, C₁-C₆-Alkylamino und Phenyl,
und
wobei Alkylcarbonyl substituiert ist mit einem Substituenten Amino oder C₁-C₆-Alkylamino,
und
wobei Alkylcarbonyl substituiert sein kann mit weiteren 0, 1 oder 2 Substituenten unabhängig voneinander ausgewählt aus der Gruppe bestehend aus Halogen, Hydroxy, Trimethylsilyl, C₁-C₆-Alkoxy, C₁-C₆-Alkylthio, Benzyloxy, C₁-C₆-Cycloalkyl, Phenyl, Naphthyl, 5- bis 10-gliedriges Heteroaryl, C₁-C₆-Alkylcarbonylamino, C₁-C₆-Alkoxycarbonylamino, C₆-C₁₀-Arylcarbonylamino, C₆-C₁₀-Arylcarbonyloxy, Benzyloxycarbonyl und Benzyloxycarbonylamino,
worin Phenyl und Heteroaryl ihrerseits substituiert sein können mit 0, 1, 2 oder 3 Substituenten unabhängig voneinander ausgewählt aus der Gruppe bestehend aus Halogen, Hydroxy, Nitro, C₁-C₆-Alkyl, C₁-C₆-Alkoxy und Phenyl,
- R⁴: Wasserstoff, C₁-C₄-Alkyl, Cyclopropyl oder Cyclopropylmethyl bedeutet,
und
- R⁵: C₆-C₁₀-Arylaminocarbonyl, C₆-C₁₀-Arylcarbonyl, C₆-C₁₀-Arylaminothiocarbonyl, C₆-C₁₀-Arylthiocarbonyl, C₆-C₁₀-Arylsulfonylaminocarbonyl, 5- bis 10-gliedriges Heteroarylaminocarbonyl, 5- bis 10-gliedriges Heteroarylcarbonyl, 5- bis 10-gliedriges Heteroarylaminothiocarbonyl oder 5- bis 10-gliedriges Heteroarylthiocarbonyl bedeutet,
wobei Arylaminocarbonyl, Arylcarbonyl, Arylaminothiocarbonyl, Arylthiocarbonyl, Arylsulfonylaminocarbonyl, Heteroarylaminocarbonyl, Heteroarylcarbonyl, Heteroaryl-aminothiocarbonyl und Heteroarylthiocarbonyl substituiert sein können mit 0, 1, 2 oder 3 Substituenten unabhängig voneinander ausgewählt aus der Gruppe bestehend aus Halogen, C₁-C₆-Alkyl, C₁-C₆-Alkoxy, C₁-C₆-Alkylamino, C₁-C₆-Alkoxycarbonyl, C₁-C₆-Alkylaminocarbonyl, C₁-C₆-Alkylaminosulfonyl, gegebenenfalls Oxo substituiertes 5- bis 7-gliedriges Heterocyclyl und 5- bis 10-gliedriges Heteroaryl,
- R⁶: Wasserstoff bedeutet,
- R⁷: Wasserstoff bedeutet,
oder
- R⁵: Wasserstoff bedeutet,
- R⁶: C₆-C₁₀-Arylaminocarbonyl, C₆-C₁₀-Arylcarbonyl, C₆-C₁₀-Arylaminothiocarbonyl, C₆-C₁₀-Arylthiocarbonyl, C₆-C₁₀-Arylsulfonylaminocarbonyl, 5- bis 10-gliedriges Heteroarylaminocarbonyl, 5- bis 10-gliedriges Heteroarylcarbonyl, 5- bis 10-gliedriges Heteroarylaminothiocarbonyl oder 5- bis 10-gliedriges Heteroarylthiocarbonyl bedeutet,
wobei Arylaminocarbonyl, Arylcarbonyl, Arylaminothiocarbonyl, Arylthiocarbonyl, Arylsulfonylaminocarbonyl, Heteroarylaminocarbonyl, Heteroarylcarbonyl, Heteroarylaminothiocarbonyl und Heteroarylthiocarbonyl substituiert sein können mit 0, 1, 2 oder 3 Substituenten unabhängig voneinander ausgewählt aus der Gruppe bestehend aus Halogen, C₁-C₆-Alkyl, C₁-C₆-Alkoxy, C₁-C₆-Alkylamino, C₁-C₆-Alkoxycarbonyl, C₁-C₆-Alkylaminocarbonyl, C₁-C₆-Alkylaminosulfonyl, gegebenenfalls Oxo substituiertes 5- bis 7-gliedriges Heterocyclyl und 5- bis 10-gliedriges Heteroaryl,
- R⁷: Wasserstoff bedeutet,
oder
- R⁵: Wasserstoff bedeutet,
- R⁶: Wasserstoff bedeutet,
- R⁷: C₆-C₁₀-Arylaminocarbonyl, C₆-C₁₀-Arylcarbonyl, C₆-C₁₀-Arylaminothiocarbonyl, C₆-C₁₀-Arylthiocarbonyl, 5- bis 10-gliedriges Heteroarylaminocarbonyl, 5- bis 10-gliedriges Heteroarylcarbonyl, 5- bis 10-gliedriges Heteroarylaminothiocarbonyl oder 5- bis 10-gliedriges Heteroarylthiocarbonyl bedeutet,
wobei Arylaminocarbonyl, Arylcarbonyl, Arylaminothiocarbonyl, Arylthiocarbonyl, Heteroarylaminocarbonyl, Heteroarylcarbonyl, Heteroarylaminothiocarbonyl und Heteroarylthiocarbonyl substituiert sein können mit 0, 1, 2 oder 3 Substituenten unabhängig voneinander ausgewählt aus der Gruppe bestehend aus Halogen, C₁-C₆-Alkyl, C₁-C₆-Alkoxy, C₁-C₆-Alkylamino, C₁-C₆-Alkoxycarbonyl, C₁-C₆-Alkylaminocarbonyl, C₁-C₆-Alkylaminosulfonyl, gegebenenfalls Oxo substituiertes 5- bis 7-gliedriges Heterocyclyl und 5- bis 10-gliedriges Heteroaryl,
oder
- R⁵ und R⁶: gleich sind, und
C₆-C₁₀-Arylaminocarbonyl, C₆-C₁₀-Arylcarbonyl, C₆-C₁₀-Arylaminothiocarbonyl, C₆-C₁₀-Arylthiocarbonyl, C₆-C₁₀-Arylsulfonylaminocarbonyl, 5- bis 10-gliedriges Heteroarylaminocarbonyl, 5- bis 10-gliedriges Heteroarylcarbonyl, 5- bis 10-gliedriges Heteroarylaminothiocarbonyl oder 5- bis 10-gliedriges Heteroarylthiocarbonyl bedeuten,
wobei Arylaminocarbonyl, Arylcarbonyl, Arylaminothiocarbonyl, Arylthiocarbonyl, Arylsulfonylaminocarbonyl, Heteroarylaminocarbonyl, Heteroarylcarbonyl, Heteroaryl-aminothiocarbonyl und Heteroarylthiocarbonyl substituiert sein können mit 0, 1, 2 oder 3 Substituenten unabhängig voneinander ausgewählt aus der Gruppe bestehend aus Halogen, C₁-C₆-Alkyl, C₁-C₆-Alkoxy, C₁-C₆-Alkylamino, C₁-C₆-Alkoxycarbonyl, C₁-C₆-Alkylaminocarbonyl, C₁-C₆-Alkylaminosulfonyl, gegebenenfalls Oxo substituiertes 5- bis 7-gliedriges Heterocyclyl und 5- bis 10-gliedriges Heteroaryl,
- R⁷: Wasserstoff bedeutet,
und ihre Salze, ihre Solvate und die Solvate ihrer Salze.

Erfindungsgemäße Verbindungen sind die Verbindungen der Formel (I) und deren Salze, Solvate, Solvate der Salze und Prodrugs, die von Formel (I) umfassten Verbindungen der nachfolgend genannten Formeln und deren Salze, Solvate, Solvate der Salze und Prodrugs sowie die von Formel (I) umfassten, nachfolgend als Ausführungsbeispiele genannten Verbindungen und deren Salze, Solvate, Solvate der Salze und Prodrugs, soweit es sich bei den von Formel (I) umfassten, nachfolgend genannten Verbindungen nicht bereits um Salze, Solvate, Solvate der Salze und Prodrugs handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegenden Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methylmorpholin, Arginin, Lysin, Ethylendiamin und *N*-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:
Alkyl per se und "Alk" und "Alkyl" in Alkoxy Alkylamino, Alkylthio, Alkylcarbonyl, Alkoxycarbonyl, Alkylaminocarbonyl, Alkylaminosulfonyl, Alkylcarbonylamino und Alkoxycarbonylamino steht für einen linearen oder verzweigten Alkylrest mit in der Regel 1 bis 6, vorzugsweise 1 bis 4, besonders bevorzugt 1 bis 3 Kohlenstoffatomen, beispielhaft und vorzugsweise für Methyl, Ethyl, n-Propyl, Isopropyl, *tert*-Butyl, 2,2-Dimethylprop-1-yl, n-Pentyl und n-Hexyl.
Alkoxγ steht beispielhaft und vorzugsweise für Methoxy, Ethoxy, n-Propoxy, Isopropoxy, *tert-*Butoxy, n-Pentoxy und n-Hexoxy.
Alkylthio steht beispielhaft und vorzugsweise für Methylthio, Ethylthio, n-Propylthio, Isopropylthio, *tert*-Butylthio, n-Pentylthio und n-Hexylthio.
Alkenyl steht für einen geradkettigen oder verzweigten Alkenylrest mit 2 bis 6 Kohlenstoffatomen. Bevorzugt ist ein geradkettiger oder verzweigter Alkenylrest mit 2 bis 4, besonders bevorzugt mit 2 bis 3 Kohlenstoffatomen. Beispielsweise und vorzugsweise seien genannt: Vinyl, Allyl, n-Prop-1-en-1-yl, n-But-2-en-1-yl, 2-Methylprop-1-en-1-yl und 2-Methylprop-2-en-1-yl.
Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, Isopropylamino, *tert*-Butylamino, n-Pentylamino, n-Hexylamino, *N*,*N*-Dimethylamino, *N,N-*Diethylamino, *N*-Ethyl-*N*-methylamino, *N*-Methyl-*N*-n-propylamino, *N*-Isopropyl-*N*-n-propylamino, *N*-*tert*-Butyl-*N*-methylamino, *N*-Ethyl-*N*-n-pentylamino und *N*-n-Hexyl-*N*-methylamino. C₁-C₃-Alkylamino steht beispielsweise für einen Monoalkylaminorest mit 1 bis 3 Kohlenstoffatomen oder für einen Dialkylaminorest mit jeweils 1 bis 3 Kohlenstoffatomen pro Alkylsubstituent.
Arylamino steht für einen über eine Aminogruppe gebundenen Arylsubstituenten, wobei an die Aminogruppe gegebenenfalls ein weiterer Substituenten, wie z.B. Aryl oder Alkyl, gebunden ist, beispielhaft und vorzugsweise für Phenylamino, Naphthylamino, Phenylmethylamino oder Diphenylamino.
Alkylcarbonyl steht beispielhaft und vorzugsweise für Methylcarbonyl, Ethylcarbonyl, n-Propylcarbonyl, Isopropylcarbonyl, *tert*-Butylcarbonyl, n-Pentylcarbonyl und n-Hexylcarbonyl.
Alkoxycarbonyl steht beispielhaft und vorzugsweise für Methoxycarbonyl, Ethoxycarbonyl, n-Propoxycarbonyl, Isopropoxycarbonyl, *tert*-Butoxycarbonyl, n-Pentoxycarbonyl und n-Hexoxycarbonyl.
Alkoxycarbonylamino steht beispielhaft und vorzugsweise für Methoxycarbonylamino, Ethoxycarbonylamino, n-Propoxy-carbonylamino, Isopropoxycarbonylamino, *tert*-Butoxycarbonyl-amino, n-Pentoxycarbonylamino und n-Hexoxycarbonylamino.
Cycloalkycarbonyl steht für einen über eine Carbonylgruppe gebundenen Cycloalkylsubstituenten, beispielhaft und vorzugsweise für Cyclopropylcarbonyl, Cyclobutylcarbonyl, Cyclopentylcarbonyl und Cyclohexylcarbonyl.
Heterocyclylcarbonyl steht für einen über eine Carbonylgruppe gebundenen Heterocyclylsubstituenten, beispielhaft und vorzugsweise für Tetrahydrofuranylcarbonyl, Pyrrolidinylcarbonyl, Pyrrolinylcarbonyl, Piperidinylcarbonyl, Tetrahydropyranylcarbonyl, Piperazinylcarbonyl, Morpholinylcarbonyl und Perhydroazepinylcarbonyl.
Arylcarbonyl steht für einen über eine Carbonylgruppe gebundenen Arylsubstituenten, beispielhaft und vorzugsweise für Phenylcarbonyl, Naphthylcarbonyl und Phenanthrenylcarbonyl.
Heteroarylcarbonyl steht für einen über eine Carbonylgruppe gebundenen Heteroarylsubstituenten, beispielhaft und vorzugsweise für Thienylcarbonyl, Furylcarbonyl, Pyrrolylcarbonyl, Thiazolylcarbonyl, Oxazolylcarbonyl, Imidazolylcarbonyl, Pyridylcarbonyl, Pyrimidylcarbonyl, Pyridazinylcarbonyl, Indolylcarbonyl, Indazolylcarbonyl, Benzofuranylcarbonyl, Benzothiophenyl-carbonyl, Chinolinylcarbonyl und Isochinolinylcarbonyl.
Alkylcarbonylamino steht beispielhaft und vorzugsweise für Methylcarbonylamino, Ethylcarbonylamino, n-Propylcarbonylamino, Isopropylcarbonylamino, *tert*-Butylcarbonylamino, n-Pentylcarbonylamino und n-Hexylcarbonylamino.
Arylcarbonylamino steht beispielhaft und vorzugsweise für Phenylcarbonylamino, Naphthylcarbonylamino und Phenanthrenylcarbonylamino.
Arylcarbonyloxy steht beispielhaft und vorzugsweise für Phenylcarbonyloxy, Naphthylcarbonyloxy und Phenanthrenylcarbonyloxy.
Alkylaminocarbonyl steht für einen Alkylaminocarbonylrest mit einem oder zwei (unabhängig voneinander gewählten) Alkylsubstituenten, beispielhaft und vorzugsweise für Methylaminocarbonyl, Ethylaminocarbonyl, n-Propylaminocarbonyl, Isopropylaminocarbonyl, *tert*-Butylaminocarbonyl, n-Pentylaminocarbonyl, n-Hexylaminocarbonyl, *N*,*N*-Dimethylaminocarbonyl, *N*,*N*-Diethylaminocarbonyl, *N*-Ethyl-*N*-methylaminocarbonyl, *N*-Methyl-*N*-n-propylaminocarbonyl, *N*-Isopropyl-*N*-n-propylaminocarbonyl, *N*-*tert*-Butyl-*N*-methylaminocarbonyl, *N*-Ethyl-*N*-n-pentylamino-carbonyl und *N*-n-Hexyl-*N*-methylaminocarbonyl. C₁-C₃-Alkylaminocarbonyl steht beispielsweise für einen Monoalkylaminocarbonylrest mit 1 bis 3 Kohlenstoffatomen oder für einen Dialkylaminocarbonylrest mit jeweils 1 bis 3 Kohlenstoffatomen pro Alkylsubstituent.
Alkylaminosulfonyl steht für einen Alkylaminosulfonylrest mit einem oder zwei (unabhängig voneinander gewählten) Alkylsubstituenten, beispielhaft und vorzugsweise für Methylaminosulfonyl, Ethylaminosulfonyl, n-Propylaminosulfonyl, Isopropylaminosulfonyl, *tert*-Butylaminosulfonyl, n-Pentylaminosulfonyl, n-Hexylaminosulfonyl, *N*,*N*-Dimethylaminosulfonyl, *N*,*N*-Diethylaminosulfonyl, *N*-Ethyl-*N*-methylaminosulfonyl, *N*-Methyl-*N*-n-propylaminosulfonyl, *N*-Isopropyl-*N*-n-propylaminosulfonyl, *N*-*tert*-Butyl-*N*-methylaminosulfonyl, *N*-Ethyl-*N*-n-pentylaminosulfonyl und *N-*n-Hexyl-*N*-methylaminosulfonyl. C₁-C₃-Alkylaminosulfonyl steht beispielsweise für einen Monoalkylaminosulfonylrest mit 1 bis 3 Kohlenstoffatomen oder für einen Dialkylaminosulfonylrest mit jeweils 1 bis 3 Kohlenstoffatomen pro Alkylsubstituent.
Arylaminocarbonyl steht für einen über eine Aminocarbonylgruppe gebundenen Arylsubstituenten, wobei an die Aminocarbonylgruppe gegebenenfalls ein weiterer Substituenten, wie z.B. Aryl oder Alkyl, gebunden ist, beispielhaft und vorzugsweise für Phenylaminocarbonyl, Naphthylaminocarbonyl, Phenylmethylaminocarbonyl oder Diphenylaminocarbonyl.
Arylaminothiocarbonyl steht für einen über eine Aminothiocarbonylgruppe gebundenen Arylsubstituenten, wobei an die Aminothiocarbonylgruppe gegebenenfalls ein weiterer Substituenten, wie z.B. Aryl oder Alkyl, gebunden ist, beispielhaft und vorzugsweise für Phenylaminothiocarbonyl, Naphthylaminothiocarbonyl, Phenylmethylaminothiocarbonyl oder Diphenylaminothiocarbonyl.
Arylthiocarbonyl steht beispielhaft und vorzugsweise für Phenylthiocarbonyl, Naphthylthiocarbonyl und Phenanthrenylthiocarbonyl.
Arylsulfonylaminocarbonyl steht beispielhaft und vorzugsweise für Phenylsulfonylaminocarbonyl, Naphthylsulfonylaminocarbonyl und Phenanthrenylsulfonylaminocarbonyl.
Heteroarylaminocarbonyl steht für einen über eine Aminocarbonylgruppe gebundenen Heteroarylsubstituenten, wobei an die Aminocarbonylgruppe gegebenenfalls ein weiterer Substituenten, wie z.B. Aryl oder Alkyl, gebunden ist, beispielhaft und vorzugsweise für Thienylaminocarbonyl, Furylaminocarbonyl, Pyrrolylaminocarbonyl, Thiazolylaminocarbonyl, Oxazolylaminocarbonyl, Imidazolylaminocarbonyl, Pyridylaminocarbonyl, Pyrimidylamino-carbonyl, Pyridazinylaminocarbonyl, Indolylaminocarbonyl, Indazolylaminocarbonyl, Benzofuranylaminocarbonyl, Benzothiophenylaminocarbonyl, Chinolinylaminocarbonyl, Isochinolinylaminocarbonyl, Furylmethylaminocarbonyl und Pyridylmethylaminocarbonyl.
Heteroarylaminothiocarbonyl steht für einen über eine Aminothiocarbonylgruppe gebundenen Heteroarylsubstituenten, wobei an die Aminothiocarbonylgruppe gegebenenfalls ein weiterer Substituenten, wie z.B. Aryl oder Alkyl, gebunden ist, beispielhaft und vorzugsweise für Thienylaminothiocarbonyl, Furylaminothiocarbonyl, Pyrrolylaminothiocarbonyl, Thiazolylamino-thiocarbonyl, Oxazolylaminothiocarbonyl, Imidazolylaminothiocarbonyl, Pyridylaminothiocarbonyl, Pyrimidylaminothiocarbonyl, Pyridazinylaminothiocarbonyl, Indolylaminothiocarbonyl, Indazolylaminothiocarbonyl, Benzofuranylaminothiocarbonyl, Benzothiophenylaminothiocarbonyl, Chinolinylaminothiocarbonyl, Isochinolinylaminothiocarbonyl, Furylmethylaminothiocarbonyl und Pyridylmethylaminothiocarbonyl.
Heteroarylthiocarbonyl steht für einen über eine Thiocarbonylgruppe gebundenen Heteroarylsubstituenten, beispielhaft und vorzugsweise für Thienylthiocarbonyl, Furylthiocarbonyl, Pyrrolylthiocarbonyl, Thiazolylthiocarbonyl, Oxazolylthiocarbonyl, Imidazolylthiocarbonyl, Pyridylthiocarbonyl, Pyrimidylthiocarbonyl, Pyridazinylthiocarbonyl, Indolylthiocarbonyl, Indazolylthiocarbonyl, Benzofuranylthiocarbonyl, Benzothiophenylthiocarbonyl, Chinolinylthio-carbonyl und Isochinolinylthiocarbonyl.
Cycloalkyl steht für eine Cycloalkylgruppe mit in der Regel 3 bis 6 Kohlenstoffatomen, beispielhaft und vorzugsweise für Cyclopropyl, Cyclobutyl, Cyclopentyl und Cyclohexyl.
Aryl steht für einen mono- bis tricyclischen aromatischen, carbocyclischen Rest mit in der Regel 6 bis 14 Kohlenstoffatomen; beispielhaft und vorzugsweise für Phenyl, Naphthyl und Phenanthrenyl.
Heterocyclyl steht für einen mono- oder polycyclischen, vorzugsweise mono- oder bicyclischen, heterocyclischen Rest mit in der Regel 5 bis 7 Ringatomen und bis zu 3, vorzugsweise bis zu 2 Heteroatomen und/oder Heterogruppen aus der Reihe N, O, S, SO, SO₂. Die Heterocyclyl-Reste können gesättigt oder teilweise ungesättigt sein. Bevorzugt sind 5- bis 7-gliedrige, monocyclische gesättigte Heterocyclylreste mit bis zu zwei Heteroatomen aus der Reihe O, N und S, wie beispielhaft und vorzugsweise Tetrahydrofuran-2-yl, Tetrahydrofuran-3-yl, Pyrrolidin-2-yl, Pyrrolidin-3-yl, Pyrrolinyl, Piperidin-1-yl, Piperidin-2-yl, Piperidin-3-yl, Piperidin-4-yl, Tetrahydropyran-2-yl, Tetrahydropyran-3-yl, Tetrahydropyran-4-yl, Piperazin-1-yl, Piperazin-2-yl, Morpholin-2-yl, Morpholin-3-yl, Morpholin-4-yl, Thiomorpholin-2-yl, Thiomorpholin-3-yl, Thiomorpholin-4-yl und Perhydroazepinyl.
Heteroaryl steht für einen aromatischen, mono- oder bicyclischen Rest mit in der Regel 5 bis 10, vorzugsweise 5 bis 6 Ringatomen und bis zu 5, vorzugsweise bis zu 4 Heteroatomen aus der Reihe S, O und N, beispielhaft und vorzugsweise für Thien-2-yl, Thien-3-yl, Fur-2-yl, Fur-3-yl, Pynol-1-yl, Pyrrol-2-yl, Pyrrol-3-yl, Thiazol-2-yl, Thiazol-4-yl, Thiazol-5-yl, Oxazol-2-yl, Oxazol-4-yl, Oxazol-5-yl, Imidazol-1-yl, Imidazol-2-yl, Imidazol-4-yl, Imidazol-5-yl, Pyrid-2-yl, Pyrid-3-yl, Pyrid-4-yl, Pyrimid-2-yl, Pynmid-4-yl, Pyrimid-5-yl, Pyrazin-2-yl, Pyrazin-3-yl, Pyridazin-3-yl, Pyridazin-4-yl, Indolyl, Indazolyl, Benzofuranyl, Benzothiophenyl, Chinolinyl und Isochinolinyl.
Halogen steht für Fluor, Chlor, Brom und Jod, bevorzugt Fluor und Chlor.

Bevorzugt sind Verbindungen der Formel (I), in welcher
- R¹: 2-Methylprop-1-yl, 2,2-Dimethylprop-1-yl, 2-Pyridylmethyl oder 3-Pyridylmethyl bedeutet,
wobei 2-Pyridylmethyl oder 3-Pyridylmethyl substituiert sein können mit 0, 1, 2 oder 3 Substituenten unabhängig voneinander ausgewählt aus der Gruppe bestehend aus Hydroxy, Amino, Trifluormethyl, Methyl, Methoxy und Morpholinyl,
- R²: Wasserstoff bedeutet,
- R³: 1-Amino-3-methylbut-1-ylcarbonyl, 1-Amino-3,3-dimethylbut-1-ylcarbonyl oder 1-Amino-2-trimethylsilyleth-1-ylcarbonyl bedeutet,
- R⁴: Wasserstoff bedeutet,
und
- R⁵: C₆-C₁₀-Arylaminocarbonyl, C₆-C₁₀-Arylcarbonyl, C₆-C₁₀-Arylaminothlocarbonyl, C₆-C₁₀-Arylthiocarbonyl, C₆-C₁₀-Arylsulfonylaminocarbonyl, 5- bis 10-gliedriges Heteroarylaminocarbonyl, 5- bis 10-gliedriges Heteroarylcarbonyl, 5- bis 10-gliedriges Heteroarylaminothiocarbonyl oder 5- bis 10-gliedriges Heteroarylthiocarbonyl bedeutet,
wobei Arylaminocarbonyl, Arylcarbonyl, Arylaminothiocarbonyl, Arylthiocarbonyl, Arylsulfonylaminocarbonyl, Heteroarylaminocarbonyl, Heteroarylcarbonyl, Heteroarylaminothiocarbonyl und Heteroarylthiocarbonyl substituiert sein können mit 0, 1, 2 oder 3 Substituenten unabhängig voneinander ausgewählt aus der Gruppe bestehend aus Halogen, C₁-C₆-Alkyl, C₁-C₆-Alkoxy, C₁-C₆-Alkylamino, C₁-C₆-Alkoxycarbonyl, C₁-C₆-Alkylaminocarbonyl, C₁-C₆-Alkylaminosulfonyl, gegebenenfalls Oxo substituiertes 5- bis 7-gliedriges Heterocyclyl und 5- bis 10-gliedriges Heteroaryl,
- R⁶: Wasserstoff bedeutet,
- R⁷: Wasserstoff bedeutet,
oder
- R⁵: Wasserstoff bedeutet,
- R⁶: C₆-C₁₀-Arylaminocarbonyl, C₆-C₁₀-Arylcarbonyl, C₆-C₁₀-Arylaminothiocarbonyl, C₆-C₁₀-Arylthiocarbonyl, C₆-C₁₀-Arylsulfonylaminocarbonyl, 5- bis 10-gliedriges Heteroarylaminocarbonyl, 5- bis 10-gliedriges Heteroarylcarbonyl, 5- bis 10-gliedriges Heteroarylaminothiocarbonyl oder 5- bis 10-gliedriges Heteroarylthiocarbonyl bedeutet,
wobei Arylaminocarbonyl, Arylcarbonyl, Arylaminothiocarbonyl, Arylthiocarbonyl, Arylsulfonylaminocarbonyl, Heteroarylaminocarbonyl, Heteroarylcarbonyl, Heteroarylaminothiocarbonyl und Heteroarylthiocarbonyl substituiert sein können mit 0, 1, 2 oder 3 Substituenten unabhängig voneinander ausgewählt aus der Gruppe bestehend aus Halogen, C₁-C₆-Alkyl, C₁-C₆-Alkoxy, C₁-C₆-Alkylamino, C₁-C₆-Alkoxycarbonyl, C₁-C₆-Alkylaminocarbonyl, C₁-C₆-Alkylaminosulfonyl, gegebenenfalls Oxo substituiertes 5- bis 7-gliedriges Heterocyclyl und 5- bis 10-gliedriges Heteroaryl,
- R⁷: Wasserstoff bedeutet,
oder
- R⁵: Wasserstoff bedeutet,
- R⁶: Wasserstoff bedeutet,
- R⁷: C₆-C₁₀-Arylaminocarbonyl, C₆-C₁₀-Arylcarbonyl, C₆-C₁₀-Arylaminothiocarbonyl, C₆-C₁₀-Arylthiocarbonyl, 5- bis 10-gliedriges Heteroarylaminocarbonyl, 5- bis 10-gliedriges Heteroarylcarbonyl, 5- bis 10-gliedriges Heteroarylaminothiocarbonyl oder 5- bis 10-gliedriges Heteroarylthiocarbonyl bedeutet,
wobei Arylaminocarbonyl, Arylcarbonyl, Arylaminothiocarbonyl, Arylthiocarbonyl, Heteroarylaminocarbonyl, Heteroarylcarbonyl, Heteroarylaminothiocarbonyl und Heteroarylthiocarbonyl substituiert sein können mit 0, 1, 2 oder 3 Substituenten unabhängig voneinander ausgewählt aus der Gruppe bestehend aus Halogen, C₁-C₆-Alkyl, C₁-C₆-Alkoxy, C₁-C₆-Alkylamino, C₁-C₆-Alkoxycarbonyl, C₁-C₆-Alkylaminocarbonyl, C₁-C₆-Alkylaminosulfonyl, gegebenenfalls Oxo substituiertes 5- bis 7-gliedriges Heterocyclyl und 5- bis 10-gliedriges Heteroaryl,
oder
- R⁵ und R⁶: gleich sind, und
C₆-C₁₀-Arylaminocarbonyl, C₆-C₁₀-Arylcarbonyl, C₆-C₁₀-Arylaminothiocarbonyl, C₆-C₁₀-Arylthiocarbonyl, C₆-C₁₀-Arylsulfonylaminocarbonyl, 5- bis 10-gliedriges Heteroarylaminocarbonyl, 5- bis 10-gliedriges Heteroarylcarbonyl, 5- bis 10-gliedriges Heteroarylaminothiocarbonyl oder 5- bis 10-gliedriges Heteroarylthiocarbonyl bedeuten,
wobei Arylaminocarbonyl, Arylcarbonyl, Arylaminothiocarbonyl, Arylthiocarbonyl, Arylsulfonylaminocarbonyl, Heteroarylaminocarbonyl, Heteroarylcarbonyl, Heteroarylaminothiocarbonyl und Heteroarylthiocarbonyl substituiert sein können mit 0, 1, 2 oder 3 Substituenten unabhängig voneinander ausgewählt aus der Gruppe bestehend aus Halogen, C₁-C₆-Alkyl, C₁-C₆-Alkoxy, C₁-C₆-Alkylamino, C₁-C₆-Alkoxycarbonyl, C₁-C₆-Alkylaminocarbonyl, C₁-C₆-Alkylaminosulfonyl, gegebenenfalls Oxo substituiertes 5- bis 7-gliedriges Heterocyclyl und 5- bis 10-gliedriges Heteroaryl,
- R⁷: Wasserstoff bedeutet,
und ihre Salze, ihre Solvate und die Solvate ihrer Salze.

Bevorzugt sind auch Verbindungen der Formel (I), in welcher
- R¹: 2-Methylprop-1-yl bedeutet,
- R²: Wasserstoff bedeutet,
- R³: 1-Amino-3-methylbut-1-ylcarbonyl bedeutet,
- R⁴: Wasserstoff bedeutet,
und
- R⁵: Wasserstoff bedeutet,
- R⁶: C₆-C₁₀-Arylaminocarbonyl, C₆-C₁₀-Arylcarbonyl, C₆-C₁₀-Arylaminothiocarbonyl, C₆-C₁₀-Arylthiocarbonyl, C₆-C₁₀-Arylsulfonylaminocarbonyl, 5- bis 10-gliedriges Heteroarylaminocarbonyl, 5- bis 10-gliedriges Heteroarylcarbonyl, 5- bis 10-gliedriges Heteroarylaminothiocarbonyl oder 5- bis 10-gliedriges Heteroarylthiocarbonyl bedeutet,
wobei Arylaminocarbonyl, Arylcarbonyl, Arylaminothiocarbonyl, Arylthiocarbonyl, Arylsulfonylaminocarbonyl, Heteroarylaminocarbonyl, Heteroarylcarbonyl, Heteroarylaminothiocarbonyl und Heteroarylthiocarbonyl substituiert sein können mit 0, 1, 2 oder 3 Substituenten unabhängig voneinander ausgewählt aus der Gruppe bestehend aus Halogen, C₁-C₆-Alkyl, C₁-C₆-Alkoxy, C₁-C₆-Alkylamino, C₁-C₆-Alkoxycarbonyl, C₁-C₆-Alkylaminocarbonyl, C₁-C₆-Alkylaminosulfonyl, gegebenenfalls Oxo substituiertes 5- bis 7-gliedriges Heterocyclyl und 5- bis 10-gliedriges Heteroaryl,
- R⁷: Wasserstoff bedeutet,
oder
- R⁵: Wasserstoff bedeutet,
- R⁶: Wasserstoff bedeutet,
- R⁷: C₆-C₁₀-Arylaminocarbonyl, C₆-C₁₀-Arylcarbonyl, C₆-C₁₀-Arylaminothiocarbonyl, C₆-C₁₀-Arylthlocarbonyl, 5- bis 10-gliedriges Heteroarylaminocarbonyl, 5- bis 10-gliedriges Heteroarylcarbonyl, 5- bis 10-gliedriges Heteroarylaminothiocarbonyl oder 5- bis 10-gliedriges Heteroarylthiocarbonyl bedeutet,
wobei Arylaminocarbonyl, Arylcarbonyl, Arylaminothiocarbonyl, Arylthiocarbonyl, Heteroarylaminocarbonyl, Heteroarylcarbonyl, Heteroarylaminothiocarbonyl und Heteroarylthiocarbonyl substituiert sein können mit 0, 1, 2 oder 3 Substituenten unabhängig voneinander ausgewählt aus der Gruppe bestehend aus Halogen, C₁-C₆-Alkyl, C₁-C₆-Alkoxy, C₁-C₆-Alkylamino, C₁-C₆-Alkoxycarbonyl, C₁-C₆-Alkylaminocarbonyl, C₁-C₆-Alkylaminosulfonyl, gegebenenfalls Oxo substituiertes 5- bis 7-gliedriges Heterocyclyl und 5- bis 10-gliedriges Heteroaryl,
und ihre Salze, ihre Solvate und die Solvate ihrer Salze.

Bevorzugt sind auch Verbindungen der Formel (I), in welcher
- R¹: 2-Methylprop-1-yl bedeutet,
- R²: Wasserstoff bedeutet,
- R³: 1-Amino-3-methylbut-1-ylcarbonyl bedeutet,
- R⁴: Wasserstoff bedeutet,
und
- R⁵: Wasserstoff bedeutet,
- R⁶: Phenylaminocarbonyl, Phenylcarbonyl, Pyridylaminocarbonyl oder Pyridylcarbonyl bedeutet,
wobei Phenylaminocarbonyl, Phenylcarbonyl, Pyridylaminocarbonyl und Pyridylcarbonyl substituiert sein können mit 0, 1, 2 oder 3 Substituenten unabhängig voneinander ausgewählt aus der Gruppe bestehend aus Pyrrolidinyl, Piperidinyl, Tetrahydropyranyl, Piperazinyl, Morpholinyl, 2-Oxo-pyrrolidinyl und 2-Oxo-piperidinyl,
- R⁷: Wasserstoff bedeutet,
oder
- R⁵: Wasserstoff bedeutet,
- R⁶: Wasserstoff bedeutet,
- R⁷: Phenylaminocarbonyl, Phenylcarbonyl, Pyridylaminocarbonyl oder Pyridylcarbonyl bedeutet,
wobei Phenylaminocarbonyl, Phenylcarbonyl, Pyridylaminocarbonyl und Pyridylcarbonyl substituiert sein können mit 0, 1, 2 oder 3 Substituenten unabhängig voneinander ausgewählt aus der Gruppe bestehend aus Pyrrolidinyl, Piperidinyl, Tetrahydropyranyl, Piperazinyl, Morpholinyl, 2-Oxo-pyrrolidinyl und 2-Oxo-piperidinyl,
und ihre Salze, ihre Solvate und die Solvate ihrer Salze.

Bevorzugt sind auch Verbindungen der Formel (I), in welcher R¹ 2-Methylprop-1-yl, R² Wasserstoff, R³ 1-Amino-3-methylbut-1-ylcarbonyl und R⁴ Wasserstoff bedeutet, und R⁵, R⁶ und R⁷ die oben angegebene Bedeutung haben.

Bevorzugt sind auch Verbindungen der Formel (I), in welcher das von einer Aminosäure stammende Stereozentrum in R³ D-Konfiguration hat.

Bevorzugt sind auch Verbindungen der Formel (I), in welcher R⁶ und R⁷ Wasserstoff bedeuten und R⁵ Phenylaminocarbonyl, Phenylcarbonyl, Pyridylaminocarbonyl oder Pyridylcarbonyl bedeutet, wobei Phenylaminocarbonyl, Phenylcarbonyl, Pyridylaminocarbonyl und Pyridylcarbonyl substituiert sein können mit 0, 1, 2 oder 3 Substituenten unabhängig voneinander ausgewählt aus der Gruppe bestehend aus Pyrrolidinyl, Piperidinyl, Tetrahydropyranyl, Piperazinyl, Morpholinyl, 2-Oxo-pyrrolidinyl und 2-Oxo-piperidinyl, und R¹, R², R³ und R⁴ die oben angegebene Bedeutung haben.

Bevorzugt sind auch Verbindungen der Formel (I), in welcher R⁵ und R⁷ Wasserstoff bedeuten und R⁶ Phenylaminocarbonyl, Phenylcarbonyl, Pyridylaminocarbonyl oder Pyridylcarbonyl bedeutet, wobei Phenylaminocarbonyl, Phenylcarbonyl, Pyridylaminocarbonyl und Pyridylcarbonyl substituiert sein können mit 0, 1, 2 oder 3 Substituenten unabhängig voneinander ausgewählt aus der Gruppe bestehend aus Pyrrolidinyl, Piperidinyl, Tetrahydropyranyl, Piperazinyl, Morpholinyl, 2-Oxo-pyrrolidinyl und 2-Oxo-piperidinyl, und R¹, R², R³ und R⁴ die oben angegebene Bedeutung haben.

Bevorzugt sind auch Verbindungen der Formel (I), in welcher R⁵ und R⁶ Wasserstoff bedeuten und R⁷ Phenylaminocarbonyl, Phenylcarbonyl, Pyridylaminocarbonyl oder Pyridylcarbonyl bedeutet, wobei Phenylaminocarbonyl, Phenylcarbonyl, Pyridylaminocarbonyl und Pyridylcarbonyl substituiert sein können mit 0, 1, 2 oder 3 Substituenten unabhängig voneinander ausgewählt aus der Gruppe bestehend aus Pyrrolidinyl, Piperidinyl, Tetrahydropyranyl, Piperazinyl, Morpholinyl, 2-Oxo-pyrrolidinyl und 2-Oxo-piperidinyl, und R¹, R², R³ und R⁴ die oben angegebene Bedeutung haben.

Die in den jeweiligen Kombinationen bzw. bevorzugten Kombinationen von Resten im einzelnen angegebenen Restedefinitionen werden unabhängig von den jeweiligen angegebenen Kombinationen der Reste beliebig auch durch Restedefinitionen anderer Kombination ersetzt.

Ganz besonders bevorzugt sind auch Kombinationen von zwei oder mehreren der oben genannten Vorzugsbereiche.

Gegenstand der Erfindung ist weiterhin ein Verfahren zur Herstellung der Verbindungen der Formeln (I), wobei Verbindungen der Formel 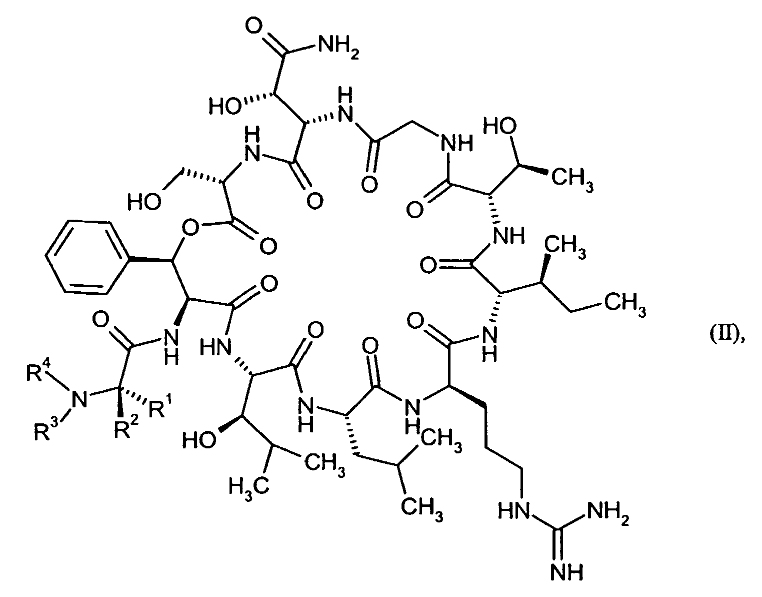 in welcher
R¹, R², R³ und R⁴ die oben angegebene Bedeutung haben,
mit 1 bis 10 Äquivalenten, bevorzugt 2 bis 5 Äquivalenten, eines Aryl- oder Heteroarylcarbonsäurechlorides, eines Aryl- oder Heteroarylisocyanates, eines Aryl- oder Heteroarylthio-carbonsäurechlorides, eines Aryl- oder Heteroarylisothiocyanates oder eines Arylsulfonyl-isocyanates, wobei die Aryl- und Heteroaryl-Reste den Aryl- und Heteroaryl-Resten in den Resten R⁵, R⁶ und R⁷ entsprechen, die die oben angegebene Bedeutung haben,
umgesetzt werden,
und anschließend das entstehende Gemisch von Verbindungen der Formel (I) durch Chromatographie in die Einzelverbindungen der Formel (I) getrennt wird.

Freie Amino-Gruppen in den Resten R¹, R², R³ und R⁴ werden vor der Umsetzung nach dem Fachmann bekannten Methoden zum Beispiel mit einer Boc-Schutzgruppe geschützt, die nach der Umsetzung und vor der Chromatographie wieder abgespalten wird.

Die Umsetzung mit Aryl- und Heteroarylcarbonsäurechloriden, Aryl- und Heteroarylisocyanaten, Aryl- und Heteroarylthiocarbonsäurechloriden, Aryl- und Heteroarylisothiocyanaten und Arylsulfonylisocyanaten erfolgt im Allgemeinen in inerten Lösungsmitteln, gegebenenfalls in Gegenwart einer Base, bevorzugt in einem Temperaturbereich von -30°C bis 50°C bei Normaldruck.

Inerte Lösungsmittel sind beispielsweise Tetrahydrofuran, Methylenchlorid, Pyridin, Dioxan, Dimethylformamid oder ein Gemisch der angegebenen Lösungsmittel. Als inerte Lösungsmittel sind Tetrahydrofuran, Methylenchlorid und Dimethylformamid bevorzugt.

Basen sind beispielsweise Triethylamin, Diisopropylethylamin oder N-Methylmorpholin, bevorzugt ist Diisopropylethylamin.

Die Verbindungen der Formel (I), die als Salze vorliegen, können zum Beispiel durch Umsetzung mit Salzsäure oder Methansulfonsäure in ein Salz mit einem anderen Gegenion überführt werden.

Die Aryl- und Heteroarylearbonsäurechloride, Aryl- und Heteroarylisocyanate, Aryl- und Heteroarylthiocarbonsäurechloride, Aryl- und Heteroarylisothiocyanate und Arylsulfonyl-isocyanate sind bekannt oder können analog bekannten Verfahren hergestellt werden.

Die Verbindungen der Formel (II) sind bekannt oder können hergestellt werden, indem die Verbindung der Formel 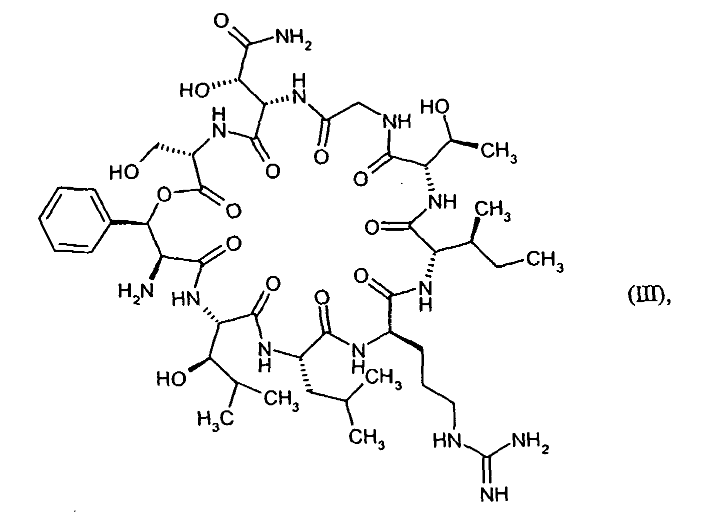 mit Verbindungen der Formel 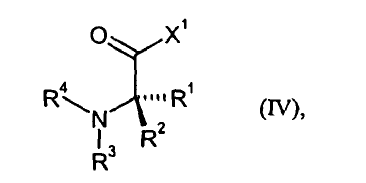 in welcher
- R¹, R², R³ und R⁴: die oben angegebene Bedeutung haben, und
- X¹: Halogen, bevorzugt Brom, Chlor oder Fluor, oder Hydroxy bedeutet,
umgesetzt wird.

Falls X¹ für Halogen steht, erfolgt die Umsetzung im Allgemeinen in inerten Lösungsmitteln, gegebenenfalls in Gegenwart einer Base, bevorzugt in einem Temperaturbereich von -30°C bis 50°C bei Normaldruck.

Inerte Lösungsmittel sind beispielsweise Tetrahydrofuran, Methylenchlorid, Pyridin, Dioxan oder Dimethylformamid. Als inerte Lösungsmittel sind Tetrahydrofuran oder Methylenchlorid bevorzugt.

Basen sind beispielsweise Triethylamin, Diisopropylethylamin oder N-Methylmorpholin, bevorzugt ist Diisopropylethylamin.

Falls X¹ für Hydroxy steht, erfolgt die Umsetzung im Allgemeinen in inerten Lösungsmitteln, in Gegenwart eines Dehydratisierungsreagenzes, gegebenenfalls in Gegenwart einer Base, bevorzugt in einem Temperaturbereich von -30°C bis 50°C bei Normaldruck.

Inerte Lösungsmittel sind beispielsweise Halogenkohlenwasserstoffe wie Dichlormethan oder Trichlormethan, Kohlenwasserstoff wie Benzol, Nitromethan, Dioxan, Dimethylformamid oder Acetonitril. Ebenso ist es möglich, Gemische der Lösemittel einzusetzen. Besonders bevorzugt ist Dichlormethan oder Dimethylformamid.

Als Dehydratisierungsreagenzien eignen sich hierbei beispielsweise Carbodiimide wie z.B. *N,N'-*Diethyl-, *N*,*N*,'-Dipropyl-, *N*,*N*'-Di isopropyl-, *N*,*N*'-Dicyclohexylcarbodiimid, *N-*(3*-*Dimethylaminoisopropyl)-*N*'-ethy lcarbodiimid-hydrochlorid (EDC), *N*-Cyclohexylcarbodiimid-*N*'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2*-tert-*Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)-phosphoniumhexafluorophosphat, oder *O*-(Benzotriazol-1-yl)-*N*,*N*,*N*'*N*'-tetra-methyluroniumhexafluorophosphat (HBTU), 2-(2-Oxo-1-(2H)-pyridyl)-1,1,3,3-tetramethyluroniumtetrafluoroborat (TPTU) oder *O*-(7-Azabenzotriazol-1-yl)-*N*,*N*,*N*'*N*'-tetramethyl-uroniumhexafluorophosphat (HATU), oder 1-Hydroxybenztriazol (HOBt), oder Benzotriazol-1-yloxytris(dimethylamino)-phosphoniumhexafluorophosphat (BOP), oder *N*-Hydroxysuccinimid, oder Mischungen aus diesen, mit Basen.

Basen sind beispielsweise Alkalicarbonate, wie z.B. Natrium- oder Kaliumcarbonat, oder -hydrogencarbonat, oder organische Basen wie Trialkylamine z.B. Triethylamin, *N*-Methylmorpholin, *N*-Methylpiperidin, 4-Dimethylaminopyridin oder Diisopropylethylamin.

Vorzugsweise wird die Kondensation mit HATU oder mit EDC in Gegenwart von HOBt durchgeführt.

Die Verbindungen der Formel (IV) tragen gegebenenfalls Schutzgruppen, so dass sich in diesen Fällen der Umsetzung der Verbindung der Formel (III) mit Verbindungen der Formel (IV) eine Abspaltung der Schutzgruppen mit zum Beispiel Trifluoressigsäure nach dem Fachmann bekannten Methoden anschließt.

Die Verbindung der Formel (III) lässt sich durch doppelten Edmann-Abbau aus Lysobactin (Beispiel 1A) synthetisieren.

Die Verbindungen der Formel (IV) sind bekannt oder lassen sich nach bekannten Verfahren aus den entsprechenden Edukten synthetisieren.

Die Herstellung der erfindungsgemäßen Verbindungen kann durch folgendes Syntheseschemata verdeutlicht werden.

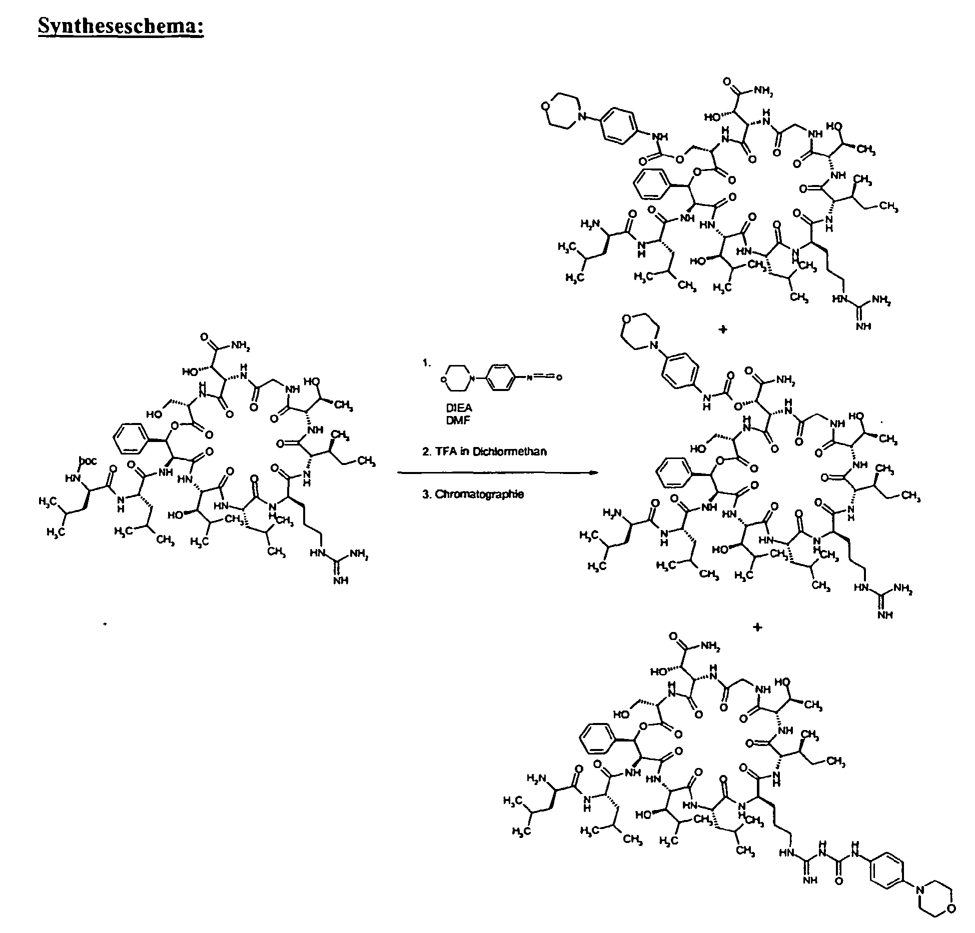

Die erfindungsgemäßen Verbindungen zeigen ein nicht vorhersehbares, wertvolles pharmakologisches Wirkspektrum. Sie zeigen eine antibakterielle Wirkung.

Sie eignen sich daher zur Verwendung als Arzneimittel zur Behandlung und/oder Prophylaxe von Krankheiten bei Menschen und Tieren.

Die erfindungsgemäßen Verbindungen zeichnen sich durch eine geringere Nephrotoxizität gegenüber Lysobactin aus.

Die erfindungsgemäßen Verbindungen zeichnen sich durch eine bessere Löslichkeit gegenüber Lysobactin aus.

Die beschriebenen Nonadepsipeptide wirken als Inhibitoren der bakteriellen Zellwandbiosynthese.

Besonders wirksam sind die erfindungsgemäßen Zubereitungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Grundsätzlich können die erfindungsgemäßen Zubereitungen gegen alle Bakterien und bakterienähnlichen Mikroorganismen verwendet werden, die im Besitz einer bakteriellen Zellwand (Murein sacculus) bzw. den dazugehörigen Enzymsystemen sind, beispielsweise durch die folgenden Erreger oder durch Mischungen der folgenden Erreger:

Gram-negative Kokken *(Neisseria gonorrhoeae)* sowie Gram-negative Stäbchen wie Enterobakteriaceen, z.B. *Escherichia coli, Hämophilus influenzae,* Pseudomonas, Klebsiella, Citrobacter (*C. freundii, C. divernis*), Salmonella und Shigella; ferner Enterobacter (*E. aerogenes, E. agglomerans*), Hafnia, Serratia *(S. marcescens),* Providencia, Yersinia, sowie die Gattung Acinetobacter, Branhamella und Chlamydia. Darüber hinaus umfasst das antibakterielle Spektrum strikt anaerobe Bakterien wie z.B. *Bacteroides fragilis,* Vertreter der Gattung Peptococcus, Peptostreptococcus sowie die Gattung Clostridium; ferner Mykobakterien, z.B. *M. tuberculosus.* Besonders ausgeprägte Wirkung zeigen die erfindungsgemäßen Verbindungen gegen Gram-positive Kokken, z.B. Staphylokokken *(S. aureus, S. epidermidis, S. haemolyticus, S. carnosus),* Enterokokken *(E. faecalis, E. faecium)* und Streptokokken *(S. agalactiae, S. pneumoniae, S. pyogenes).*

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Zubereitungen verhindert, gebessert oder geheilt werden können, seien beispielsweise genannt:

Infektionskrankheiten beim Menschen wie z.B. unkomplizierte und komplizierte Hamwegsinfekte, unkomplizierte Haut- und Oberflächeninfektionen, komplizierte Haut- und Weichteilinfektionen, im Hospital und ambulant erworbene Lungenentzündung, nosokomiale Pneumonien, akute Exazerbationen und sekundäre bakterielle Infektionen der chronischen Bronchitis, akute Otitis media, akute Sinusitis, streptokokkale Pharyngitis, bakterielle Meningitis, unkomplizierte gonokokkale und nicht-gonokokkale Urethritis/Cervizitis, akute Prostatitis, Endocarditis, unkomplizierte und komplizierte intra-abdominale Infektionen, gynäkologische Infektionen, pelvische Entzündungskrankheit, bakterielle Vaginose, akute und chronische Osteomyelitis, akute bakterielle Arthritis, empirische Therapie in febrilen neutropenischen Patienten, desweiteren Bakterämien, MRSA Infektionen, akute infektiöse Diarrhoe, *Helicobacter pylori* Infektionen, postoperative Infektionen, odontogene Infektionen, ophthalmologische Infektionen, postoperative Infektionen (inkl. periproktaler Abszess, Wundinfektionen, Galleninfektionen, Mastitis und akute Appendizitis), zystische Fibrose und Bronchiectasis.

Außer beim Menschen können bakterielle Infektionen auch bei anderen Spezies behandelt werden. Beispielhaft seien genannt:

Schwein: Diarrhoe, Enterotoxamie, Sepsis, Dysenterie, Salmonellose, Metritis-Mastitis-Agalaktiae-Syndrom, Mastitis;

Wiederkäuer (Rind, Schaf, Ziege): Diarrhoe, Sepsis, Bronchopneumonie, Salmonellose, Pasteurellose, Genitalinfektionen;

Pferd: Bronchopneumonien, Fohlenlähme, puerperale und postpuerperale Infektionen, Salmonellose;

Hund und Katze: Bronchopneumonie, Diarrhoe, Dermatitis, Otitis, Harnwegsinfekte, Prostatitis;

Geflügel (Huhn, Pute, Wachtel, Taube, Ziervögel und andere): *E. coli*-Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie z.B. Pasteurella, Brucella, Campylobacter, Listeria, Erysipelothris, Corynebakterien, Borellia, Treponema, Nocardia, Rikettsia, Yersinia, erweitert.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Bevorzugt werden die erfindungsgemäßen Verbindungen zur Herstellung von Arzneimitteln verwendet, die zur Prophylaxe und/oder Behandlung von bakteriellen Erkrankungen geeignet sind.

Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen. Bevorzugte Kombinationswirkstoffe sind antibakteriell wirkende Verbindungen, die ein anderes Wirkspektrum, insbesondere ergänzendes Wirkspektrum, haben und/oder synergistisch zu den erfindungsgemäßen Verbindungen sind.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich als vorteilhaft erwiesen, bei intravenöser Applikation Mengen von etwa 0.001 bis 100 mg/kg, vorzugsweise etwa 0.1 bis 10 mg/kg Körpergewicht zur Erzielung wirksamer Ergebnisse zu verabreichen, und bei oraler Applikation beträgt die Dosierung etwa 0.01 bis 50 mg/kg, vorzugsweise 0.5 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen

- Allg.: Allgemein(e)
- Area: (Peak)fläche
- ber.: berechnet
- BHI: Brain Heart Infusion
- Boc: *tert*-Butyloxycarbonyl
- br.: breites Signal (bei NMR-Spektren)
- Bsp.: Beispiel
- d: Dublett (bei NMR-Spektren)
- DC: Dünnschichtchromatographie
- DCI: direkte chemische Ionisation (bei MS)
- DCM: Dichlormethan
- DIEA: *N*,*N*-Diisopropylethylamin
- DMSO: Dimethylsulfoxid
- DMF: *N*,*N*-Dimethylformamid
- d. Th.: der Theorie
- EDC: 1-Ethyl-3-(3-dimethylaminopropyl)carbodiimide (auch EDCI)
- EDCxHCl: 1-Ethyl-3-(3-dimethylaminopropyl)carbodiimide-hydrochlorid
- EE: Ethylacetat (Essigsäureethylester)
- EI: Elektronenstoß-Ionisation (bei MS)
- ESI: Elektrospray-Ionisation (bei MS)
- Fp.: Schmelzpunkt
- gef.: gefunden
- ges.: gesättigt
- h: Stunde
- HATU: *O*-(7-Azabenzotriazol-1-yl)-*N*,*N*,*N*`,*N*'-tetramethyluronium-hexafluorphosphat
- HOBt: 1-Hydroxybenzotriazol
- HPLC: Hochdruck-, Hochieistungsflüssigchromatographie
- HR: High Resolution (Hochauflösung)
- i. V.: im Vakuum
- konz.: konzentriert
- LC-MS: Flüssigchromatographie-gekoppelte Massenspektroskopie
- LDA: Lithium-Diisopropylamid
- m: middle (mittel) (bei UV- und IR-Spektren)
- m: Multiplett (bei NMR-Spektren)
- MALDI: Matrix-Assisted Laser Desorption/Ionization
- MHK: Minimale Hemmkonzentration
- min: Minute/Minuten
- MRSA: Methicillin-resistenter *Staphylococcus aureus*
- MS: Massenspektroskopie
- NCCLS: National Committee for Clinical Laboratory Standards
- neg.: negativ
- NMM: *N*-Methylmorpholin
- NMR: Kernresonanzspektroskopie (nuclear magnetic resonance)
- p.a.: pro analysi
- Pd-C: Palladium auf Kohle
- pos.: positiv
- proz.: Prozentig
- quant.: quantitativ
- RP-HPLC: Reverse Phase HPLC
- RT: Raumtemperatur
- R,: Retentionszeit (bei HPLC)
- s: strong (stark) (bei UV- und IR-Spektren)
- s: Singulett (bei NMR-Spektren)
- TBTU: *O*-(Benzotriazol-1-yl)-*N*,*N*,*N*',*N*'-tetramethyluronium-Tetrafluoroborat
- TCTU: *O*-(1H-6-Chlorbenzotriazol-1-yl)-1,1,3,3-tetramethyluronium-Tetrafluoroborat
- TFA: Trifluoressigsäure
- TFE: 2,2,2-Trifluorethanol
- THF: Tetrahydrofuran
- TOF: Flugzeit (time of flight)
- UV: Ultraviolett
- Vis: sichtbar (visible)
- VRSA: Vancomycin-resistenter *Staphylococcus aureus*
- w: weak (schwach) (bei UV- und IR-Spektren)
- Z, Cbz: Benzyloxycarbonyl

### Literatur

Zur Nomenklatur der Peptide und Cyclodepsipeptide vergleiche:
1. A Guide to IUPAC Nomenclature of Organic Compounds (Recommendations 1993), 1993, Blackwell Scientific publications.
2. Nomenclature and symbolism for amino acids and peptides. Recommendations 1983. IUPAC-IUB Joint Commission on Biochemical Nomenclature, UK. Biochemical Journal 1984, 219, 345-373. Sowie zitierte Literatur.
3. Zur Nomenklatur von Nonadepsipeptid-Derivaten, die in den Aminosäure-Seitenketten derivatisiert sind, wird das IUPAC-Prefix-System zur Adressierung der jeweiligen Derivatisierungsstelle verwendet (IUPAC, Nomenclature and Symbolism for Amino Acids and Peptides, Names and Symbols for Derivatives of Named Peptides, Section 3AA-22, Recommendations 1983-1992). So bezeichnet beispielsweise *N*^{*ω.*6}-Acetyl-lysobaclin ein an der Aminosäure 6 (vom *N*-Terminus des Depsipeptids gerechnet, d.h. hier D-Arg) speziell am terminalen Stickstoffatom acetyliertes Lysobactin. Analog bezeichnet *O*^{3.11}-Methyl-lysobactin ein an der Aminosäure 11 (Ser) am Seitenkettensauerstoffatom (*O*³) methyliertes Derivat.

### Allgemeine Methoden LC-MS, HR-MS, HPLC und Gelchromatographie

**Methode 1 (HPLC)**: Gerätetyp HPLC: HP 1100 Series; UV DAD Säule: Zorbax Eclipse XBD-C8 (Agilent), 150 mm x 4.6 mm, 5 µm; Eluent A: 5 ml HClO₄/1 Wasser, Eluent B: Acetonitril; Gradient: 0-1 min 10%B, 1-4 min 10-90%B, 4-5 min 90%B; Fluss: 2.0 ml/min; Ofen: 30°C; UV-Detektion: 210 und 254 nm.

**Methode 2 (HPLC)**: Säule: Kromasil RP-18, 60 mm x 2 mm, 3.5 µm; Eluent A: 5 ml HClO₄/l Wasser, Eluent B: Acetonitril; Gradient: 0 min 2%B, 0.5 min 2%B, 4.5 min 90%B, 9 min 90%B; Fluss: 0.75 ml/min; Ofen: 30°C; UV-Detektion: 210 nm.

**Methode 3 (LC-MS)**: Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min. 2 ml/min; Ofen: 50°C; UV-Detektion: 210 nm.

**Methode 4 (HPLC):** Säule: Kromasil RP-18, 250 mm x 4 mm, 5 µm; Eluent A: 5 ml HCIO₄/l Wasser, Eluent B: Acetonitril; Gradient: 0 min 5%B, 10 min 95%B; Fluss: 1 ml/min; Ofen: 40°C; UV-Detektion: 210 nm.

**Methode 5 (HPLC):** Säule: Kromasil RP-18, 250 mm x 4 mm, 5 µm; Eluent A: 2 ml HCIO₄/I Wasser, Eluent B: Acetonitril; Isokratisch: 45%B, 55%A; Fluss: 1 ml/min; Ofen: 40°C; UV-Detektion: 210 nm.

**Methode 6 (HPLC):** Säule: Kromasil RP-18, 250 mm x 4 mm, 5 µm; Eluent A: 2 ml HCIO₄/I Wasser, Eluent B: Acetonitril; Isokratisch: 50%B, 50%A; Fluss: 1 ml/min; Ofen: 40°C; UV-Detektion: 210 nm.

**Methode 7 (MALDI-MS):** Die *MALDI-MS*/*MS-Untersuchungen* werden mit einem 4700 Proteomics Analyzer (Applied Biosystems, Framingham, MA, USA) durchgeführt, der mit TOF/TOF Ionenoptik und 200 Hz Nd:YAG Laser (355 nm) ausgestattet ist. Die Quasimolekülionen werden in der Ionenquelle mit 8 kV beschleunigt, mit einem elektrischen Deflektor selektiert (MS1), und in einer Stoßzelle, die zwischen MS1 und MS2 angeordnet ist, mit Argonatomen gestoßen. Die entstehenden Fragmentionen werden mit 15 kV nachbeschleunigt und mit dem zweiten Flugzeit-Massenanalysator (MS2) charakterisiert.

**Methode 8 (TOF-HR-MS):** *TOF-HR-MS-ESI+* Spektren werden mit einem Micromass LCT Gerät (Kapillarspannung: 3.2 KV, Cone-Spannung: 42 V, Quellentemperatur: 120°C, Desolvations-Temperatur: 280°C) aufgenommen. Hierzu wird eine Spritzenpumpe (Firma Harvard Apparatus) für die Probenzuführung verwendet. Als Standart dient Leucin-Enkephalin (Tyr-Gly-Gly-Phe-Leu).

**Methode 9 (Gelchromatographie Sephadex LH-20):** Gelchromatographie wird ohne Druck an Sephadex LH-20 (Firma Pharmacia) durchgeführt. Es wird nach UV-Aktivität (UV Detektor für 210 nm, Firma Knauer) fraktioniert (Fraktionssammler ISCO Foxy 200). Säulendimensionen: 60 x 21 cm (2500-5000 µmol Maßstab); 50 x 10 cm (500-2500 µmol Maßstab); 30 x 5 cm (250-500 µmol Maßstab); 25 x 4 cm (50-250 µmol Maßstab); 40 x 2 cm (5-50 µmol Maßstab).

**Methode 10 (Reprosil):** Säule: Gilson Abimed HPLC; Varian binäres Pumpensystem; Reprosil ODS-A 5µ 250 mm x 20 mm; Fluß: 25 ml/min; Ofen: RT; UV-Detektion: 210 nm; Eluent A: Wasser/0.2% TFA, Eluent B: Acetonitril; Gradient: 0-10 min 5-95%B, anschließend Regeneration der Chromatographiesäule.

**Methode 11 (Reprosil):** Säule: Gilson Abimed HPLC; Varian binäres Pumpensystem; Reprosil ODS-A 5µ 250 mm x 20 mm; Fluß: 25 ml/min; Ofen: RT; UV-Detektion: 210 nm; Eluent A: Wasser/0.2% TFA, Eluent B: Acetonitril; Gradient: 0-10 min 15-65%B, anschließend Regeneration der Chromatographiesäule.

**Methode 12 (Phenomenex Luna):** Säule: Gilson Abimed HPLC; Varian binäres Pumpensystem; Phenomenex Luna C18 5µ 250 mm x 20 mm; Fluß: 25 ml/min; Ofen: RT; UV-Detektion: 210 nm; Eluent A: Wasser/0.2% TFA, Eluent B: Acetonitril; Isokratisch 50%B.

**Methode 13 (Kromasil):** Säule: Gilson Abimed HPLC; Varian binäres Pumpensystem; Kromasil 100 C18 5µ 250 mm x 20 mm; Fluß: 25 ml/min; Ofen: RT; UV-Detektion: 210 nm; Eluent A: Wasser/0.2% TFA, Eluent B: Acetonitril; Isokratisch 65%B.

**Methode 14 (Reprosil):** Säule: Gilson Abimed HPLC; Varian binäres Pumpensystem; Reprosil ODS-A 5µ 250 mm x 20 mm; Fluß: 25 ml/min; Ofen: RT; UV-Detektion: 210 nm; Eluent A: Wasser/0.2% TFA, Eluent B: Acetonitril; Gradient: 0-15 min 10-90%B, anschließend Regeneration der Chromatographiesäule.

**Methode 15 (Kromasil):** Säule: Gilson Abimed HPLC; Varian binäres Pumpensystem; Kromasil 100 C18 5µ 250 mm x 20 mm; Fluß: 25 ml/min; Ofen: 40°C; UV-Detektion: 210 nm; Eluent A: Wasser/0.2% TFA, Eluent B: Acetonitril; Gradient: 0-15 min 70-55%A, 15.1-20 min 70%A, anschließend Regeneration der Chromatographiesäule

**Methode 16 (Reprosil):** Säule: Gilson Abimed HPLC; Varian binäres Pumpensystem; Reprosil ODS-A 5µ 250 mm x 20 mm; Fluß: 25 ml/min; Ofen: RT; UV-Detektion: 210 nm; Eluent A: Wasser/0.2% TFA, Eluent B: Acetonitril; Isokratisch 57%A.

**Methode 17 (Symmetryprep):** Gerät: Gilson Abimed HPLC; UV Detektor 210 nm; binäres Pumpensystem; Säule: SymmetryPrep^{™}C₁₈, Firma Waters, 7 µm; 300 mm x 19 mm; Fluß: 7 ml/min; Eluent A: Wasser/0.5% TFA, Eluent B: Acetonitril/0.5% TFA; Gradient: 0-5 min 5%B, 5-30 min 5-60%B, 30-35 min 60-98%B, 35-40 min 98%B, anschließend Regeneration der Chromatographiesäule.

**Methode 18 (cHPLC-MALDI-MS)**: Die *MALDI-MS*/*MS-Untersuchungen* werden mit einem 4700 Proteomics Analyzer (Applied Biosystems, Framingham, MA, USA) durchgeführt, der mit TOF/TOF Ionenoptik und 200 Hz Nd:YAG Laser (355 nm) ausgestattet ist. Die Quasimolekülionen werden in der Ionenquelle mit 8 kV beschleunigt, mit einem elektrischen Deflektor selektiert (MS1), und in einer Stoßzelle, die zwischen MS1 und MS2 angeordnet ist, mit Argonatomen gestoßen. Die entstehenden Fragmentionen werden mit 15 kV nachbeschleunigt und mit dem zweiten Flugzeit-Massenanalysator (MS2) charakterisiert. Die cHPLC-MALDI-TOF/TOF Kopplung wird offline mittels eines PROBOT-Systems (Fa. Dionex) durchgeführt.

### Allgemeine Arbeitsvorschriften

### Allgemeine Arbeitsvorschrift 1 (Veresterung)

Zu einer Lösung des *N*-Boc geschützten Peptids (0.3 mmol) in trockenem DCM (10 ml) werden unter Argonschutzgasatmosphäre DIEA (1 mmol) und ein Säurechlorid (0.3 mmol) zugegeben. Das Reaktionsgemisch wird bei RT gerührt. Der Reaktionsverlauf wird mittels analytischer HPLC (Methode 13) kontrolliert. Weitere Portionen Säurechlorid (je 0.3 mmol) werden so lange zugegeben, bis die analytische HPLC ausreichenden Umsatz anzeigt (>95%). Das Reaktionsgemisch wird mit Essigsäure versetzt (pH ca. 7) und dann chromatographisch mit Methode 9 (Methanol/Aceton: 4/1, 0.5% Essigsäure) und / oder Methode 12 aufgereinigt.

### Allgemeine Arbeitsvorschrift 2 (Carbamoylierung)

Zu einer Lösung des N-Boc geschützten Peptids (0.3 mmol) in trockenem DMF (100 ml) werden unter Argonschutzgasatmosphäre DIEA (1 mmol) und ein Isocyanat (0.3 mmol) zugegeben. Das Reaktionsgemisch wird bei RT gerührt. Der Reaktionsverlauf wird mittels analytischer HPLC (Methode 13) kontrolliert. Weitere Portionen Isocyanat (je 0.3 mmol) werden so lange zugegeben, bis die analytische HPLC ausreichenden Umsatz anzeigt (>95%). Das Reaktionsgemisch wird mit Essigsäure versetzt (pH ca. 7) und dann chromatographisch mit Methode 9 (Methanol/Aceton: 4/1, 0.5% Essigsäure) und / oder Methode 12 aufgereinigt.

### Allgemeine Arbeitsvorschrift 3 (Hydrolytische Probenvorbereitung für MALDI-MS)

Das zu öffnende Depsipeptid (z.B. Lysobactin, 0.05 µMol) wird in einem Microvial (500 µl bis 1000 µl) zunächst mit einem Borat-Salzsäure Puffer (Fa. Merck) pH 8 (250 µl) versetzt. Man lässt über Nacht stehen, versetzt mit Essigsäure (100 µl) und gefriertrocknet die Probe. Das Rohprodukt wird ohne weitere Reinigungsschritte mittels MALDI-MS-Sequenzierung untersucht.

### Ausgangsverbindungen

### Beispiel 1A

### D-Leucyl-N¹-{(3S,6S,12S,15S,18R,21S,24S,27S,28R)-6-[(1S)-1-amino-1-hydroxy-2-oxoethyl]-18-(3-{[amino(imino)methyl]amino}propyl)-12-[(1S)-1-hydroxyethyl)-3-(hydroxymethyl)-24-[(1R)-1-hydroxy-2-methylpropyl]-21-isobutyl-15-[(1S)-1-methylpropyl]-2,5,8,11,14,17,20,23,26-nonaoxo-28-phenyl-1-oxa-4,7,10,13,16,19,22,25-octaazacyclooctacosan-27-yl}-L-leucinamid-bistrifluoracetat (Lysobactin)

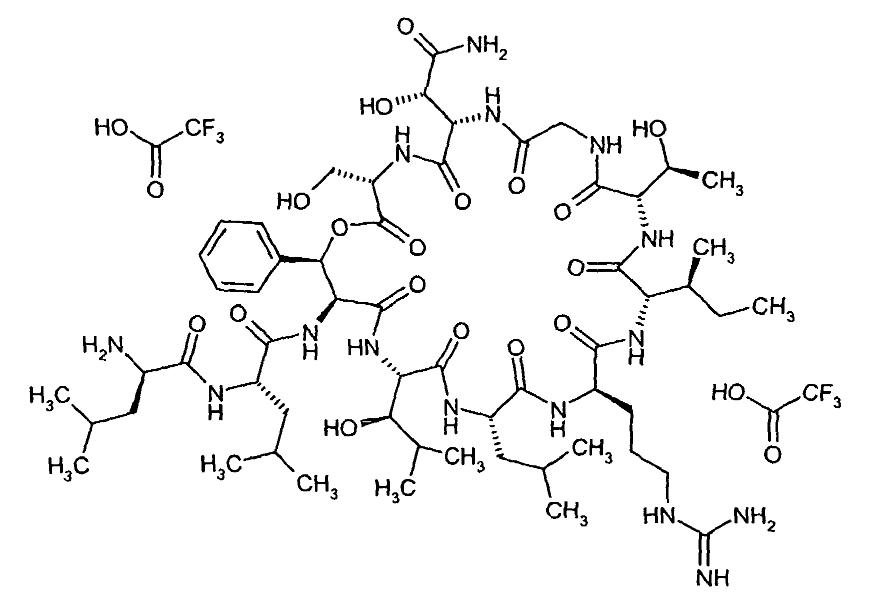

### Fermentation:

### Kultur Medium:

YM: Hefe-Malz Agar: D-Glucose (4 g/l), Hefe Extrakt (4 g/l), Malz Extrakt (10 g/l), 1 Liter Lewatit Wasser. Vor dem Sterilisieren (20 Minuten bei 121°C) wird der pH auf 7.2 eingestellt.
HPM: Mannit (5.4 g/l), Hefe Extrakt (5 g/l), Fleischpepton (3 g/l).
Arbeitskonserve: Der lyophilisierte Stamm (ATCC 53042) wird in 50 ml YM Medium angezogen.
Kelbenfertnentation: 150 ml YM Medium oder 100 ml HPM Medium in einem 1 1 Erlenmeyerkolben werden mit 2 ml der Arbeitskonserve beimpft und für 30-48 Stunden bei 28°C auf einem Schüttler bei 240 Upm wachsen gelassen.

30 l Fermentation: 300 ml der Kolbenfermentation (HPM Medium) werden zum Animpfen einer sterilen 30 1 Nährmedienlösung verwandt (1 ml Antifoam SAG 5693/1). Diese Kultur wird für 21 Stunden bei 28°C, 300 Upm und einer Belüftung mit steriler Luft von 0.3 vvm wachsen gelassen. Der pH wird mit 1 M Salzsäure auf pH = 7.2 konstant gehalten. Insgesamt werden während der Kultivierungszeit 880 ml 1 M Salzsäure zugeführt.

Hauptkultur (2001): 15 x 150 ml YM Medium in 1 1 Erlenmeyerkolben werden mit 2 ml der Arbeitskonserve beimpft und bei 28°C für 48 Stunden und 240 Upm auf dem Schüttler wachsen gelassen. 2250 ml dieser Kultur werden zum Beimpfen einer sterilen 200 1 Nährmedienlösung (YM) verwandt (1 ml Antifoam SAG 5693/1) und für 18.5 Stunden bei 28°C, 150 Upm und einer Belüftung mit steriler Luft von 0.3 vvm wachsen gelassen.

Zur Kontrolle des Fermentationsverlaufs werden stündlich Proben (50 ml) entnommen. 2 ml dieser Kulturbrühe werden mit 1 ml Methanol (0.5% Trifluoressigsäure) versetzt und über einen 0.45 µm Filter filtriert. 30 µl dieser Suspension werden mittels HPLC analysiert (Methode 1 und Methode 2).

Nach 18.5 Stunden wird die Kulturbrühe der Hauptkultur bei 17000 Upm in Überstand und Sediment getrennt.

### Isolierung:

Der Überstand (183 1) wird mit konzentrierter Trifluoressigsäure bzw. Natronlauge auf pH 6.5-7 eingestellt und auf eine Lewapolsäule (OC 1064, 60 1 Inhalt) aufgetragen. Anschließend wird mit reinem Wasser, Wasser/Methanol 1:1 und anschließend mit reinem Methanol (mit 0,1% Trifluoressigsäure) eluiert. Diese organische Phase wird im Vakuum bis zu einem verbleibenden wässrigen Rest von 11.51 eingeengt.

Die verbleibende wässrige Phase wird an Kieselgel C₁₈ gebunden und aufgetrennt (MPLC, Biotage Flash 75, 75 x 30 cm, KP-C18-WP, 15-20 µm, Fluss: 30 ml; Eluent: Acetonitril/ Wasser mit 0.1% Trifluoressigsäure; Gradient: 10%, 15% and 40% Acetonitril). Die 40% Acetonitril Phase, die die Hauptmenge von Beispiel 1A enthält, wird im Vakuum eingeengt und anschließend lyophilisiert (ca. 13 g). Dieses Feststoffgemisch wird in 1.2 g Portionen zunächst an einer präparativen HPLC (Methode 3), anschließend durch Gelfiltration an Sephadex LH-20 (5 x 70 cm, Acetonitril/Wasser 1:1, jeweils mit 0.05% Trifluoressigsäure) und einer weiteren präparativen HPLC (Methode 4) getrennt.

Dieser Prozess liefert 2250 mg Beispiel 1A.

Das Sediment wird in 4 l Aceton/Wasser 4:1 aufgenommen, mit 2 kg Celite versetzt, mit Trifluoressigsäure auf pH = 6 eingestellt, ausgerührt und zentrifugiert. Das Lösungsmittel wird im Vakuum eingeengt und der Rückstand gefriergetrocknet. Das erhaltene Lyophilisat (89.9 g) wird in Methanol aufgenommen, abfiltriert, eingeengt und an Kieselgel (Methode 5) getrennt. Beispiel 1A wird danach per Gelfiltration (Sephadex LH-20, 5 x 68 cm, Wasser/Acetonitril 9:1 (mit 0.05% Trifluoressigsäure), Fluss: 2.7 ml/min, Fraktionsgröße 13.5 ml) zur Reinsubstanz aufgereinigt.

Dieser Prozess liefert 447 mg Beispiel 1A.
HPLC (Methode 1): Rₜ = 6.19 min
MS (ESIpos): m/z = 1277 (M+H)⁺
¹H NMR (500.13 MHz, *d₆*-DMSO): δ = 0.75 (d, 3H), 0.78 (d, 6H), 0.80 (t, 3H), 0.82 (d, 3H), 0.90 (d, 3H), 0.91 (d, 3H), 0.92 (d, 3H), 0.95 (d, 3H), 0.96 (d, 3H), 1.05 (m, 1H), 1.19 (d, 3H), 1.25 (m, 2H), 1.50 (m, 4H), 1.51 (m, 2H), 1.55 (m, 1H), 1.61 (m, 1H), 1.65 (m, 1H), 1.84 (m, 1H), 1.85 (m, 1H), 1.86 (m, 1H), 1.89 (m, 1H), 1.95 (m, 1H), 2.75 (m, 2H), 3.40 (m, 1H), 3.52 (m, 2H), 3.53 (dd, 1H), 3.64 (m, 2H), 3.66 (m, 1H), 3.68 (dd, 1H), 3.73 (m, 2H), 4.00 (dd, 1H), 4.02 (br., 1H), 4.13 (br., 1H), 4.32 (dd, 1H), 4.39 (t, 1H), 4.55 (m, 1H), 4.75 (dd, 1H), 5.19 (t, 1H), 5.29 (d, 1H), 5.30 (br., 1H), 5.58 (m, 2H), 6.68 (m, 3H), 6.89 (d, 1H), 6.93 (m, 3H), 6.94 (br., 1H), 6.98 (d, 1H), 7.12 (br., 1H), 7.20 (br., 2H), 7.23 (m, 2H), 7.42 (m, 2H), 7.54 (d, 1H), 7.58 (d, 1H), 8.32 (br., 1H), 9.18 (br., 1H), 9.20 (m, 2H), 9.50 (br., 1H).
¹³C-NMR (125.77 MHz, *d₆*-DMSO): δ = 10.3, 15.3, 19.0, 19.2, 19.6, 20.0, 20.9, 22.0, 22.4, 23.0, 23.2, 24.3, 24.4, 25.0, 25.4, 26.0, 27.8, 30.9, 35.4, 39.5, 40.8, 40.9, 41.6, 44.1, 51.5, 52.7, 55.9, 56.2, 56.4, 57.9, 58.8, 60.2, 61.1, 62.6, 70.1, 71.6, 71.7, 75.5, 128.1, 128.6, 136.7, 156.8, 168.2, 170.1, 170.4, 171.2, 171.5, 171.9, 172.2, 172.4, 173.7.

Die Zuordnung der Signale erfolgte nach der in der Literatur beschriebenen Zuordnung (T. Kato, H. Hinoo, Y. Terui, *J*. *Antibiot.,* **1988**, *61*, 719-725).

### Beispiel 2A

### N¹-tert-Butoxycarbonyl-lysobactin-acetat

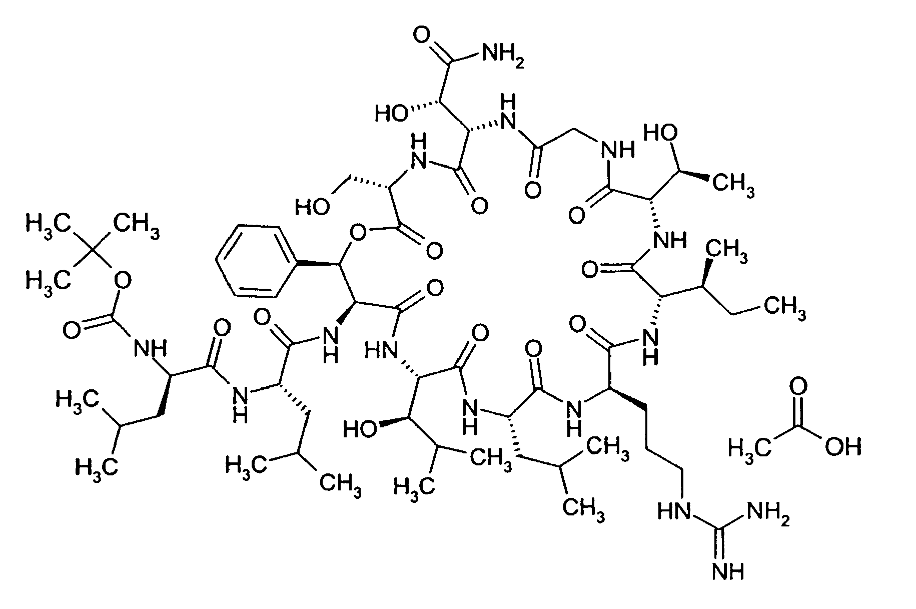

10.0 g (5.18 mmol; 78%ig) Lysobactin (Beispiel 1A) werden in 21 eines Gemisches aus *tert-*Butanol/Puffer-Lösung (pH6)/Puffer-Lösung (pH7) (2:1:1) gelöst. Bei 20°C werden zunächst 6.7 mmol (1.2 Äquivalente) Di-*tert*-butyldicarbonat in 5 ml *tert*-Butanol/Puffergemisch und anschließend 6.7 mmol (1.2 Äquivalente) DIEA in 5 ml *tert*-Butanol/Puffergemisch zugetropft. Nach 12 Stunden beobachtet man mittels analytischer HPLC (Methode 1) keinen vollständigen Umsatz. Es werden weitere 6.7 mmol (1.2 Äquivalente) Di-*tert*-butyldicarbonat in 5 ml *tert*-Butanol/Puffergemisch zugetropft. Nach einer Stunde ist die Umsetzung vollständig, woraufhin 2.58 ml (45 mmol) Essigsäure zugegeben werden. Das Rohprodukt wird eingeengt, lyophilisiert, gelchromatographisch grobgereinigt (Methode 9; Methanol:Aceton:Essigsäure / 80:20:0.1) und mittels präparativer HPLC (Methode 10) feingereinigt. Man erhält 5.76 g (74% d. Th.) Produkt.
[α]²⁰_{Na} = -56° (c = 0.21 in Methanol).
HPLC/UV-Vis (Methode 2): Rₜ = 4.7 min.
HPLC (Methode 1): Rₜ = 4.29 min
LC-MS (Methode 3): Rₜ = 2.02 min;
MS (ESIpos.): m/z (%) = 639 (100), 1376 (40) [M - CO₂- C₄H₈ + H]⁺
MS (ESIneg.): m/z (%) = 687 (100), 1374 (5) [M - CO₂ - C₄H₈ - H]⁻.
HR-TOF-MS (Methode 8): C₆₃H₁₀₆N₁₅O₁₉ ber. 1376.7789, gef. 1376.7820;

### Ausfuhrungsbeispiele

### Beispiele 1 bis 3

250 mg (0.17 mmol) Beispiel 2A werden nach der Allgemeinen Arbeitsvorschrift 2 umgesetzt. Nach der Reaktion mit Phenylisocyanat werden 105 mg eines Gemisches aus mehreren Mono-substituierten Boc-geschützten Lysobactin Derivaten isoliert.
HPLC (Methode 2): Rₜ = 4.54 min; λₘₐₓ (qualitativ) = 208 nm (s), 234 nm (m);
LC-MS (Methode 3): Rₜ = 2.47 min;
MS (ESIpos.): m/z (%) = 698 (100) [M - CO₂ - C₄H₈+ 2H]²⁺, 748 (5) [M + 2H]²⁺, 1495 (100) [M + H]⁺;
MS (ESIneg.): m/z (%) = 687 (80), 1493 (100) [M - H]⁻.

Das Gemisch wird als Suspension in 3 ml DCM vorgelegt, mit 1 ml TFA versetzt und bei RT 15 min gerührt, bis die analytische HPLC (Methode 1) vollständigen Umsatz anzeigt. Das Rohprodukt wird im Vakuum von Lösungsmittel befreit. Abschließend wird das Rohprodukt gelchromatographisch grobgereinigt (Methode 9; Methanol:Aceton:TFA / 80:20:0.1) und mittels präparativer HPLC (Methode 11) feingereinigt und getrennt. Man erhält 41.4 mg (17% d. Th.) Beispiel 1, 30.1 mg (25% d. Th.) Beispiel 2 und 5.8 mg (3% d. Th.) Beispiel 3.

### Beispiel 1

### N^{ω6}-(Phenylaminocarbonyl)-lysobactin-trifluoracetat

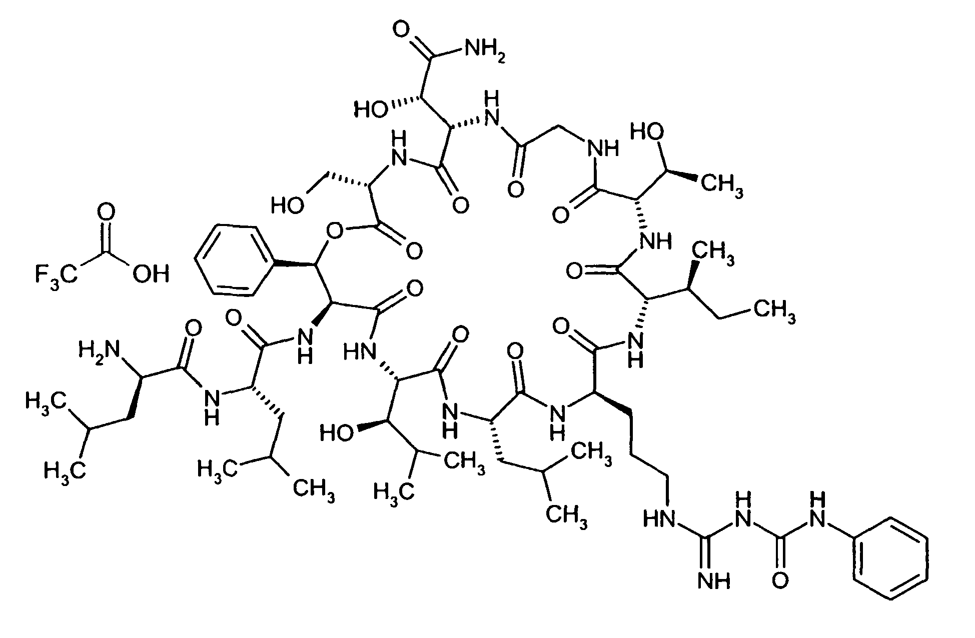

HPLC/UV-Vis (Methode 2): Rₜ = 4.1 min.
λₘₐₓ (qualitativ) = 220 nm (s), 240 nm (m).
HPLC (Methode 1): Rₜ = 3.70 min
HPLC (Methode 4): Rₜ = 8.36 min
LC-MS (Methode 3): Rₜ = 1.52 min;
MS (ESIpos.): m/z (%) = 698 (100) [M + 2H]²⁺, 1395 (5) [M + H]⁺
MS (ESIneg.): m/z (%) = 696 (100), 1393 (30) [M - H]⁻.
HR-TOF-MS (Methode 8): C₆₅H₁₀₃N₁₆O₁₈ (MH⁺) ber. 1395.7636, gef. 1395.7604;

Zur Aminosäure-Sequenzbestimmung wird eine analytische Probe des Produktes nach der Allgemeinen Arbeitsvorschrift 3 hydrolysiert und analysiert (Methode 7).

### Beispiel 2

### O^{3.10}-(Phenylaminocarbonyl)-lysobactin-bistrifluoracetat

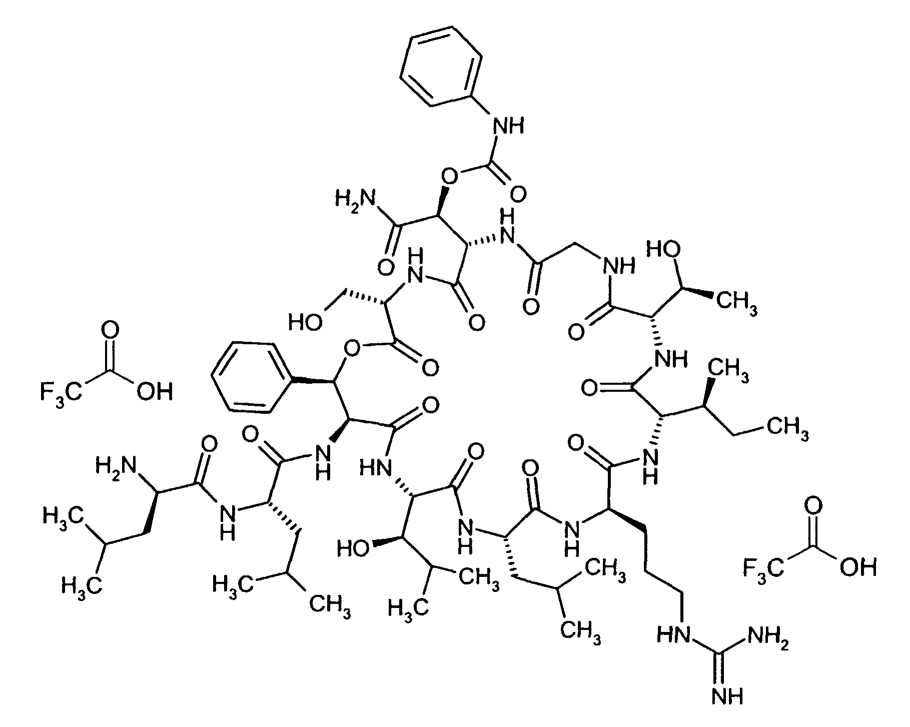

HPLC/UV-Vis (Methode 1): Rₜ = 4.1 min.
λₘₐₓ (qualitativ) = 242 nm (m).
HPLC (Methode 2): Rₜ = 3.70 min
HPLC (Methode 4): Rₜ = 8.70 min
LC-MS (Methode 3): Rₜ = 1.60 min;
MS (ESIpos.): m/z (%) = 698 (100) [M + 2H]²⁺, 1395 (5) [M + H]⁺
MS (ESIneg.): m/z (%) = 636 (100), 1393 (5) [M - H]⁻.
HR-TOF-MS (Methode 8): C₆₅H₁₀₃N₁₆O₁₈ (MH⁺) ber. 1395.7636, gef. 1395.7653;

Zur Aminosäure-Sequenzbestimmung wird eine analytische Probe des Produktes nach der Allgemeinen Arbeitsvorschrift 3 hydrolysiert und analysiert (Methode 7).

### Beispiel 3

### O^{3.11}-(Phenylaminocarbonyl)-lysobactin-bistrifluoracetat

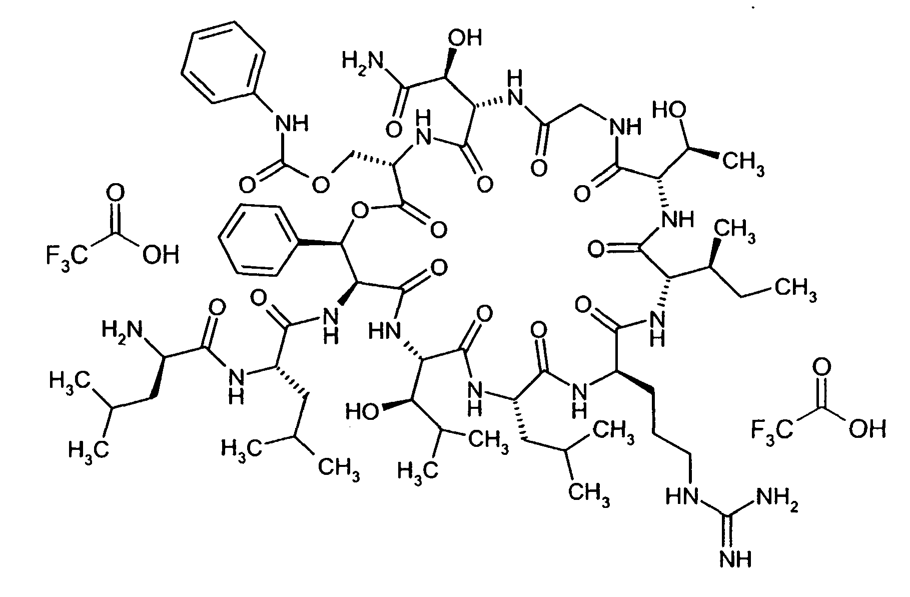

HPLC/UV-Vis (Methode 1): Rₜ = 4.3 min.
λₘₐₓ (qualitativ) = 232 nm (m).
HPLC (Methode 2): Rₜ = 3.79 min
HPLC (Methode 4): Rₜ = 8.89 min
LC-MS (Methode 3): Rₜ = 1.72 min;
MS (ESIpos.): m/z (%) = 698 (100) [M + 2H]²⁺, 1395 (5) [M + H]⁺
MS (ESIneg.): m/z (%) = 136 (10), 637 (60), 696 (30), 1393 (30) [M - H]⁻.
HR-TOF-MS (Methode 8): C₆₅H₁₀₃N₁₆O₁₈ (MH⁺) ber. 1395.7636, gef. 1395.7639;

Zur Aminosäure-Sequenzbestimmung wird eine analytische Probe des Produkts nach der Allgemeinen Arbeitsvorschrift 3 hydrolysiert und analysiert (Methode 7).

### Beispiel 4 bis 6

500 mg (0.35 mmol) Beispiel 2A werden nach der Allgemeinen Arbeitsvorschrift 2 umgesetzt. Nach der Reaktion mit 3-Methoxyphenylisocyanat werden 125.7 mg eines Gemisches aus mehreren mono-substituierten Derivaten von Beispiel 2A isoliert.
HPLC (Methode 1): Rₜ = 4.55 min;
LC-MS (Methode 3): Rₜ = 2.21 min;
MS (ESIpos.): m/z (%) = 713 (100) [M - Boc + 2H]²⁺, 763 (5) [M + 2H]²⁺, 1525 (60) [M + H]⁺;
MS (ESIneg.): m/z (%) = 761 (100), 1523 (15) [M-H]⁻).

Das Gemisch wird als Suspension in 4.5 ml DCM vorgelegt, mit 1.5 ml TFA versetzt und bei RT 10 min gerührt, bis durch analytische HPLC (Methode 1) vollständiger Umsatz anzeigt wird. Das Rohprodukt wird im Vakuum von Lösungsmittel befreit. Abschließend wird das Rohprodukt gelchromatographisch grobgereinigt (Methode 9; Methanol:Aceton:TFA / 80:20:0.1) und mittels präparativer HPLC (Methode 13) feingereinigt und getrennt. Man erhält 30.5 mg (5% d. Th.) Beispiel 4, 30.5 mg (5% d. Th.) Beispiel 5 und 30.5 mg (4% d. Th.) Beispiel 6.

### Beispiel 4

### N^{ω6}-(3-Methoxyphenylaminocarbonyl)-lysobactin-trifluoracetat

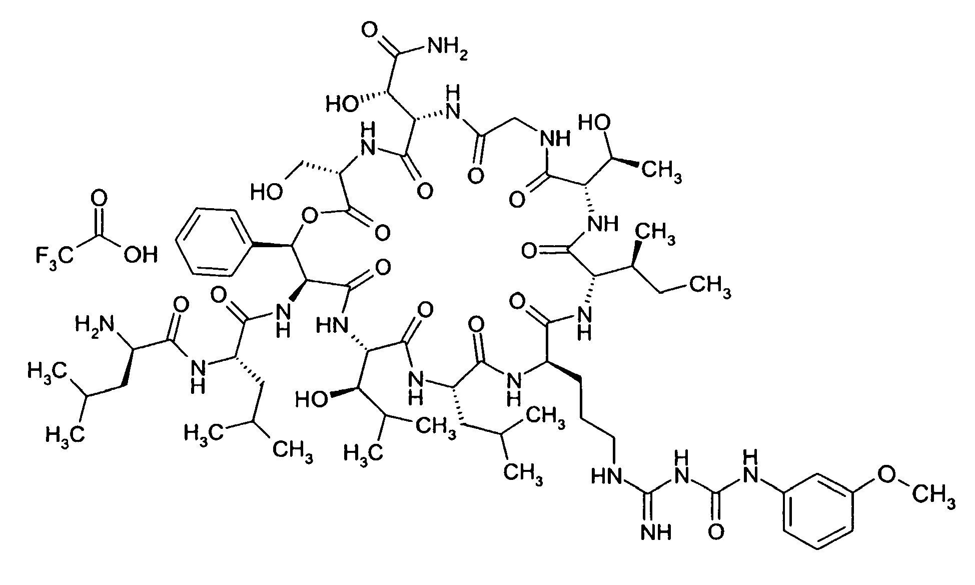

HPLC/UV-Vis (Methode 2): Rₜ = 4.1 min.
λₘₐₓ (qualitativ) = 210 nm (m), 245 nm (w).
HPLC (Methode 5): Rₜ = 4.88 min.
LC-MS (Methode 3): Rₜ = 1.56 min;
MS (ESIpos.): m/z (%) = 713 (100) [M + 2H]²⁺, 1425 (10) [M + H]⁺
MS (ESIneg.): m/z (%) = 711 (100), 1423 (20) [M - H]⁻.
HR-TOF-MS (Methode 8): C₆₆H₁₀₅N₁₆O₁₉ (MH⁺) ber. 1425.7742, gef. 1425.7766;

Zur Aminosäure-Sequenzbestimmung wird eine analytische Probe des Produktes nach der Allgemeinen Arbeitsvorschrift 3 hydrolysiert und analysiert (Methode 7).

### Beispiel 5

### O^{3.10}-(3-Methoxyphenylaminocarbonyl)-lysobactin-bistrifluoracetat

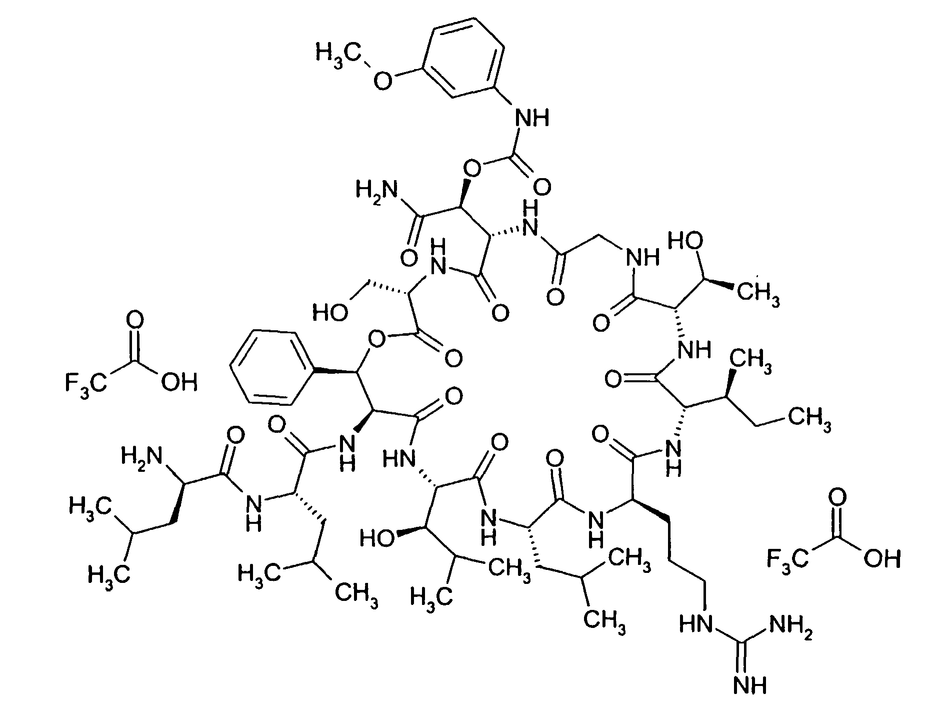

HPLC/UV-Vis (Methode 2): Rₜ = 4.2 min.
λₘₐₓ (qualitativ) = 210 nm (m), 245 nm (w).
HPLC (Methode 5): Rₜ = 6.71 min.
LC-MS (Methode 3): Rₜ = 1.67 min;
MS (ESIpos.): m/z (%) = 713 (100) [M + 2H]²⁺, 1425 (10) [M + H]⁺
HR-TOF-MS (Methode 8): C₆₆H₁₀₅N₁₆O₁₉ (MH⁺) ber. 1425.7742, gef. 1425.7761;

Zur Aminosäure-Sequenzbestimmung wird eine analytische Probe des Produkts nach der Allgemeinen Arbeitsvorschrift 3 hydrolysiert und analysiert (Methode 7).

### Beispiel 6

### O^{3.11}-(3-Methoxyphenylaminocarbonyl)-lysobactin-bistrifluoracetat

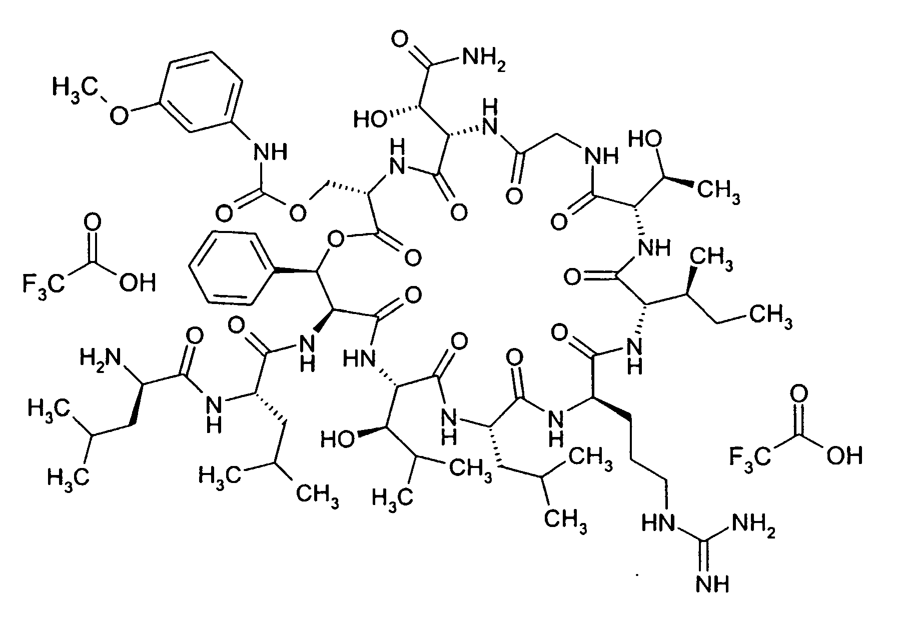

HPLC/UV-Vis (Methode 2): Rₜ = 4.3 min.
λₘₐₓ (qualitativ) = 210 nm (m), 245 nm (w).
HPLC (Methode 5): Rₜ = 8.21 min.
LC-MS (Methode 3): Rₜ = 1.71 min;
MS (ESIpos.): m/z (%) = 713 (100) [M + 2H]²⁺, 1425 (10) [M + H]⁺
MS (ESIneg.): m/z (%) = 637 (100), 711 (80), 1423 (20) [M - H]⁻.
HR-TOF-MS (Methode 8): C₆₆H₁₀₅N₁₆O₁₉ (MH⁺) ber. 1425.7742, gef. 1425.7789;

Zur Aminosäure-Sequenzbestimmung wird eine analytische Probe des Produktes nach der Allgemeinen Arbeitsvorschrift 3 hydrolysiert und analysiert (Methode 7).

### Beispiel 7 bis 9

500 mg (0.35 mmol) Beispiel 2A werden nach der Allgemeinen Arbeitsvorschrift 2 umgesetzt. Nach der Reaktion mit 3-Bromphenylisocyanat werden 202 mg eines Gemisches aus mehreren mono-substituierten Derivaten von Beispiel 2A isoliert.
HPLC (Methode 1): Rₜ = 4.62 min;
LC-MS (Methode 3): Rₜ = 2.28 min;
MS (ESIpos.): m/z (%) = 738 (100) [M - Boc + 2H]²⁺, 788 (5) [M + 2H]²⁺, 1574 (5) [M + H]⁺;
MS (ESIneg.): m/z (%) = 786 (100), 1573 (10) [M - H]⁻).

Das Gemisch wird als Suspension in 6 ml DCM vorgelegt, mit 2 ml TFA versetzt und bei RT 15 min gerührt, bis durch analytische HPLC (Methode 1) vollständiger Umsatz anzeigt wird. Das Rohprodukt wird im Vakuum von Lösungsmittel befreit. Abschließend wird das Rohprodukt gelchromatographisch grobgereinigt (Methode 9; Methanol:Aceton:TFA / 80:20:0.1) und mittels präparativer HPLC (Methode 12) feingereinigt und getrennt. Man erhält 105 mg (19% d. Th.) Beispiel 7, 51 mg (9% d. Th.) Beispiel 8 und 10 mg (2% d. Th.) Beispiel 9.

### Beispiel 7

### N^{ω}⁶-(3-Bromphenylaminocarbonyl)-lysobactin-trifluoracetat

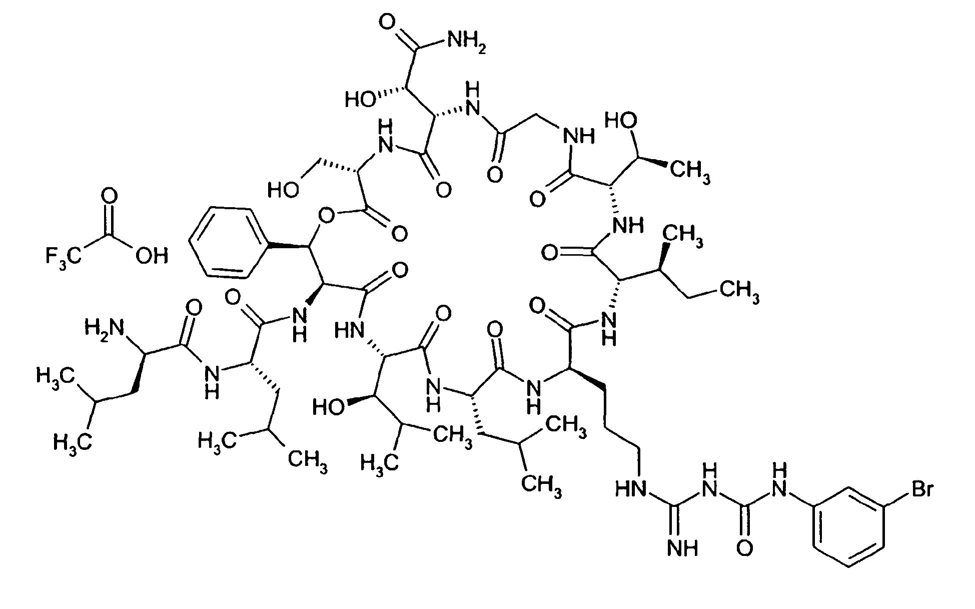

HPLC/UV-Vis (Methode 2): Rₜ = 4.2 min.
λₘₐₓ (qualitativ) = 220 (m), 246 nm (w).
HPLC (Methode 1): Rₜ = 3.73 min
HPLC (Methode 6): Rₜ = 3.39 min
LC-MS (Methode 3): Rₜ = 1.61 min;
MS (ESIpos.): m/z (%) = 652 (50), 738 (100) [M + 2H]²⁺, 1473 (10) [M + H]⁺
MS (ESIneg.): m/z (%) = 736 (100), 1471 (15) [M - H]⁻.
HR-TOF-MS (Methode 8): C₆₅H₁₀₂N₁₆O₁₈ (MH⁺) ber. 1473.6741, gef. 1473.6750.

Zur Aminosäure-Sequenzbestimmung wird eine analytische Probe des Produktes nach der Allgemeinen Arbeitsvorschrift 3 hydrolysiert und analysiert (Methode 7).

### Beispiel 8

### O^{3.10}-(3-Bromphenylaminocarbonyl)-lysobactin-bistrifluoracetat

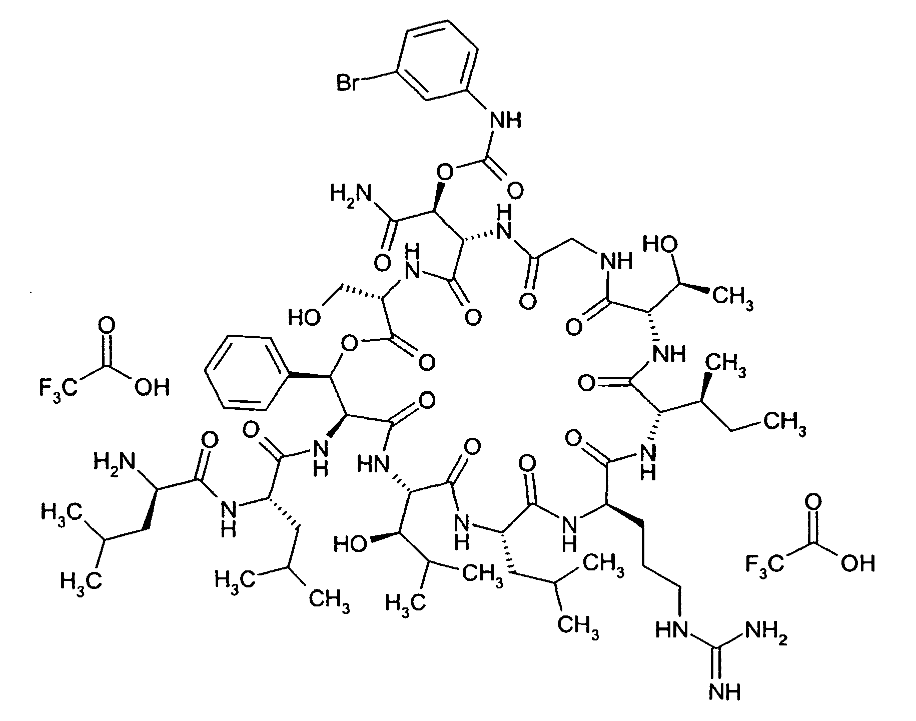

HPLC/UV-Vis (Methode 2): Rₜ = 4.4 min.
λₘₐₓ (qualitativ) = 200 (m), 240 nm (w).
HPLC (Methode 1): Rₜ = 3.86 min
HPLC (Methode 6): Rₜ = 4.82 min
LC-MS (Methode 3): Rₜ = 1.71 min;
MS (ESIpos.): m/z (%) = 738 (100) [M + 2H]²⁺, 1473 (5) [M + H]⁺
MS (ESIneg.): m/z (%) = 637 (100), 736 (10), 1471 (5) [M - H]⁻.
HR-TOF-MS (Methode 8): C₆₅H₁₀₂N₁₆O₁₈ (MH⁺) ber. 1473.6741, gef. 1473.6781.

Zur Aminosäure-Sequenzbestimmung wird eine analytische Probe des Produktes nach der Allgemeinen Arbeitsvorschrift 3 hydrolysiert und analysiert (Methode 7).

**Tabelle 1: ¹H-NMR (500 MHz, d₅-Pyridin, 302 K) und ¹³C-NMR (d₅-Pyridin) Daten:**

| **Rest** | **NH** | **CO** | **CH-α** | **CH-β** | **CH-χ** | **Weitere Reste** |
|---|---|---|---|---|---|---|
| **Leu1** | - | 173.96 | 4.88 | 2.08;2.21 | 2.02 | Me 0.97(22.87) |
| | | | (52.70) | (42.06) | (25.31) | Me 0.86(21.58) |
| **Leu2** | 11.1 | 176.82 | 4.51 | 1.84;2.33 | 2.17 | Me 0.78(23.55) |
| | 7 | | (57.06) | (40.32) | (25.36) | Me 1.04(20.66) |
| **Ph-Ser** | 9.60 | 173.63 | 6.25 | 7.38 | | Ph-o:8.14(128.56) |
| | | | (62.70) | (75.41) | | Ph-m:7.57(129.31) |
| | | | | | | Ph-p:7.30(129.31) |
| | | | | | | Ph-i: 137.28 |
| **Hy-Leu** | 9.37 | 173.65 | 4.33 | 3.90 | 2.39 | Me 0.65(19.17) |
| | | | (60.93) | (75.50) | (31.22) | Me 1.07(20.17) |
| **Leu3** | 7.94 | 174.33 | 5.11 | 2.24 | 2.45 | Me 0.98(21.05) |
| | | | (53.45) | (42.21) | (25.02) | Me 1.10(24.41) |
| **Arg** | 7.69 | 173.39 | 4.61 | 2.16;2.21 | 1.40;2.14 | CH₂-δ:3.18;3.34(41.49) |
| | | | (56.30) | (29.17) | (27.30) | NH-δ:8.96 |
| | | | | | | C=N : 158.26 |
| **Ile** | 8.26 | 172.71 | 4.69 | 2.29 | 1.30;2.01 | |
| | | | (61.09) | (36.69) | (26.90) | |
| **Thr** | 7.76 | 174.54 | 5.41 | 4.38 | | Me 1.72(22.02) |
| | | | (58.64) | (70.80) | | |
| **Gly** | 11.0 | 169.45 | 4.01;4.63 | | | |
| | 2 | | (44.82) | | | |
| **Hy-Asn** | 8.56 | 168.79 | 6.11 | 6.50 | | CONH₂ 170.68 |
| | | | (55.80) | (74.53) | | O-CO-N 152.45 |
| | | | | | | C-1 140.66 |
| | | | | | | C-2 7.81(121.46) |
| | | | | | | C-3 122.89 |
| | | | | | | C-4 7.32(117.19) |
| | | | | | | C-5 6.97(130.79) |
| | | | | | | C-6 7.06(126.07) |
| **Ser** | 8.13 | 168.49 | 5.33 | 4.12;4.18 | | |
| | | | 56.42) | (62.85) | | |

### Beispiel 9

### O^{3.11}-(3-Bromphenylaminocarbonyl)-lysobactin-bistrifluoracetat

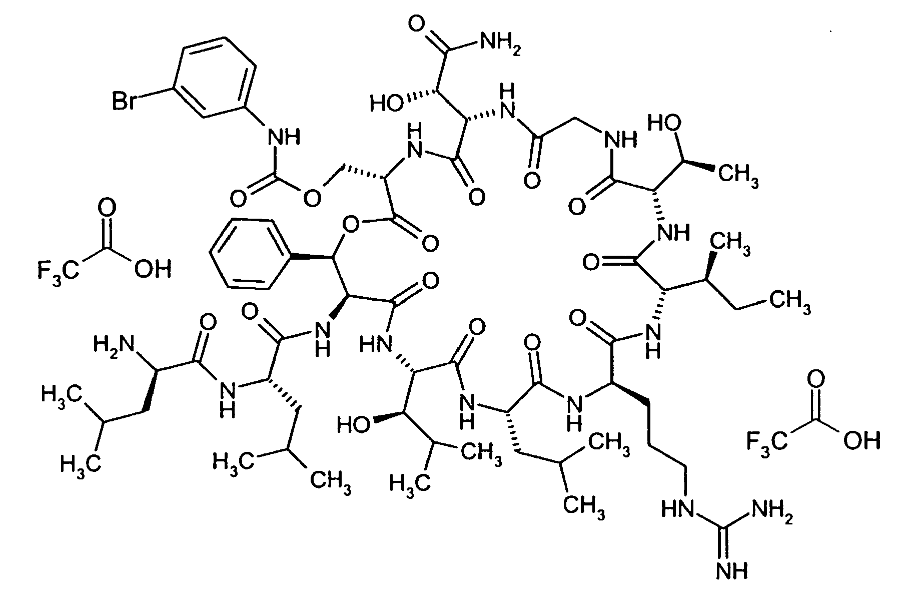

HPLC/CTV-Vis (Methode 2): Rₜ = 4.4 min.
λₘₐₓ (qualitativ) = 200 (m), 240 nm (w).
HPLC (Methode 1): Rₜ = 3.92 min
HPLC (Methode 6): Rₜ = 6.01 min
LC-MS (Methode 3): Rₜ = 1.83 min;
MS (ESIpos.): m/z (%) = 738 (100) [M + 2H]²⁺, 1473 (5) [M + H]⁺
MS (ESIneg.): m/z (%) = 637 (100), 736 (40), 1471 (10) [M - H]⁻.
HR-TOF-MS (Methode 8): C₆₅H₁₀₂N₁₆O₁₈ (MH⁺) ber. 1473.6741, gef. 1473.6766.

Zur Aminosäure-Sequenzbestimmung wird eine analytische Probe des Produktes nach der Allgemeinen Arbeitsvorschrift 3 hydrolysiert und analysiert (Methode 7).

### Beispiel 10

### N^{ω6}-(3-(N,N-Dimethylsulfonyl)-phenylaminocarbonyl)-lysobactin-trifluoracetat

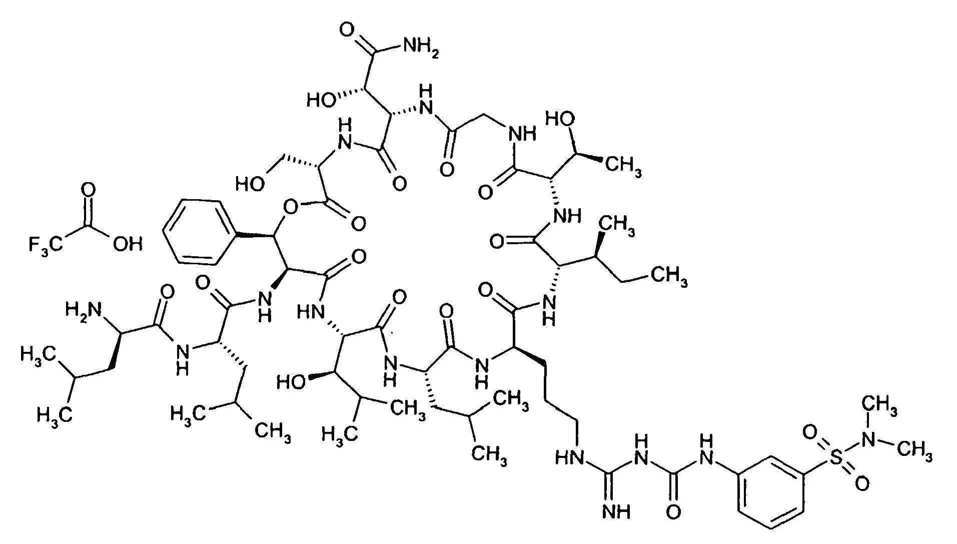

500 mg (0.35 mmol) Beispiel 2A werden nach der Allgemeinen Arbeitsvorschrift 2 umgesetzt. Nach der Reaktion mit 3-(*N,N-*Dimethylsulfonyl)-phenylisocyanat werden 40 mg eines Gemisches aus mehreren mono-substituierten Derivaten von Beispiel 2A isoliert.
HPLC (Methode 1): Rₜ = 4.48 min;
HPLC (Methode 2): Rₜ = 5.0 min; LC-MS (Methode 3): Rₜ = 2.20 min;
MS (ESIpos.): m/z (%) = 752 (100) [M - Boc + 2H]²⁺, 1602 (10) [M + H]⁺;
MS (ESIneg.): m/z (%) = 687 (100), 800 (35), 1600 (10) [M - H]⁻);
HR-TOF-MS (Methode 8): C₇₂H₁₁₆N₁₇O₂₂ (MH⁺) ber. 1602.8202, gef. 1602.8148).

Das Gemisch wird als Suspension in 6 ml DCM vorgelegt, mit 2 ml TFA versetzt und bei RT 15 min gerührt, bis durch analytische HPLC (Methode 1) vollständiger Umsatz anzeigt wird. Das Rohprodukt wird im Vakuum von Lösungsmittel befreit. Abschließend wird das Rohprodukt gelchromatographisch grobgereinigt (Methode 9; Methanol:Aceton:TFA / 80:20:0.1) und mittels präparativer HPLC (Methode 14) feingereinigt und getrennt. Man erhält 12.5 mg (2% d. Th.) der Titelverbindung (Beispiel 10).
HPLC/CTV-Vis (Methode 2): Rₜ = 4.2 min.
λₘₐₓ (qualitativ) = 200 (m), 250 nm (w).
HPLC (Methode 1): Rₜ = 3.70 min;
LC-MS (Methode 3): Rₜ = 1.72 min;
MS (ESIpos.): m/z (%) = 652 (60), 752 (100) [M + 2H]²⁺, 1502 (5) [M + H]⁺
MS (ESIneg.): m/z (%) = 637 (30), 750 (100), 1500 (10) [M - H]⁻.
HR-TOF-MS (Methode 8): C₆₇H₁₀₈N₁₇O₂₀ (MH⁺) ber. 1502.7677, gef. 1502.7721.

### Beispiele 11 bis 13

500 mg (0.35 mmol) Beispiel 2A werden nach der Allgemeinen Arbeitsvorschrift 2 umgesetzt. Nach der Reaktion mit 4-Morpholino-phenylisocyanat werden 66 mg eines Gemisches aus mehreren mono-substituierten Derivaten von Beispiel 2A isoliert.
HPLC (Methode 1): Rₜ = 4.35 min;
HPLC (Methode 2): Rₜ = 4.7 min; λₘₐₓ (qualitativ) = 200 nm (s), 256 nm (m);
LC-MS (Methode 3): Rₜ = 2.37 min;
MS (ESIpos.): m/z (%) = 741 (75) [M - Boc + 2H]²⁺, 791 (100) [M + 2H]²⁺;
MS (ESIneg.): m/z (%) = 687 (50), 789 (100);
HR-TOF-MS (Methode 8): C₇₄H₁₁₈N₁₇O₂₁ (MH⁺) ber. 1580.8688, gef. 1580.8671).

Das Gemisch wird als Suspension in 5 ml DCM vorgelegt, mit 1.7 ml TFA versetzt und bei RT 15 min gerührt, bis durch analytische HPLC (Methode 1) vollständiger Umsatz anzeigt wird. Das Rohprodukt wird im Vakuum von Lösungsmittel befreit. Abschließend wird das Rohprodukt gelchromatographisch grobgereinigt (Methode 9; Methanol:Aceton:TFA / 80:20:0.1) und mittels präparativer HPLC (Methode 15) feingereinigt und getrennt. Man erhält 5.7 mg (9% d. Th.) Beispiel 11, 3.7 mg (5% d. Th.) Beispiel 12 und 1.2 mg (2% d. Th.) Beispiel 13.

### Beispiel 11

### N^{ω.6}-(4-Morpholino-phenylaminocarbonyl)-lysobactin-trifluoracetat

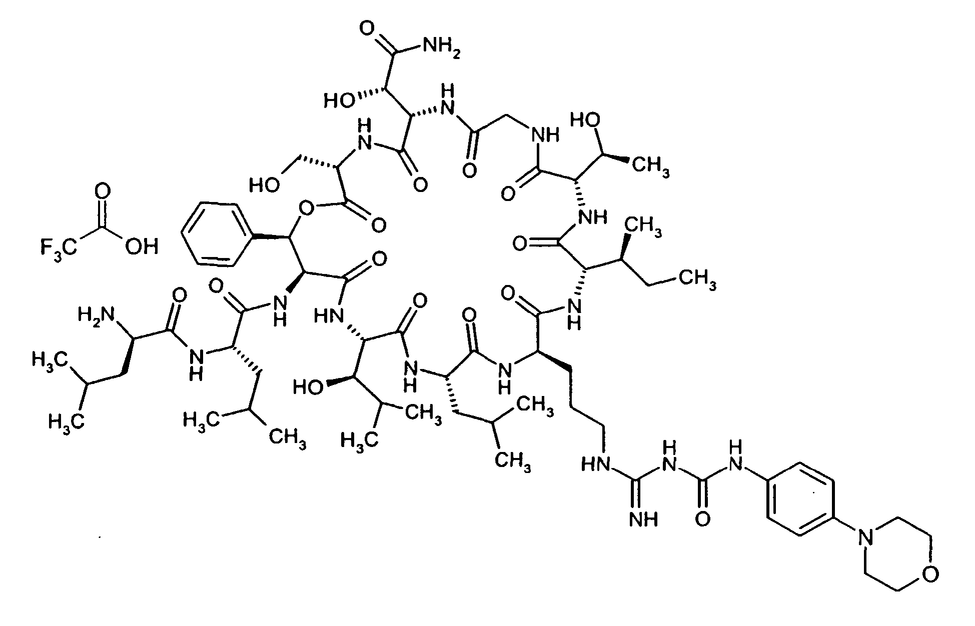

HPLC/UV-V is (Methode 2): Rₜ = 4.0 min.
λₘₐₓ (qualitativ) = 200 nm (s), 246 nm (m).
HPLC (Methode 1): Rₜ = 3.62 min.
LC-MS (Methode 3): Rₜ = 1.55 min;
MS (ESIpos.): m/z (%) = 741 (100) [M + 2H]²⁺, 1480 (5) [M + H]⁺
MS (ESIneg.): m/z (%) = 740 (100), 1478 (10) [M - H]⁻.
HR-TOF-MS (Methode 8): C₆₉H₁₁₀N₁₇O₁₉ (MH⁺) ber. 1480.8164, gef. 1480.8112.

Zur Aminosäure-Sequenzbestimmung wird eine analytische Probe des Produktes nach der Allgemeinen Arbeitsvorschrift 3 hydrolysiert und analysiert (Methode 7).

### Beispiel 12

### O^{3.10}-(4-Morpholino-phenylaminocarbonyl)-lysobactin-bistrifluoracetat

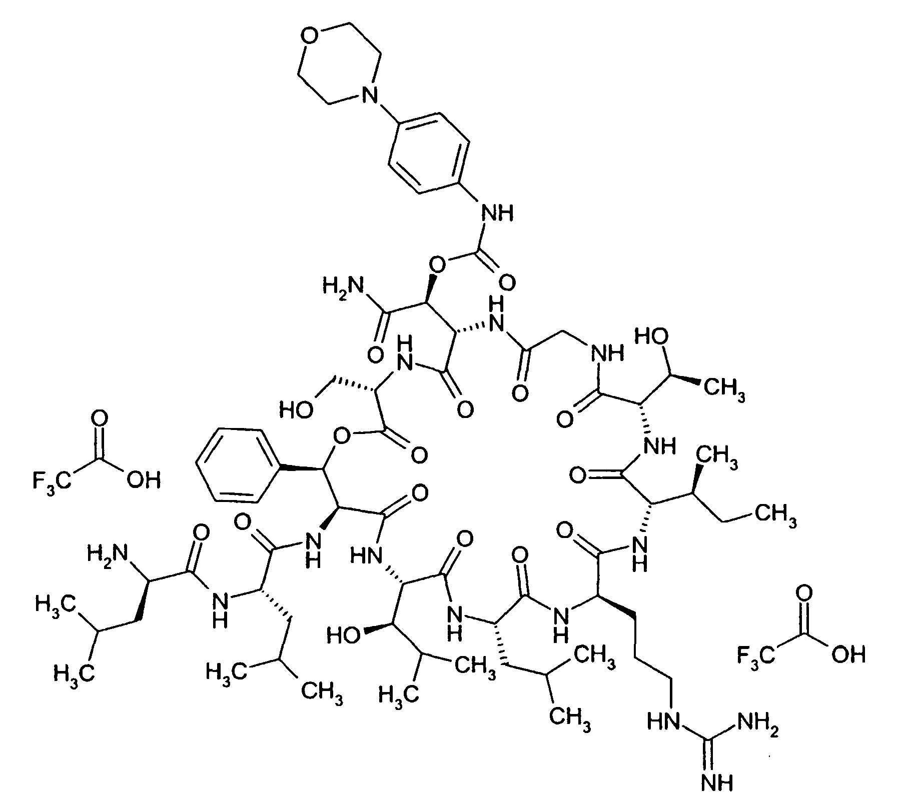

HPLC/UV-Vis (Methode 2): Rₜ = 4.0 min.
λₘₐₓ (qualitativ) = 200 (s), 242 nm (m).
HPLC (Methode 1): Rₜ = 3.66 min
LC-MS (Methode 3): Rₜ = 1.63 min;
MS (ESIpos.): m/z (%) = 741 (100) [M + 2H]²⁺, 1480 (5) [M + H]⁺
MS (ESIneg.): m/z (%) = 637 (100).
HR-TOF-MS (Methode 8): C₆₉H₁₁₀N₁₇O₁₉ (MH⁺) ber. 1480.8164, gef. 1480.8188.

Zur Aminosäure-Sequenzbestimmung wird eine analytische Probe des Produktes nach der Allgemeinen Arbeitsvorschrift 3 hydrolysiert und analysiert (Methode 7).

### Beispiel 13

### O^{3.11}-(4-Morpholino-phenylaminocarbonyl)-lysobactin-bistrifluoracetat

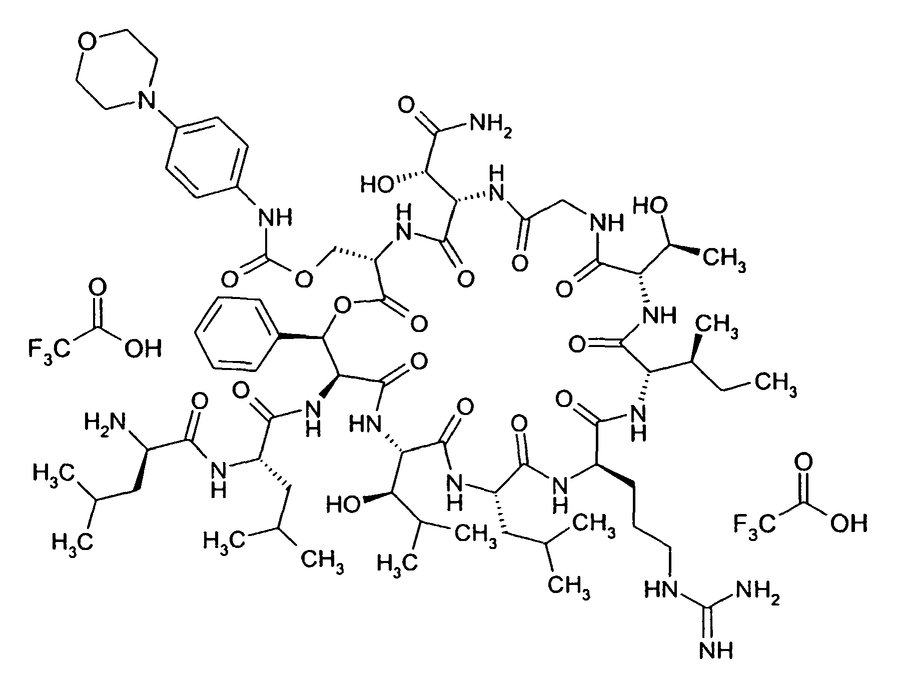

HPLC/UV-Vis (Methode 2): Rₜ = 4.0 min.
λₘₐₓ (qualitativ) = 200 (s), 242 nm (m).
HPLC (Methode 1): Rₜ = 3.66 min
LC-MS (Methode 3): Rₜ = 1.73 min;
MS (ESIpos.): m/z (%) = 494 (75), 741 (100) [M + 2H]²⁺.
HR-TOF-MS (Methode 8): C₆₉H₁₁₀N₁₇O₁₉ (MH⁺) ber. 1480.8164, gef. 1480.8164.

Zur Aminosäure-Sequenzbestimmung wird eine analytische Probe des Produktes nach der Allgemeinen Arbeitsvorschrift 3 hydrolysiert und analysiert (Methode 7).

### Beispiele 14 und 15

300 mg (0.21 mmol) Beispiel 2A werden nach der Allgemeinen Arbeitsvorschrift 1 umgesetzt. Nach der Reaktion mit Phenylcarbonsäurechlorid werden 221 mg eines Gemisches aus zwei mono-substituierten Derivaten von Beispiel 2A isoliert.
HPLC (Methode 1): Rₜ = 4.52 min;
LC-MS (Methode 3): Rₜ = 2.49 min;
MS (ESIpos.): m/z (%) = 1480 (40) [M + H]⁺.

Das Gemisch wird als Suspension in 3 ml DCM vorgelegt, mit 1 ml TFA versetzt und bei RT 10 min gerührt, bis durch analytische HPLC (Methode 1) vollständiger Umsatz anzeigt wird. Das Rohprodukt wird im Vakuum von Lösungsmittel befreit. Abschließend wird das Rohprodukt gelchromatographisch grobgereinigt (Methode 9; Methanol:Aceton:TFA / 80:20:0.1) und mittels präparativer HPLC (Methode 11) feingereinigt. Man erhält 163 mg (73% d. Th.) der Beispiele 14 und 15 als 1:1 Gemisch.
HPLC (Methode 1): Rₜ = 3.88 min
LC-MS (Methode 3): Rₜ = 1.83 min;
MS (ESIpos.): m/z (%) = 199 (100), 691 (50), 1380 (10) [M + H]⁺

Zur Aminosäure-Sequenzbestimmung wird eine analytische Probe des Produktes nach der Allgemeinen Arbeitsvorschrift 3 hydrolysiert und analysiert (Methode 7) (1/1 Gemisch).

### Beispiel 14

### O^{3.10}-(Phenylcarbonyl)-lysobactin-bistrifluoracetat

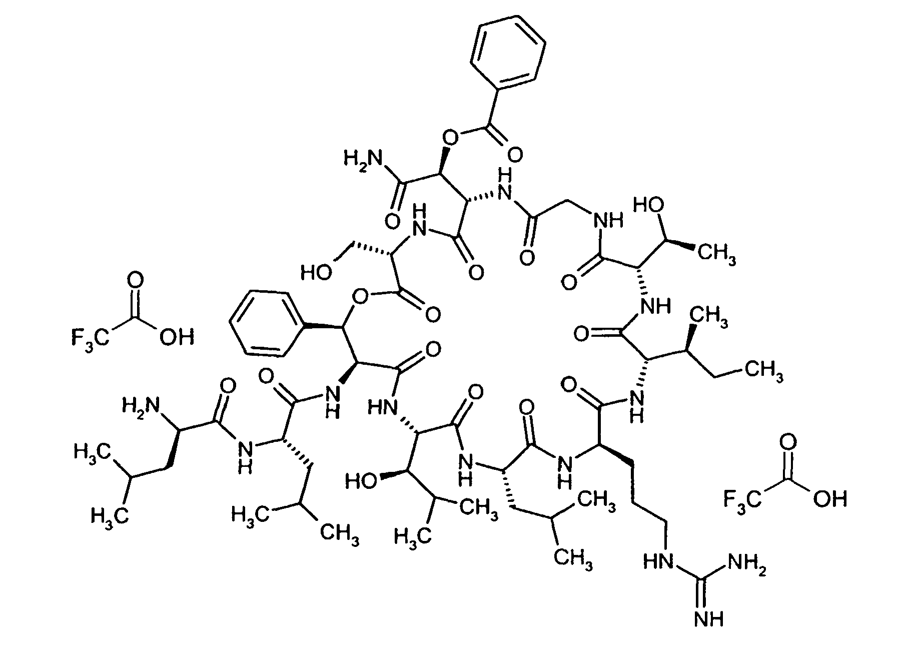

### Beispiel 15

### O^{3.11}-(Phenylcarbonyl)-lysobactin-bistrifluoracetat

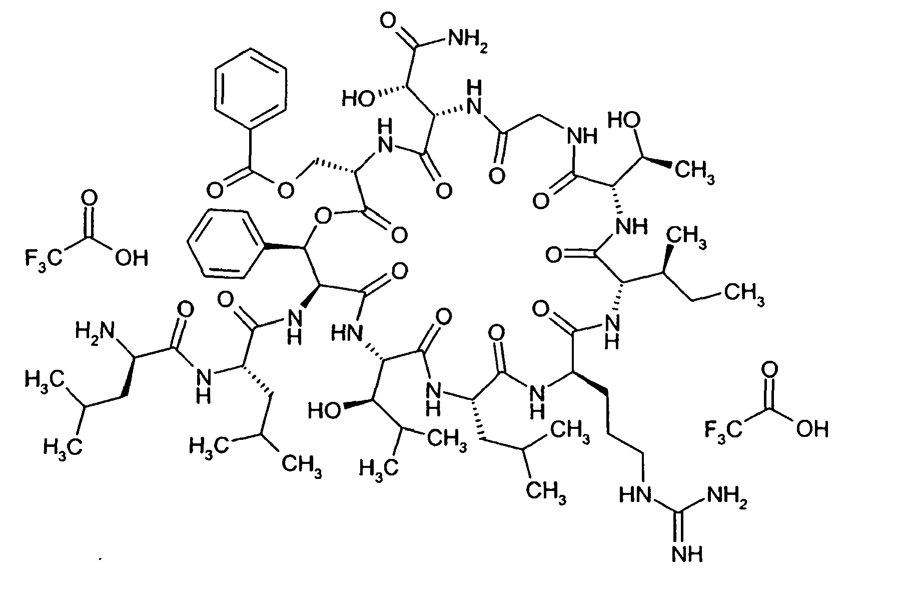

### Beispiel 16

### O^{3.10},O^{3.11}-Di-(phenylcarbonyl)-lysobactin-bisacetat

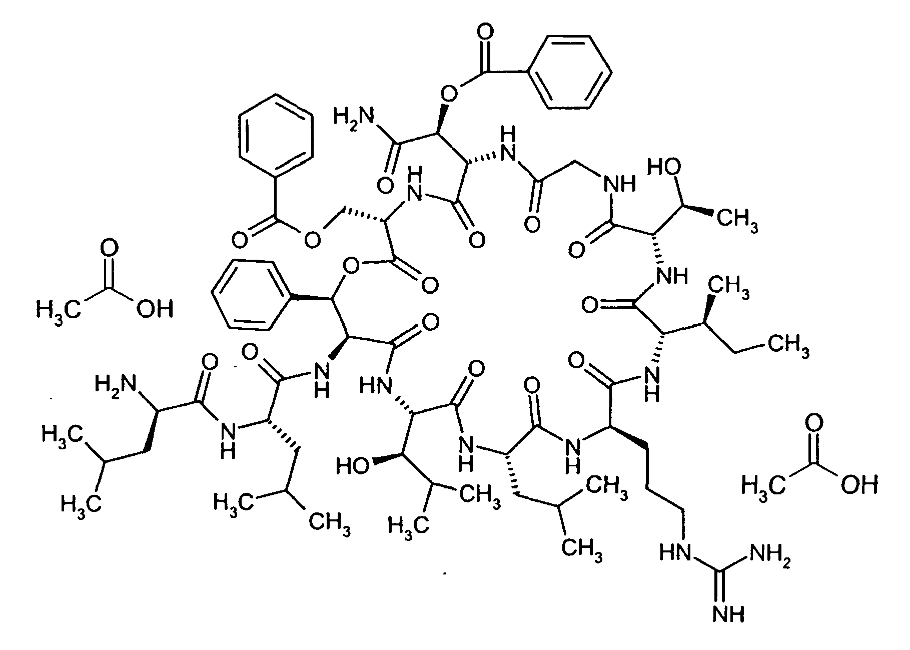

23 mg (0.02 mmol) Beispiel 2A werden nach der Allgemeinen Arbeitsvorschrift 1 umgesetzt. Nach der Reaktion mit Phenylcarbonsäurechlorid werden neben den zwei Mono-substituierten Boc-geschützten Lysobactin Derivaten (Beispiel 14 und 15) bei dieser Umsetzung 3.2 mg einer Verbindung mit zwei Benzoyl-Gruppen isoliert.
HPLC (Methode 1): Rₜ = 4.81 min;
LC-MS (Methode 3): Rₜ = 2.52 min;
MS (ESIpos.): m/z (%) = 743 (100), 1584 (5) [M + H]⁺;
MS (ESIneg.): m/z (%) = 121 (10), 1582 (5) [M - H]⁻.

Das Gemisch wird als Suspension in 0.15 ml DCM vorgelegt, mit 0.05 ml TFA versetzt und bei RT 6 min gerührt, bis durch analytische HPLC (Methode 1) vollständiger Umsatz anzeigt wird. Das Rohprodukt wird im Vakuum von Lösungsmittel befreit. Abschließend wird das Rohprodukt gelchromatographisch (Methode 9; Methanol:Acetone:Essigsäure / 80:20:0.1) getrennt. Man erhält 1.7 mg (6.5% d. Th.) der Titelverbindung.
HPLC (Methode 1): Rₜ = 3.88 min.
LC-MS (Methode 3): Rₜ = 1.98 min;
MS (ESIpos.): m/z (%) = 743 (100) [M + 2H]²⁺;
MS (ESIneg.): m/z (%) = 121(100), 741 (40).

Zur Aminosäure-Sequenzbestimmung wird eine analytische Probe des Produktes nach der Allgemeinen Arbeitsvorschrift 3 hydrolysiert und analysiert (Methode 7).

### Beispiele 17

### O^{3.10}-(3-(6-Morpholino-3-pyridyl)-carbonyl)-lysobactin-bistrifluoracetat

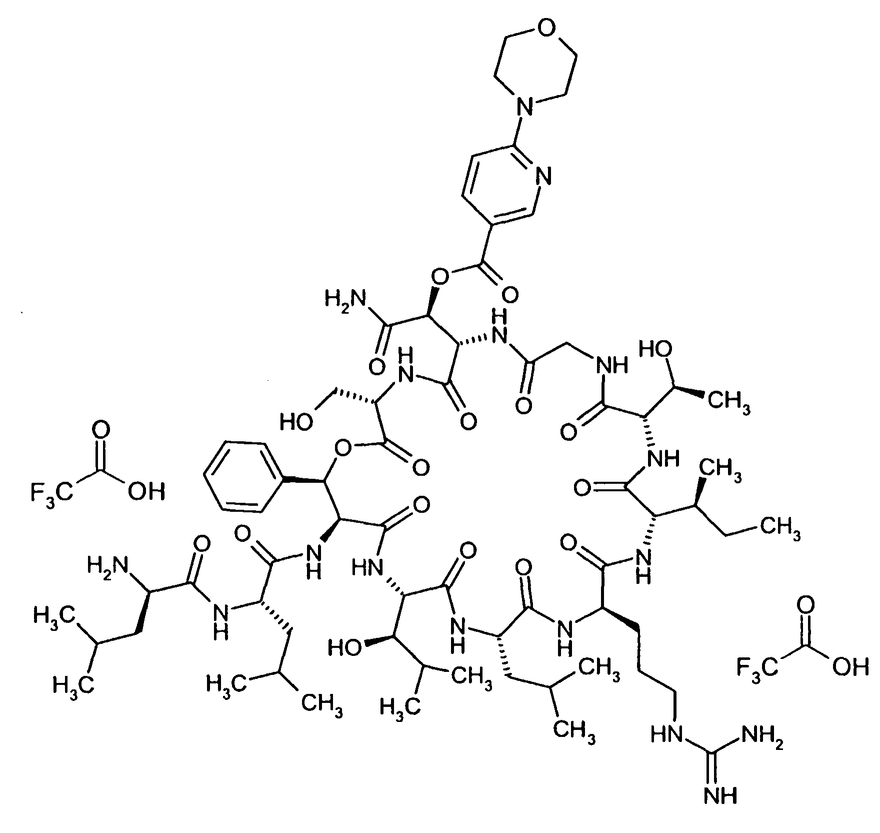

500 mg (0.35 mmol) Beispiel 2A werden nach der Allgemeinen Arbeitsvorschrift 1 umgesetzt. Nach der Reaktion mit 6-(Morpholin-4-yl)-pyridin-3-carbonsäurechlorid werden 151 mg eines mono-substituierten Derivates von Beispiel 2A isoliert.
HPLC (Methode 1): Rₜ = 4.38 min;
LC-MS (Methode 3): Rₜ = 2.07 min;
MS (ESIpos.): m/z (%) = 734 (20) [M - Boc + 2H]²⁺, 784 (100 [M + 2H]²⁺, 1566 (100) [M + H]⁺;
MS (ESIneg.): m/z (%) = 782 (100), 1564 (100) [M-H]⁻).

Das Boc-geschützte Lysobactin Derivat wird als Suspension in 3 ml DCM vorgelegt, mit 1 ml TFA versetzt und bei RT 15 min gerührt, bis durch analytische HPLC (Methode 1) vollständiger Umsatz anzeigt wird. Das Rohprodukt wird im Vakuum von Lösungsmittel befreit. Abschließend wird das Rohprodukt mittels präparativer HPLC (Methode 11) feingereinigt. Man erhält 135 mg (23% d. Th.) der Titelverbindung (Beispiel 17).
HPLC (Methode 1): Rₜ = 3.64 min.
HPLC (Methode 4): Rₜ = 7.98 min.
LC-MS (Methode 3): Rₜ = 1.60 min;
MS (ESIpos.): m/z (%) = 734 (100) [M + 2H]²⁺, 1466 (2) [M + H]⁺
MS (ESIneg.): m/z (%) = 628 (100), 732 (50), 1464 (10) [M - H]⁻.
HR-TOF-MS (Methode 8): C₆₈H₁₀₈N₁₇O₁₉ (MH⁺) ber. 1466.8007, gef. 11466.7960.

Zur Aminosäure-Sequenzbestimmung wird eine analytische Probe des Produktes analysiert (Methode 18).

### Beispiele 18 und 19

500 mg (0.35 mmol) Beispiel 2A werden nach der Allgemeinen Arbeitsvorschrift 1 umgesetzt. Nach der Reaktion mit 3-Methoxyphenylcarbonsäurechlorid werden 115 mg eines Gemisches aus zwei mono-substituierten Derivaten von Beispiel 2A isoliert.
HPLC (Methode 1): Rₜ = 4.58 min;
LC-MS (Methode 3): Rₜ = 2.24 min;
MS (ESIpos.): m/z (%) = 706 (100), 1510 (20) [M + H]⁺;
MS (ESIneg.): m/z (%) = 754 (100), 1508 (20) [M - H]⁻.

Das Gemisch wird als Suspension in 3 ml DCM vorgelegt, mit 1 ml TFA versetzt und bei RT 25 min gerührt, bis durch analytische HPLC (Methode 1) vollständiger Umsatz anzeigt wird. Das Rohprodukt wird im Vakuum von Lösungsmittel befreit. Abschließend wird das Rohprodukt gelchromatographisch grobgereinigt (Methode 9; Methanol:Aceton:TFA / 80:20:0.1) und mittels präparativer HPLC (Methode 16) feingereinigt. Man erhält 3 mg (0.7% d. Th.) der Beispiele 18 und 19 als 1:1 Gemisch.
HPLC (Methode 1): Rₜ = 3.79 min.
HPLC (Methode 5): Rₜ = 7.56 min
LC-MS (Methode 3): Rₜ = 1.63 min;
MS (ESIpos.): m/z (%) = 706 (100), 1410 (10) [M + H]⁺
MS (ESIneg.): m/z (%) = 704 (60), 1408 (15) [M - H]⁻.

Zur Aminosäure-Sequenzbestimmung wird eine analytische Probe des Produktes nach der Allgemeinen Arbeitsvorschrift 3 hydrolysiert und analysiert (Methode 7) (1/1 Gemisch).

### Beispiel 18

### O^{3.10}-(3-Methoxy-phenylcarbonyl)-lysobactin-bistrifluoracetat

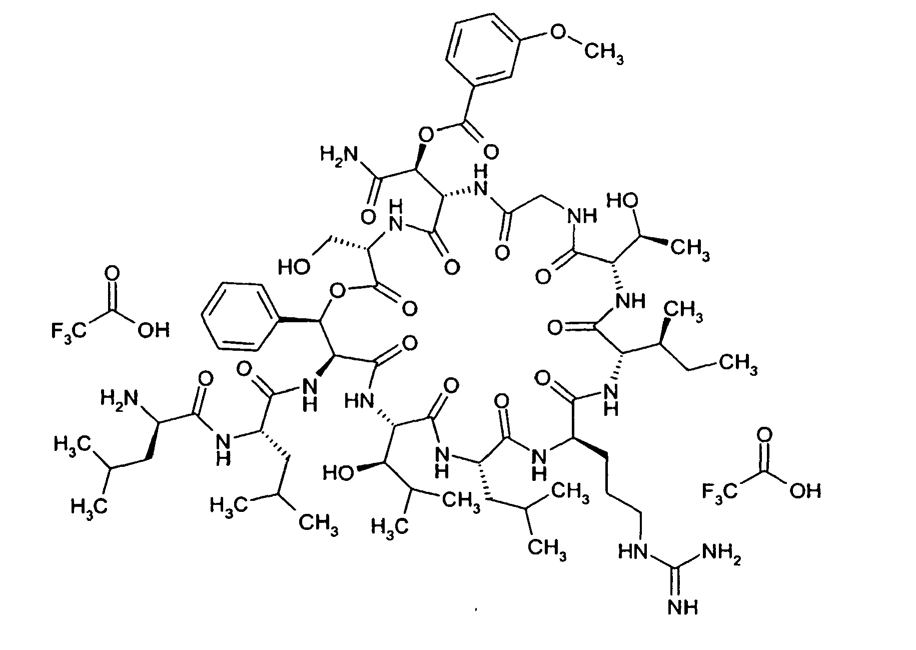

### Beispiel 19

### O^{3.11}-(3-Methoxy-phenylcarbonyl)-lysobactin-bistrifluoracetat

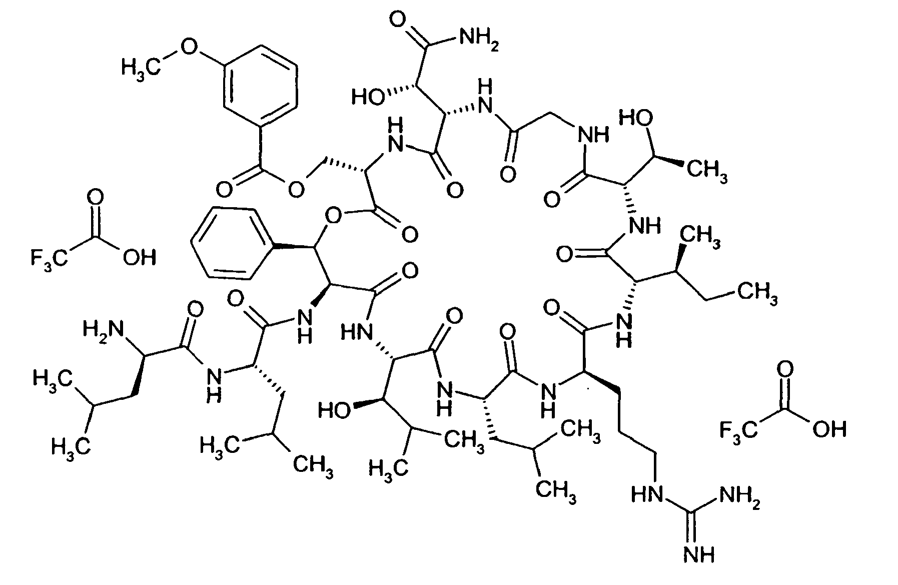

### Beispiel 20

### O^{3.10},O^{3.11}-Di-(phenylcarbonyl)-lysobactin-bistrifluoracetat

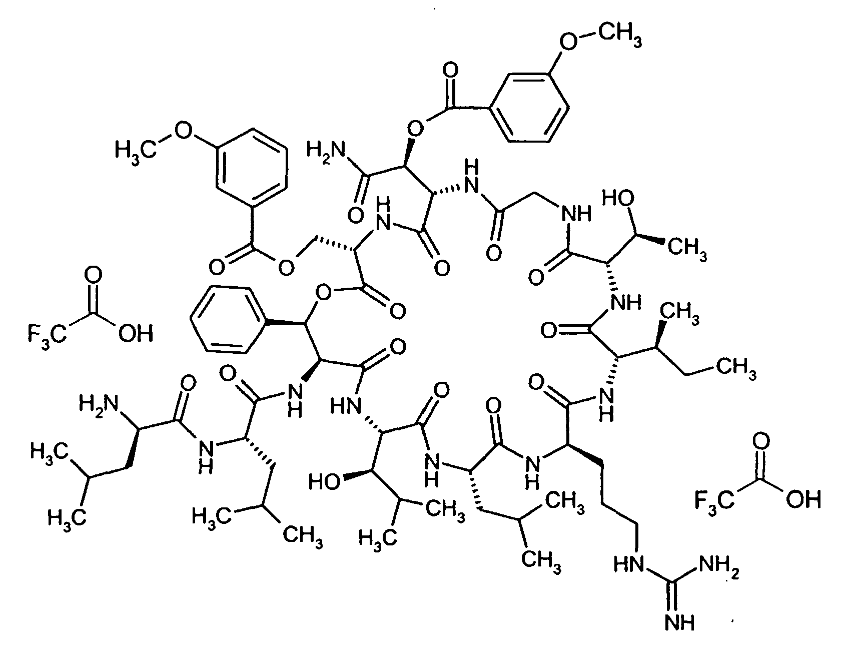

53 mg (0.035 mmol) Beispiel 2A werden nach der Allgemeinen Arbeitsvorschrift 1 umgesetzt. Nach der Reaktion mit 3-Methoxyphenylcarbonsäurechlorid werden neben den zwei mono-substituierten Derivaten von Beispiel 2A (Beispiel 18 und 19) bei dieser Umsetzung 11 mg einer Verbindung mit zwei Benzoyl-Gruppen isoliert.
HPLC (Methode 1): Rₜ = 4.88 min;
LC-MS (Methode 3): Rₜ = 2.62 min;
MS (ESIpos.): m/z (%) = 773 (45), 823 (100), 1644 (15).

Das Gemisch wird als Suspension in 1 ml DCM vorgelegt, mit 0.3 ml TFA versetzt und bei RT 15 min gerührt, bis durch analytische HPLC (Methode 1) vollständiger Umsatz anzeigt wird. Das Rohprodukt wird im Vakuum von Lösungsmittel befreit. Abschließend wird das Rohprodukt gelchromatographisch (Methode 9; Methanol:Aceton:TFA / 80:20:0.5) feingereinigt. Man erhält 6.2 mg (16% d. Th.) der Titelverbindung.
HPLC (Methode 1): Rₜ = 4.00 min.
LC-MS (Methode 3): Rₜ = 1.96 min;
MS (ESIpos.): m/z (%) = 773 (100), 1544 (10) [M + 2H]²⁺;
MS (ESIneg.): m/z (%) = 151 (100), 1542 (20).

Zur Aminosäure-Sequenzbestimmung wird eine analytische Probe des Produktes nach der Allgemeinen Arbeitsvorschrift 3 hydrolysiert und analysiert (Methode 7).

### Beispiel 21

### O^{3.11}-(Phenylsulfonylaminocarbonyl)-lysobactin-bistrifluoracetat

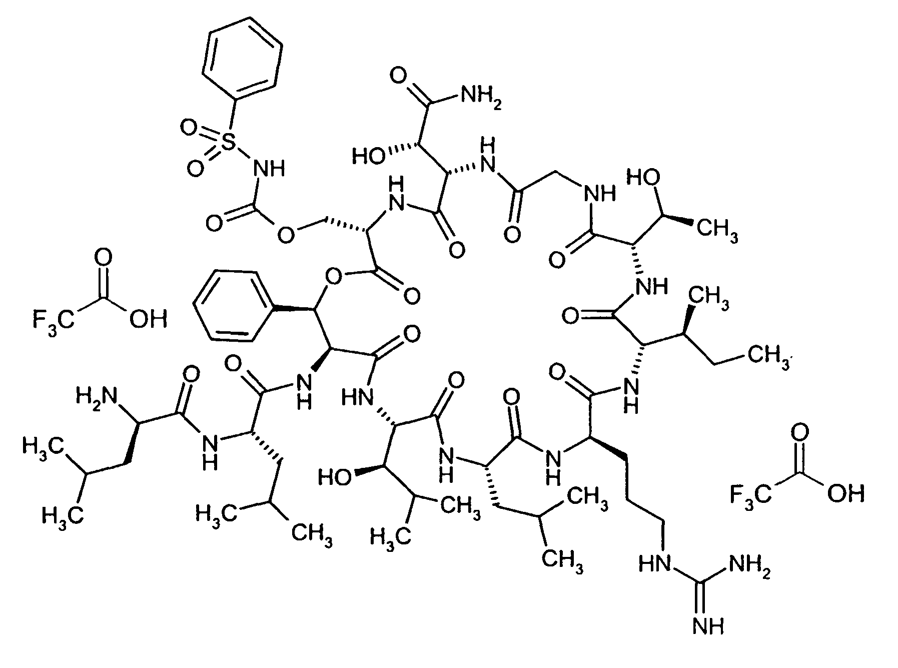

500 mg (0.35 mmol) Beispiel 2A werden nach der Allgemeinen Arbeitsvorschrift 2 umgesetzt. Nach der Reaktion mit Phenylsulfonylisocyanat werden 318 mg eines Mono-substituierten Boc-geschützten Lysobactin Derivates isoliert.
HPLC (Methode 1): Rₜ = 4.56 min;
LC-MS (Methode 3): Rₜ = 2.45 min;
MS (ESIpos.): m/z (%) = 730 (100) [M - Boc + 2H]²⁺, 1559 (20) [M + H]⁺;
MS (ESIneg.): m/z (%) = 778 (100), 1557 (50) [M - H]⁻);
HR-TOF-MS (Methode 8): C₇₀H₁₁₁N₁₆O₂₂S (MH⁺) ber. 1559.7780, gef. 1559.7739.

Das Rohprodukt wird als Suspension in 9 ml DCM vorgelegt, mit 3 ml TFA versetzt und bei RT 10 min gerührt, bis durch analytische HPLC (Methode 1) vollständiger Umsatz anzeigt wird. Das Rohprodukt wird im Vakuum von Lösungsmittel befreit. Abschließend wird das Rohprodukt gelchromatographisch grobgereinigt (Methode 9; Methanol:Aceton:TFA / 80:20:0.1) und mittels präparativer HPLC (Methode 17) feingereinigt. Man erhält 218 mg (43% d. Th.) der Titelverbindung (Beispiel 21).
HPLC (Methode 1): Rₜ = 3.86 min.
HPLC/UV-Vis (Methode 2): Rₜ = 4.23 min.
λₘₐₓ (qualitativ) = 200 nm (s), 220 nm (m).
LC-MS (Methode 3): Rₜ = 2.00 min;
MS (ESIpos.): m/z (%) = 730 (100) [M + 2H]²⁺, 1459 (5) [M + H]⁺;
MS (ESIneg.): m/z (%) = 728 (100), 1457 (80) [M - H]⁻.
HR-TOF-MS (Methode 8): C₆₅H₁₀₃N₁₆O₂₀ (MH⁺) ber. 1459.7255, gef. 1459.7209.

Zur Aminosäure-Sequenzbestimmung wird eine analytische Probe des Produktes nach der Allgemeinen Arbeitsvorschrift 3 hydrolysiert und analysiert (Methode 18).

### B. Bewertung der physiologischen Wirksamkeit

Die *in vitro*-Wirkung der erfindungsgemäßen Verbindungen kann in folgenden Assays gezeigt werden:

### Bestimmung der minimalen Hemmkonzentration (MHK):

Die MHK wird im Flüssigdilutionstest gemäß der NCCLS-Richtlinien bestimmt. Übernachtkulturen von *Staphylococcus aureus* 133, *Entercococcus faecalis* 27159, *E. faecium* 4147 und *Streptococcus pneumoniae* G9a werden mit den beschriebenen Testsubstanzen in einer 1:2 Verdünnungsreihe inkubiert. Die MHK-Bestimmung wird mit einer Zellzahl von 10⁵ Keimen pro ml in Isosensitest-Medium (Fa. Difco, Irvine/USA) durchgeführt, mit Ausnahme von *S. pneumoniae,* der in BHI-Bouillon (Fa. Difco, Irvine/USA) mit 10% Rinderserum bei einer Zellzahl von 10⁶ Keimen pro ml getestet wird. Die Kulturen werden bei 37°C für 18-24 Stunden inkubiert, *S. pneumoniae* in Gegenwart von 10% CO₂.

Die jeweils niedrigste Substanzkonzentration, bei der kein sichtbares Bakterienwachstum mehr auftritt, wird als MHK definiert. Die MHK-Werte werden in µg/ml angegeben.

Repräsentative in-vitro-Wirkdaten für die erfindungsgemäßen Verbindungen sind in Tabelle A wiedergegeben:

**Tabelle A**

| **Beispiel-Nr.** | **MHK** | **MHK** | **MHK** | **MHK** |
|---|---|---|---|---|
| | ***S. aureus* 133** | ***S. pneumoniae G9a*** | ***E. faecieum* L4001** | ***E. faecalis*** |
| | **[µg/ml]** | **[µg/ml]** | **[µg/ml]** | ***ICB27159*** |
| | | | | **[µg/ml]** |
| **2** | 0.5 | 1 | 4 | 1 |
| **11** | 0.5 | 0.5 | 1 | 0.5 |
| **14/15** | 0.4 | 0.8 | 3.2 | 1.6 |
| **17** | 0.5 | 0.5 | 4 | 2 |

Die Eignung der erfindungsgemäßen Verbindungen zur Behandlung von bakteriellen Infektionen kann im folgenden Tiermodell gezeigt werden:

### Systemische Infektion mit Staphylococcus aureus 133:

Zellen von *S. aureus* 133 werden über Nacht in BHI-Bouillon (Fa. Oxoid, New York/ USA) angezüchtet. Die Übernachtkultur wird 1:100 in frische BHI-Bouillon verdünnt und für 3 Stunden inkubiert. Die dann in der logarithmischen Wachstumsphase befindlichen Zellen werden abzentrifugiert und zweimal mit gepufferter, physiologischer Kochsalzlösung gewaschen. Danach wird photometrisch eine Zellsuspension in Kochsalzlösung mit einer Extinktion von 50 Einheiten eingestellt. Nach einem Verdünnungsschritt (1:15) wird diese Suspension 1:1 mit einer 10%igen Mucinlösung gemischt. Von dieser Infektionslösung werden 0.25 ml/20 g Maus intraperitoneal (entsprechend 1x10⁶ Keimen/Maus) appliziert. Die Therapie erfolgt intraperitoneal oder intravenös 30 Minuten nach der Infektion. Für den Infektionsversuch werden weibliche CFW1-Mäuse verwendet. Das Überleben der Tiere wird über 6 Tage protokolliert.

Die Eigenschaften der erfindungsgemäßen Verbindungen im Hinblick auf die Nierenverträglichkeit können im folgenden Tiermodell gezeigt werden:

### Maus-Modell zur Bestimmung nephrotoxischer Effekte:

Nephrotoxische Nebenwirkungen der Nonadepsipeptide werden durch histopathologische Untersuchungen der Nieren in Mäusen und/oder Ratten nach mehrfacher Gabe einer bestimmten Dosierung analysiert. Dafür werden 5-6 Tiere täglich entweder intravenös (i.v.) oder intraperitoneal (i.p.) mit Substanzen behandelt, die in wässriger Lösung oder unter Zusatz von Solutol gelöst werden. Nierentoxische Effekte werden durch lichtmikroskopische Auswertung, Hämatoxilin und Eosin (H&E) gefärbter Paraffinschnitte der Nieren bestimmt. Eine Periodic-Acid Schiff (PAS)-Reaktion wird wahlweise zur besseren Darstellung von Glykoproteinen durchgeführt. Nephrotoxische Effekte werden semiquantitativ für jedes Tier als Schweregrade der auftretenden tubulären Basophilie und Degeneration/Regeneration festgelegt (Schweregrade: 0 = kein Effekt; 1 = minimaler Effekt; 2 = leichter Effekt; 3 = moderater Effekt; 4 = schwere Läsionen). Der durchschnittliche Schweregrad der tubulären Degeneration/Regeneration sowie die Inzidenz (Anzahl der betroffenen Tiere) wird pro Tiergruppe oder Derivat berechnet. Darüber hinausgehende Nierenveränderungen wie tubuläre Dilatation sowie Nekrosen und Ansammlung nekrotischen Materials werden ebenfalls aufgeführt.

**Die Löslichkeit** einer Verbindung wird nach dem Fachmann bekannten Methoden bestimmt.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der Verbindung von Beispiel 1, 50 mg Lactose (Monohydrat), 50 mg Maisstärke (nativ), 10 mg Polyvinylpyrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus Wirkstoff, Lactose und Stärke wird mit einer 5%-igen Lösung (m/m) des PVPs in Wasser granuliert. Das Granulat wird nach dem Trocknen mit dem Magnesiumstearat für 5 min gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der Verbindung von Beispiel 1, 1000 mg Ethanol (96%), 400 mg Rhodigel (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, der Wirkstoff wird der Suspension zugefügt. Unter Rühren erfolgt die Zugabe des Wassers. Bis zum Abschluss der Quellung des Rhodigels wird ca. 6h gerührt.

### Intravenös applizierbare Lösung:

### Zusammensetzung:

100-200 mg der Verbindung von Beispiel 1, 15 g Polyethylenglykol 400 und 250 g Wasser für Injektionszwecke.

### Herstellung:

Die Verbindung von Beispiel 1 wird zusammen mit Polyethylenglykol 400 in dem Wasser unter Rühren gelöst. Die Lösung wird sterilfiltriert (Porendurchmesser 0.22 µm) und unter aseptischen Bedingungen in hitzesterilisierte Infusionsflaschen abgefüllt. Diese werden mit Infusionsstopfen und Bördelkappen verschlossen.

## Patentansprüche

1. Verbindung der Formel 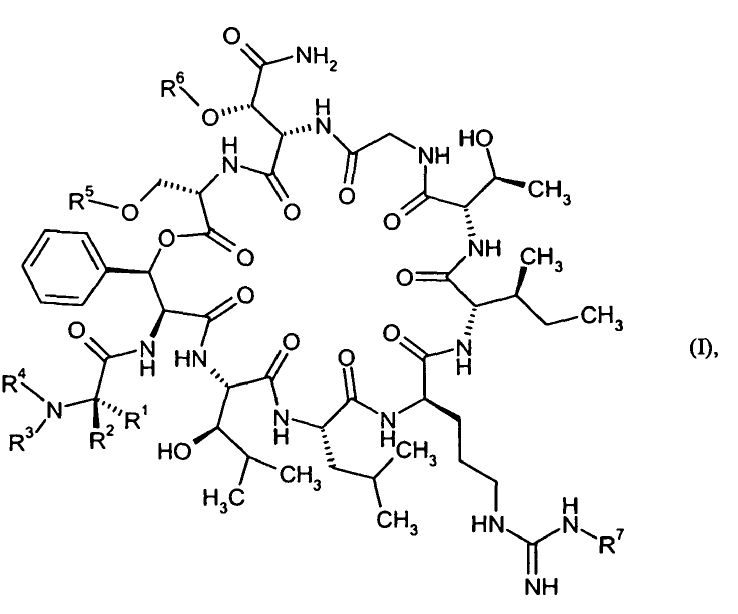 in welcher
R¹ Wasserstoff, C₁-C₆-Alkyl, C₂-C₆-Alkenyl, C₃-C₆-Cycloalkyl oder C₆-C₁₀-Aryl bedeutet,
wobei Alkyl, Alkenyl, Cycloalkyl und Aryl substituiert sein können mit 0, 1, 2 oder 3 Substituenten unabhängig voneinander ausgewählt aus der Gruppe bestehend aus Halogen, Hydroxy, Amino, Cyano, Trimethylsilyl, C₁-C₆-Alkyl, C₁-C₆-Alkoxy, Benzyloxy, C₃-C₆-Cycloalkyl, C₆-C₁₀-Aryl, 5- bis 7-gliedriges Heterocyclyl, 5- bis 10-gliedriges Heteroaryl, C₁-C₆-Alkylamino, C₆-C₁₀-Arylamino, C₁-C₆-Alkylcarbonylamino, C₆-C₁₀-Arylcarbonyl-amino, C₁-C₆-Alkylcarbonyl, C₁-C₆-Alkoxycarbonyl, C₆-C₁₀-Arylcarbonyl und Benzyloxycarbonylamino,
worin Cycloalkyl, Aryl, Heterocyclyl und Heteroaryl ihrerseits substituiert sein können mit 0, 1, 2 oder 3 Substituenten unabhängig voneinander ausgewählt aus der Gruppe bestehend aus Halogen, Hydroxy, Amino, Cyano, Nitro, Trifluormethyl, C₁-C₆-Alkyl, C₁-C₆-Alkoxy, Phenyl und 5- bis 7-gliedriges Heterocyclyl,
R² Wasserstoff oder C₁-C₄-Alkyl bedeutet,
R³ C₁-C₆-Alkyl, C₃-C₆-Cycloalkyl, 5- bis 7-gliedriges Heterocyclyl, C₆-C₁₀-Aryl, 5-oder 6-gliedriges Heteroaryl, C₁-C₆-Alkylcarbonyl, C₁-C₆-Alkoxycarbonyl, C₃-C₆-Cycloalkylcarbonyl, 5- bis 7-gliedriges Heterocyclylcarbonyl, C₆-C₁₀-Arylcarbonyl, 5- oder 6-gliedriges Heteroarylcarbonyl oder C₁-C₆-Alkylaminocarbonyl bedeutet,
wobei Alkyl, Cycloalkyl, Heterocyclyl, Aryl, Heteroaryl, Alkoxycarbonyl, Cycloalkylcarbonyl, Heterocyclylcarbonyl, Arylcarbonyl, Heteroarylcarbonyl und Alkylaminocarbonyl substituiert sein können mit 0, 1, 2 oder 3 Substituenten unabhängig voneinander ausgewählt aus der Gruppe bestehend aus Halogen, Hydroxy, Amino, C₁-C₆-Alkylamino und Phenyl,
und
wobei Alkylcarbonyl substituiert ist mit einem Substituenten Amino oder C₁-C₆-Alkylamino,
und
wobei Alkylcarbonyl substituiert sein kann mit weiteren 0, 1 oder 2 Substituenten unabhängig voneinander ausgewählt aus der Gruppe bestehend aus Halogen, Hydroxy, Trimethylsilyl, C₁-C₆-Alkoxy, C₁-C₆-Alkylthio, Benzyloxy, C₃-C₆-Cycloalkyl, Phenyl, Naphthyl, 5- bis 10-gliedriges Heteroaryl, C₁-C₆-Alkylcarbonylamino, C₁-C₆-Alkoxycarbonylamino, C₆-C₁₀-Arylcarbonylamino, C₆-C₁₀-Arylcarbonyloxy, Benzyloxycarbonyl und Benzyloxycarbonylamino,
worin Phenyl und Heteroaryl ihrerseits substituiert sein können mit 0, 1, 2 oder 3 Substituenten unabhängig voneinander ausgewählt aus der Gruppe bestehend aus Halogen, Hydroxy, Nitro, C₁-C₆-Alkyl, C₁-C₆-Alkoxy und Phenyl,
R⁴ Wasserstoff, C₁-C₄-Alkyl, Cyclopropyl oder Cyclopropylmethyl bedeutet,
und
R⁵ C₆-C₁₀-Arylaminocarbonyl, C₆-C₁₀-Axylcarbonyl, C₆-C₁₀-Arylaminothiocarbonyl, C₆-C₁₀Arylthiocarbonyl, C₆-C₁₀-Arylsulfonylaminocarbonyl, 5- bis 10-gliedriges Heteroarylaminocarbonyl, 5- bis 10-gliedriges Heteroarylcarbonyl, 5- bis 10-gliedriges Heteroarylaminothiocarbonyl oder 5- bis 10-gliedriges Heteroarylthiocarbonyl bedeutet,
wobei Arylaminocarbonyl, Arylcarbonyl, Arylaminothiocarbonyl, Arylthiocarbonyl, Arylsulfonylaminocarbonyl, Heteroarylaminocarbonyl, Heteroarylcarbonyl, Heteroarylaminothiocarbonyl und Heteroarylthiocarbonyl substituiert sein können mit 0, 1, 2 oder 3 Substituenten unabhängig voneinander ausgewählt aus der Gruppe bestehend aus Halogen, C₁-C₆-Alkyl, C₁-C₆-Alkoxy, C₁-C₆-Alkylamino, C₁-C₆-Alkoxycarbonyl, C₁-C₆-Alkylaminocarbonyl, C₁-C₆-Alkylaminosulfonyl, gegebenenfalls Oxo substituiertes 5- bis 7-gliedriges Heterocyclyl und 5- bis 10-gliedriges Heteroaryl,
R⁶ Wasserstoff bedeutet,
R⁷ Wasserstoff bedeutet,
oder
R⁵ Wasserstoff bedeutet,
R⁶ C₆-C₁₀-Arylaminocarbonyl, C₆-C₁₀-Arylcarbonyl, C₆-C₁₀-Arylaminothiocarbonyl, C₆-C₁₀-Arylthiocarbonyl, C₆-C₁₀-Arylsulfonylaminocarbonyl, 5- bis 10-gliedriges Heteroarylaminocarbonyl, 5- bis 10-gliedriges Heteroarylcarbonyl, 5- bis 10-gliedriges Heteroarylaminothiocarbonyl oder 5- bis 10-gliedriges Heteroarylthiocarbonyl bedeutet,
wobei Arylaminocarbonyl, Arylcarbonyl, Arylaminothiocarbonyl, Arylthiocarbonyl, Arylsulfonylaminocarbonyl, Heteroarylaminocarbonyl, Heteroarylcarbonyl, Heteroarylaminothiocarbonyl und Heteroarylthiocarbonyl substituiert sein können mit 0, 1, 2 oder 3 Substituenten unabhängig voneinander ausgewählt aus der Gruppe bestehend aus Halogen, C₁-C₆-Alkyl, C₁-C₆-Alkoxy, C₁-C₆-Alkylamino, C₁-C₆-Alkoxycarbonyl, C₁-C₆-Alkylaminocarbonyl, C₁-C₆-Alkylaminosulfonyl, gegebenenfalls Oxo substituiertes 5- bis 7-gliedriges Heterocyclyl und 5- bis 10-gliedriges Heteroaryl,
R⁷ Wasserstoff bedeutet,
oder
R⁵ Wasserstoff bedeutet,
R⁶ Wasserstoff bedeutet,
R⁷ C₆-C₁₀-Arylaminocarbonyl, C₆-C₁₀-Arylcarbonyl, C₆-C₁₀-Arylaminothiocarbonyl, C₆-C₁₀-Arylthiocarbonyl, 5- bis 10-gliedriges Heteroarylaminocarbonyl, 5- bis 10-gliedriges Heteroarylcarbonyl, 5- bis 10-gliedriges Heteroarylaminothiocarbonyl oder 5- bis 10-gliedriges Heteroarylthiocarbonyl bedeutet,
wobei Arylaminocarbonyl, Arylcarbonyl, Arylaminothiocarbonyl, Arylthiocarbonyl, Heteroarylaminocarbonyl, Heteroarylcarbonyl, Heteroarylaminothiocarbonyl und Heteroarylthiocarbonyl substituiert sein können mit 0, 1, 2 oder 3 Substituenten unabhängig voneinander ausgewählt aus der Gruppe bestehend aus Halogen, C₁-C₆-Alkyl, C₁-C₆-Alkoxy, C₁-C₆-Alkylamino, C₁-C₆-Alkoxycarbonyl, C₁-C₆-Alkylaminocarbonyl, C₁-C₆-Alkylaminosulfonyl, gegebenenfalls Oxo substituiertes 5- bis 7-gliedriges Heterocyclyl und 5- bis 10-gliedriges Heteroaryl,
oder
R⁵ und R⁶ gleich sind, und
C₆-C₁₀-Arylaminocarbonyl, C₆-C₁₀-Arylcarbonyl, C₆-C₁₀-Arylaminothiocarbonyl, C₆-C₁₀-Arylthiocarbonyl, C₆-C₁₀-Arylsulfonylaminocarbonyl, 5- bis 10-gliedriges Heteroarylaminocarbonyl, 5- bis 10-gliedriges Heteroarylcarbonyl, 5- bis 10-gliedriges Heteroarylaminothiocarbonyl oder 5- bis 10-gliedriges Heteroarylthiocarbonyl bedeuten,
wobei Arylaminocarbonyl, Arylcarbonyl, Arylaminothiocarbonyl, Arylthiocarbonyl, Arylsulfonylaminocarbonyl, Heteroarylaminocarbonyl, Heteroarylcarbonyl, Heteroarylaminothiocarbonyl und Heteroarylthiocarbonyl substituiert sein können mit 0, 1, 2 oder 3 Substituenten unabhängig voneinander ausgewählt aus der Gruppe bestehend aus Halogen, C₁-C₆-Alkyl, C₁-C₆-Alkoxy, C₁-C₆-Alkylamino, C₁-C₆-Alkoxycarbonyl, C₁-C₆-Alkylaminocarbonyl, C₁-C₆-Alkylaminosulfonyl, gegebenenfalls Oxo substituiertes 5- bis 7-gliedriges Heterocyclyl und 5- bis 10-gliedriges Heteroaryl,
R⁷ Wasserstoff bedeutet,
oder eines ihrer Salze, ihrer Solvate oder der Solvate ihrer Salze.

2. Verbindung nach Anspruch 1, **dadurch gekennzeichnet, dass**
R¹ 2-Methylprop-1-yl, 2,2-Dimethylprop-1-yl, 2-Pyridylmethyl oder 3-Pyridylmethyl bedeutet,
wobei 2-Pyridylmethyl oder 3-Pyridylmethyl substituiert sein können mit 0, 1, 2 oder 3 Substituenten unabhängig voneinander ausgewählt aus der Gruppe bestehend aus Hydroxy, Amino, Trifluormethyl, Methyl, Methoxy und Morpholinyl,
R² Wasserstoff bedeutet,
R³ 1-Amino-3-methylbut-1-ylcarbonyl, 1-Amino-3,3-dimethylbut-1-ylcarbonyl oder 1-Amino-2-trimethylsilyleth-1-ylcarbonyl bedeutet,
R⁴ Wasserstoff bedeutet,
und
R⁵ C₆-C₁₀-Arylaminocarbonyl, C₆-C₁₀-Arylcarbonyl, C₆-C₁₀-Arylaminothiocarbonyl, C₆-C₁₀-Arylthiocarbonyl, C₆-C₁₀-Arylsulfonylaminocarbonyl, 5- bis 10-gliedriges Heteroarylaminocarbonyl, 5- bis 10-gliedriges Heteroarylcarbonyl, 5- bis 10-gliedriges Heteroarylaminothiocarbonyl oder 5- bis 10-gliedriges Heteroarylthiocarbonyl bedeutet,
wobei Arylaminocarbonyl, Arylcarbonyl, Arylaminothiocarbonyl, Arylthiocarbonyl, Arylsulfonylaminocarbonyl, Heteroarylaminocarbonyl, Heteroarylcarbonyl, Heteroarylaminothiocarbonyl und Heteroarylthiocarbonyl substituiert sein können mit 0, 1, 2 oder 3 Substituenten unabhängig voneinander ausgewählt aus der Gruppe bestehend aus Halogen, C₁-C₆-Alkyl, C₁-C₆-Alkoxy, C₁-C₆-Alkylamino, C₁-C₆-Alkoxycarbonyl, C₁-C₆-Alkylaminocarbonyl, C₁-C₆-Alkylaminosulfonyl, gegebenenfalls Oxo substituiertes 5- bis 7-gliedriges Heterocyclyl und 5- bis 10-gliedriges Heteroaryl,
R⁶ Wasserstoff bedeutet,
R⁷ Wasserstoff bedeutet,
oder
R⁵ Wasserstoff bedeutet,
R⁶ C₆-C₁₀-Arylaminocarbonyl, C₆-C₁₀-Arylcarbonyl, C₆-C₁₀-Arylaminothiocarbonyl, C₆-C₁₀-Arylthiocarbonyl, C₆-C₁₀-Arylsulfonylaminocarbonyl, 5- bis 10-gliedriges Heteroarylaminocarbonyl, 5- bis 10-gliedriges Heteroarylcarbonyl, 5- bis 10-gliedriges Heteroarylaminothiocarbonyl oder 5- bis 10-gliedriges Heteroarylthiocarbonyl bedeutet,
wobei Arylaminocarbonyl, Arylcarbonyl, Arylaminothiocarbonyl, Arylthiocarbonyl, Arylsulfonylaminocarbonyl, Heteroarylaminocarbonyl, Heteroarylcarbonyl, Heteroarylaminothiocarbonyl und Heteroarylthiocarbonyl substituiert sein können mit 0, 1, 2 oder 3 Substituenten unabhängig voneinander ausgewählt aus der Gruppe bestehend aus Halogen, C₁-C₆-Alkyl, C₁-C₆-Alkoxy, C₁-C₆-Alkylamino, C₁-C₆-Alkoxycarbonyl, C₁-C₆-Alkylaminocarbonyl, C₁-C₆-Alkylaminosulfonyl, gegebenenfalls Oxo substituiertes 5- bis 7-gliedriges Heterocyclyl und 5- bis 10-gliedriges Heteroaryl,
R⁷ Wasserstoff bedeutet,
oder
R⁵ Wasserstoff bedeutet,
R⁶ Wasserstoff bedeutet,
R⁷ C₆-C₁₀-Arylaminocarbonyl, C₆-C₁₀-Arylcarbonyl, C₆-C₁₀-Arylaminothiocarbonyl, C₆-C1₀-Arylthiocarbonyl, 5- bis 10-gliedriges Heteroarylaminocarbonyl, 5- bis 10-gliedriges Heteroarylcarbonyl, 5- bis 10-gliedriges. Heteroarylaminothiocarbonyl oder 5- bis 10-gliedriges Heteroarylthiocarbonyl bedeutet,
wobei Arylaminocarbonyl, Arylcarbonyl, Arylaminothiocarbonyl, Arylthiocarbonyl, Heteroarylaminocarbonyl, Heteroarylcarbonyl, Heteroarylaminothiocarbonyl und Heteroarylthiocarbonyl substituiert sein können mit 0, 1, 2 oder 3 Substituenten unabhängig voneinander ausgewählt aus der Gruppe bestehend aus Halogen, C₁-C₆-Alkyl, C₁-C₆-Alkoxy, C₁-C₆-Alkylamino, C₁-C₆-Alkoxycarbonyl, C₁-C₆-Alkylaminocarbonyl, C₁-C₆-Alkylaminosulfonyl, gegebenenfalls Oxo substituiertes 5- bis 7-gliedriges Heterocyclyl und 5- bis 10-gliedriges Heteroaryl,
oder
R⁵ und R⁶ gleich sind, und
C₆-C₁₀-Arylaminocarbonyl, C₆-C₁₀-Arylcarbonyl, C₆-C₁₀-Axylaminothiocarbonyl, C₆-C₁₀-Axylthiocarbonyl, C₆-C₁₀-Arylsulfonylaminocarbonyl, 5- bis 10-gliedriges Heteroarylaminocarbonyl, 5- bis 10-gliedriges Heteroarylcarbonyl, 5- bis 10-gliedriges Heteroarylaminothiocarbonyl oder 5- bis 10-gliedriges Heteroarylthiocarbonyl bedeuten,
wobei Arylaminocarbonyl, Arylcarbonyl, Arylaminothiocarbonyl, Arylthiocarbonyl, Arylsulfonylaminocarbonyl, Heteroarylaminocarbonyl, Heteroarylcarbonyl, Heteroarylaminothiocarbonyl und Heteroarylthiocarbonyl substituiert sein können mit 0, 1,2 oder 3 Substituenten unabhängig voneinander ausgewählt aus der Gruppe bestehend aus Halogen, C₁-C₆-Alkyl, C₁-C₆-Alkoxy, C₁-C₆-Alkylamino, C₁-C₆-Alkoxycarbonyl, C₁-C₆-Alkylaminocarbonyl, C₁-C₆-Alkylaminosulfonyl, gegebenenfalls Oxo substituiertes 5- bis 7-gliedriges Heterocyclyl und 5- bis 10-gliedriges Heteroaryl,
R⁷ Wasserstoff bedeutet,
oder eines ihrer Salze, ihrer Solvate oder der Solvate ihrer Salze.

3. Verbindung nach einem der Ansprüche 1 oder 2, **dadurch gekennzeichnet, dass**
R¹ 2-Methylprop-1-yl bedeutet,
R² Wasserstoff bedeutet,
R³ 1-Amino-3-methylbut-1-ylcarbonyl bedeutet,
R⁴ Wasserstoff bedeutet,
und
R⁵ Wasserstoff bedeutet,
R⁶ C₆-C₁₀-Arylaminocarbonyl, C₆-C₁₀-Arylcarbonyl, C₆-C₁₀-Arylaminothiocarbonyl, C₆-C₁₀-Arylthiocarbonyl, C₆-C₁₀-Arylsulfonylaminocarbonyl, 5- bis 10-gliedriges Heteroarylaminocarbonyl, 5- bis 10-gliedriges Heteroarylcarbonyl, 5- bis 10-gliedriges Heteroarylaminothiocarbonyl oder 5- bis 10-gliedriges Heteroarylthiocarbonyl bedeutet,
wobei Arylaminocarbonyl, Arylcarbonyl, Arylaminothiocarbonyl, Arylthiocarbonyl, Arylsulfonylaminocarbonyl, Heteroarylaminocarbonyl, Heteroarylcarbonyl, Heteroarylaminothiocarbonyl und Heteroarylthiocarbonyl substituiert sein können mit 0, 1, 2 oder 3 Substituenten unabhängig voneinander ausgewählt aus der Gruppe bestehend aus Halogen, C₁-C₆-Alkyl, C₁-C₆-Alkoxy, C₁-C₆-Alkylamino, C₁-C₆-Alkoxycarbonyl, C₁-C₆-Alkylaminocarbonyl, C₁-C₆-Alkylaminosulfonyl, gegebenenfalls Oxo substituiertes 5- bis 7-gliedriges Heterocyclyl und 5- bis 10-gliedriges Heteroaryl,
R⁷ Wasserstoff bedeutet,
oder
R⁵ Wasserstoff bedeutet,
R⁶ Wasserstoff bedeutet,
R⁷ C₆-C₁₀-Arylaminocarbonyl, C₆-C₁₀-Arylcarbonyl, C₆-C₁₀-Arylaminothiocarbonyl, C₆-C₁₀-Arylthiocarbonyl, 5- bis 10-gliedriges Heteroarylaminocarbonyl, 5- bis 10-gliedriges Heteroarylcarbonyl, 5- bis 10-gliedriges Heteroarylaminothiocarbonyl oder 5- bis 10-gliedriges Heteroarylthiocarbonyl bedeutet,
wobei Arylaminocarbonyl, Arylcarbonyl, Arylaminothiocarbonyl, Arylthiocarbonyl, Heteroarylaminocarbonyl, Heteroarylcarbonyl, Heteroarylaminothiocarbonyl und Heteroarylthiocarbonyl substituiert sein können mit 0, 1, 2 oder 3 Substituenten unabhängig voneinander ausgewählt aus der Gruppe bestehend aus Halogen, C₁-C₆-Alkyl, C₁-C₆-Alkoxy, C₁-C₆-Alkylamino, C₁-C₆-Alkoxycarbonyl, C₁-C₆-Alkylaminocarbonyl, C₁-C₆-Alkylaminosulfonyl, gegebenenfalls Oxo substituiertes 5- bis 7-gliedriges Heterocyclyl und 5- bis 10-gliedriges Heteroaryl,
oder eines ihrer Salze, ihrer Solvate oder der Solvate ihrer Salze.

4. Verbindung nach einem der Ansprüche 1 bis 3, **dadurch gekennzeichnet, dass**
R¹ 2-Methylprop-1-yl bedeutet,
R² Wasserstoff bedeutet,
R³ 1-Amino-3-methylbut-1-ylcarbonyl bedeutet,
R⁴ Wasserstoff bedeutet,
und
R⁵ Wasserstoff bedeutet,
R⁶ Phenylaminocarbonyl, Phenylcarbonyl, Pyridylaminocarbonyl oder Pyridylcarbonyl bedeutet,
wobei Phenylaminocarbonyl, Phenylcarbonyl, Pyridylaminocarbonyl und Pyridylcarbonyl substituiert sein können mit 0, 1, 2 oder 3 Substituenten unabhängig voneinander ausgewählt aus der Gruppe bestehend aus Pyrrolidinyl, Piperidinyl, Tetrahydropyranyl, Piperazinyl, Morpholinyl, 2-Oxo-pyrrolidinyl und 2-Oxopiperidinyl,
R⁷ Wasserstoff bedeutet,
oder
R⁵ Wasserstoff bedeutet,
R⁶ Wasserstoff bedeutet,
R⁷ Phenylaminocarbonyl, Phenylcarbonyl, Pyridylaminocarbonyl oder Pyridylcarbonyl bedeutet,
wobei Phenylaminocarbonyl, Phenylcarbonyl, Pyridylaminocarbonyl und Pyridylcarbonyl substituiert sein können mit 0, 1, 2 oder 3 Substituenten unabhängig voneinander ausgewählt aus der Gruppe bestehend aus Pyrrolidinyl, Piperidinyl, Tetrahydropyranyl, Piperazinyl, Morpholinyl, 2-Oxo-pyrrolidinyl und 2-Oxopiperidinyl,
oder eines ihrer Salze, ihrer Solvate oder der Solvate ihrer Salze.

5. Verfahren zur Herstellung einer Verbindung der Formel (I) nach Anspruch 1, **dadurch gekennzeichnet, dass** eine Verbindung der Formel 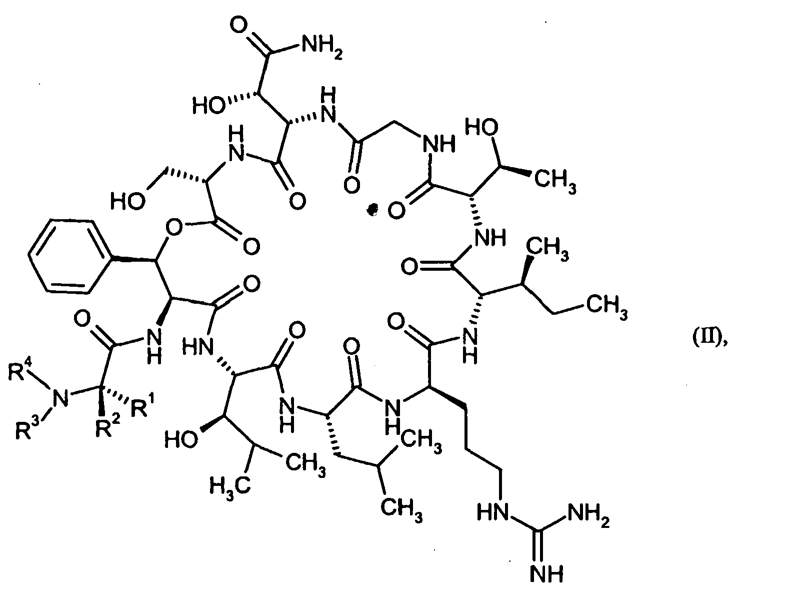 in welcher
R¹, R², R³ und R⁴ die in Anspruch 1 angegebene Bedeutung haben,
mit 1 bis 10 Äquivalenten eines Aryl- oder Heteroarylcarbonsäurechlorides, eines Aryl- oder Heteroarylisocyanates, eines Aryl- oder Heteroarylthio-carbonsäurechlorides, eines Aryl- oder Heteroarylisothiocyanates oder eines Arylsulfonyl-isocyanates, wobei die Aryl- und Heteroaryl-Reste den Aryl- und Heteroaryl-Resten in den Resten R⁵, R⁶ und R⁷ entsprechen, die die in Anspruch 1 angegebene Bedeutung haben,
umgesetzt wird,
und anschließend das entstehende Gemisch von Verbindungen der Formel (I) durch Chromatographie in die Einzelverbindungen der Formel (I) getrennt wird.

6. Verbindung nach einem der Ansprüche 1 bis 4 zur Behandlung und/oder Prophylaxe von Krankheiten.

7. Verwendung einer Verbindung nach einem der Ansprüche 1 bis 4 zur Herstellung eines Arzneimittels zur Behandlung und/oder Prophylaxe von bakteriellen Infektionen.

8. Arzneimittel enthaltend eine Verbindung nach einem der Ansprüche 1 bis 4 in Kombination mit einem inerten, nichttoxischen, pharmazeutisch geeigneten Hilfsstoff.

9. Arzneimittel nach Anspruch 8 zur Behandlung und/oder Prophylaxe von bakteriellen Infektionen.

10. Verwendung einer antibakteriell wirksamen Menge mindestens einer Verbindung nach einem der Ansprüche 1 bis 4, eines Arzneimittels nach Anspruch 8 oder 9 oder eines nach Anspruch 7 erhaltenen Arzneimittels zur Herstellung eines Medikaments zur Bekämpfung von bakteriellen Infektionen in Menschen und Tieren.

## Claims

1. Compound of formula 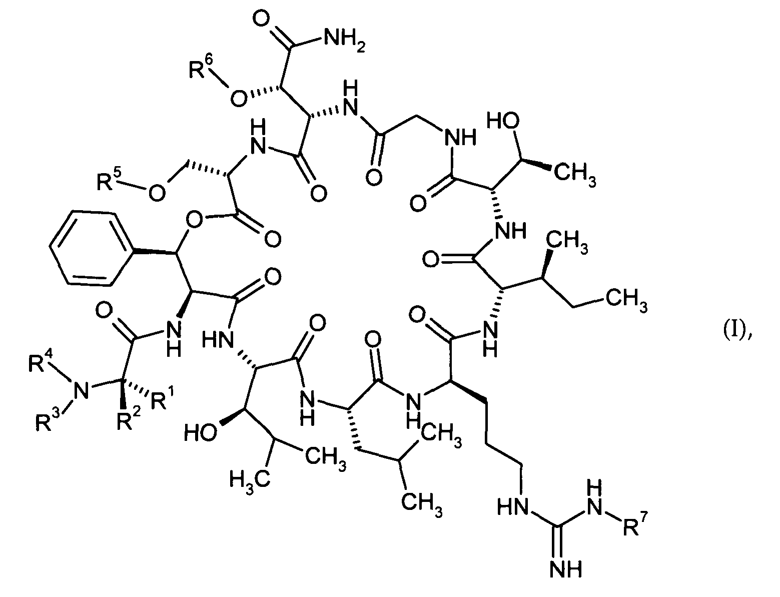 in which
R¹ represents hydrogen, C₁-C₆-alkyl, C₂-C₆-alkenyl, C₃-C₆-cycloalkyl or C₆C₁₀-aryl,
whereby alkyl, alkenyl, cycloalkyl and aryl can be substituted with 0, 1, 2 or 3 substituents selected independently of one another from the group consisting of halogen, hydroxy, amino, cyano, trimethylsilyl, C₁-C₆-alkyl, C₁-C₆-alkoxy, benzyloxy, C₃-C₆-cycloalkyl, C₆-C₁₀-aryl, 5- to 7-membered heterocyclyl, 5- to 10-membered heteroaryl, C₁-C₆-alkylamino, C₆-C₁₀-arylamino, C₁-C₆-alkylcarbonylamino, C₆-C₁₀-arylcarbonylamino, C₁-C₆-alkylcarbonyl, C₁-C₆-alkoxycarbonyl, C₆-C₁₀-arylcarbonyl and benzyloxycarbonylamino,
wherein cycloalkyl, aryl, heterocyclyl and heteroaryl for their part can be substituted with 0, 1, 2 or 3 substituents selected independently of one another from the group consisting of halogen, hydroxy, amino, cyano, nitro, trifluoromethyl, C₁-C₆-alkyl, C₁-C₆-alkoxy, phenyl and 5- to 7-membered heterocyclyl,
R² represents hydrogen or C₁-C₄-alkyl,
R³ represents C₁-C₆-alkyl, C₃-C₆-cycloalkyl, 5- to 7-membered heterocyclyl, C₆-C₁₀-aryl, 5- or 6-membered heteroaryl, C₁-C₆-alkylcarbonyl, C₁-C₆-alkoxycarbonyl, C₃-C₆-cycloalkylcarbonyl, 5- to 7-membered heterocyclylcarbonyl, C₆-C₁₀-arylcarbonyl, 5- or 6-membered heteroarylcarbonyl or C₁-C₆-alkylaminocarbonyl,
whereby alkyl, cycloalkyl, heterocyclyl, aryl, heteroaryl, alkoxycarbonyl, cycloalkylcarbonyl, heterocyclylcarbonyl, arylcarbonyl, heteroarylcarbonyl and alkylaminocarbonyl can be substituted with 0, 1, 2 or 3 substituents selected independently of one another from the group consisting of halogen, hydroxy, amino, C₁-C₆-alkylamino and phenyl,
and
whereby alkylcarbonyl is substituted with a substituent amino or C₁-C₆-alkylamino,
and
whereby alkylcarbonyl can be substituted with a further 0, 1 or 2 substituents selected independently of one another from the group consisting of halogen, hydroxy, trimethylsilyl, C₁-C₆-alkoxy, C₁-C₆-alkylthio, benzyloxy, C₃-C₆-cycloalkyl, phenyl, naphthyl, 5- to 10-membered heteroaryl, C₁-C₆-alkylcarbonylamino, C₁-C₆-alkoxycarbonylamino, C₆-C₁₀-arylcarbonylamino, C₆-C₁₀-arylcarbonyloxy, benzyloxycarbonyl and benzyloxycarbonylamino,
wherein phenyl and heteroaryl for their part can be substituted with 0, 1, 2 or 3 substituents selected independently of one another from the group consisting of halogen, hydroxy, nitro, C₁-C₆-alkyl, C₁-C₆-alkoxy and phenyl,
R⁴ represents hydrogen, C₁-C₄-alkyl, cyclopropyl or cyclopropylmethyl,
and
R⁵ represents C₆-C₁₀-arylaminocarbonyl, C₆-C₁₀-arylcarbonyl, C₆-C₁₀-arylaminothiocarbonyl, C₆-C₁₀-arylthiocarbonyl, C₆-C₁₀-arylsulfonylaminocarbonyl, 5- to 10-membered heteroarylaminocarbonyl, 5- to 10-membered heteroarylcarbonyl, 5- to 10-membered heteroarylaminothiocarbonyl or 5- to 10-membered heteroarylthiocarbonyl,
whereby arylaminocarbonyl, arylcarbonyl, arylaminothiocarbonyl, arylthiocarbonyl, arylsulfonylaminocarbonyl, heteroarylaminocarbonyl, heteroarylcarbonyl, heteroarylaminothiocarbonyl and heteroarylthiocarbonyl can be substituted with 0, 1, 2 or 3 substituents selected independently of one another from the group consisting of halogen, C₁-C₆-alkyl, C₁-C₆-alkoxy, C₁-C₆-alkylamino, C₁-C₆-alkoxycarbonyl, C₁-C₆-alkylaminocarbonyl, C₁-C₆-alkylaminosulfonyl, optionally oxo-substituted 5- to 7-membered heterocyclyl and 5- to 10-membered heteroaryl,
R⁶ represents hydrogen,
R⁷ represents hydrogen,
or
R⁵ represents hydrogen,
R⁶ represents C₆-C₁₀-arylaminocarbonyl, C₆-C₁₀-arylcarbonyl, C₆-C₁₀-arylaminothiocarbonyl, C₆-C₁₀-arylthiocarbonyl, C₆-C₁₀-arylsulfonylaminocarbonyl, 5- to 10-membered heteroarylaminocarbonyl, 5- to 10-membered heteroarylcarbonyl, 5- to 10-membered heteroarylaminothiocarbonyl or 5- to 10-membered heteroarylthiocarbonyl,
whereby arylaminocarbonyl, arylcarbonyl, arylaminothiocarbonyl, arylthiocarbonyl, arylsulfonylaminocarbonyl, heteroarylaminocarbonyl, heteroarylcarbonyl, heteroarylaminothiocarbonyl and heteroarylthiocarbonyl can be substituted with 0, 1, 2 or 3 substituents selected independently of one another from the group consisting of halogen, C₁-C₆-alkyl, C₁-C₆-alkoxy, C₁-C₆-alkylamino, C₁-C₆-alkoxycarbonyl, C₁-C₆-alkylaminocarbonyl, C₁-C₆-alkylaminosulfonyl, optionally oxo-substituted 5- to 7-membered heterocyclyl and 5- to 10-membered heteroaryl,
R⁷ represents hydrogen,
or
R⁵ represents hydrogen,
R⁶ represents hydrogen,
R⁷ represents C₆-C₁₀-arylaminocarbonyl, C₆-C₁₀-arylcarbonyl, C₆-C₁₀-arylaminothiocarbonyl, C₆-C₁₀-arylthiocarbonyl, 5- to 10-membered heteroarylaminocarbonyl, 5- to 10-membered heteroarylcarbonyl, 5- to 10-membered heteroarylaminothiocarbonyl or 5- to 10-membered heteroarylthiocarbonyl,
whereby arylaminocarbonyl, arylcarbonyl, arylaminothiocarbonyl, arylthiocarbonyl, heteroarylaminocarbonyl, heteroarylcarbonyl, heteroarylaminothiocarbonyl and heteroarylthiocarbonyl can be substituted with 0, 1, 2 or 3 substituents selected independently of one another from the group consisting of halogen, C₁-C₆-alkyl, C₁-C₆-alkoxy, C₁-C₆-alkylamino, C₁-C₆-alkoxycarbonyl, C₁-C₆-alkylaminocarbonyl, C₁-C₆-alkylaminosulfonyl, optionally oxo-substituted 5- to 7-membered heterocyclyl and 5- to 10-membered heteroaryl,
or
R⁵ and R⁶ are identical, and
represent C₆-C₁₀-arylaminocarbonyl, C₆-C₁₀-arylcarbonyl, C₆-C₁₀-arylaminothiocarbonyl, C₆-C₁₀-arylthiocarbonyl, C₆-C₁₀-arylsulfonylaminocarbonyl, 5- to 10-membered heteroarylaminocarbonyl, 5- to 10-membered heteroarylcarbonyl, 5- to 10-membered heteroarylaminothiocarbonyl or 5- to 10-membered heteroarylthiocarbonyl,
whereby arylaminocarbonyl, arylcarbonyl, arylaminothiocarbonyl, arylthiocarbonyl, arylsulfonylaminocarbonyl, heteroarylaminocarbonyl, heteroarylcarbonyl, heteroarylaminothiocarbonyl and heteroarylthiocarbonyl can be substituted with 0, 1, 2 or 3 substituents selected independently of one another from the group consisting of halogen, C₁-C₆-alkyl, C₁-C₆-alkoxy, C₁-C₆-alkylamino, C₁-C₆-alkoxycarbonyl, C₁-C₆-alkylaminocarbonyl, C₁-C₆-alkylaminosulfonyl, optionally oxo-substituted 5- to 7-membered heterocyclyl and 5- to 10-membered heteroaryl,
R⁷ represents hydrogen,
or one of its salts, its solvates or the solvates of its salts.

2. Compound according to claim 1, **characterized in that**
R¹ represents 2-methylprop-1-yl, 2,2-dimethylprop-1-yl, 2-pyridylmethyl or 3-pyridylmethyl,
whereby 2-pyridylmethyl or 3-pyridylmethyl can be substituted with 0, 1, 2 or 3 substituents selected independently of one another from the group consisting of hydroxy, amino, trifluoromethyl, methyl, methoxy and morpholinyl,
R² represents hydrogen,
R³ represents 1-amino-3-methylbut-1-ylcarbonyl, 1-amino-3,3-dimethylbut-1-ylcarbonyl or 1-amino-2-trimethylsilyleth-1-ylcarbonyl,
R⁴ represents hydrogen,
and
R⁵ represents C₆-C₁₀-arylaminocarbonyl, C₆-C₁₀-arylcarbonyl, C₆-C₁₀-arylaminothiocarbonyl, C₆-C₁₀-arylthiocarbonyl, C₆-C₁₀-arylsulfonylaminocarbonyl, 5- to 10-membered heteroarylaminocarbonyl, 5- to 10-membered heteroarylcarbonyl, 5- to 10-membered heteroarylaminothiocarbonyl or 5- to 10-membered heteroarylthiocarbonyl,
whereby arylaminocarbonyl, arylcarbonyl, arylaminothiocarbonyl, arylthiocarbonyl, arylsulfonylaminocarbonyl, heteroarylaminocarbonyl, heteroarylcarbonyl, heteroarylaminothiocarbonyl and heteroarylthiocarbonyl can be substituted with 0, 1, 2 or 3 substituents selected independently of one another from the group consisting of halogen, C₁-C₆-alkyl, C₁-C₆-alkoxy, C₁-C₆-alkylamino, C₁-C₆-alkoxycarbonyl, C₁-C₆-alkylaminocarbonyl, C₁-C₆-alkylaminosulfonyl, optionally oxo-substituted 5- to 7-membered heterocyclyl and 5- to 10-membered heteroaryl,
R⁶ represents hydrogen,
R⁷ represents hydrogen,
or
R⁵ represents hydrogen,
R⁶ represents C₆-C₁₀-arylaminocarbonyl, C₆-C₁₀-arylcarbonyl, C₆-C₁₀-arylaminothiocarbonyl, C₆-C₁₀-arylthiocarbonyl, C₆-C₁₀-arylsulfonylaminocarbonyl, 5- to 10-membered heteroarylaminocarbonyl, 5- to 10-membered heteroarylcarbonyl, 5- to 10-membered heteroarylaminothiocarbonyl or 5- to 10-membered heteroarylthiocarbonyl,
whereby arylaminocarbonyl, arylcarbonyl, arylaminothiocarbonyl, arylthiocarbonyl, arylsulfonylaminocarbonyl, heteroarylaminocarbonyl, heteroarylcarbonyl, heteroarylaminothiocarbonyl and heteroarylthiocarbonyl can be substituted with 0, 1, 2 or 3 substituents selected independently of one another from the group consisting of halogen, C₁-C₆-alkyl, C₁-C₆-alkoxy, C₁-C₆-alkylamino, C₁-C₆-alkoxycarbonyl, C₁-C₆-alkylaminocarbonyl, C₁-C₆-alkylaminosulfonyl, optionally oxo-substituted 5- to 7-membered heterocyclyl and 5- to 10-membered heteroaryl,
R⁷ represents hydrogen,
or
R⁵ represents hydrogen,
R⁶ represents hydrogen,
R⁷ represents C₆-C₁₀-arylaminocarbonyl, C₆-C₁₀-arylcarbonyl, C₆-C₁₀-arylaminothiocarbonyl, C₆-C₁₀-arylthiocarbonyl, 5- to 10-membered heteroarylaminocarbonyl, 5- to 10-membered heteroarylcarbonyl, 5- to 10-membered heteroarylaminothiocarbonyl or 5- to 10-membered heteroarylthiocarbonyl,
whereby arylaminocarbonyl, arylcarbonyl, arylaminothiocarbonyl, arylthiocarbonyl, heteroarylaminocarbonyl, heteroarylcarbonyl, heteroarylaminothiocarbonyl and heteroarylthiocarbonyl can be substituted with 0, 1, 2 or 3 substituents selected independently of one another from the group consisting of halogen, C₁-C₆-alkyl, C₁-C₆-alkoxy, C₁-C₆-alkylamino, C₁-C₆-alkoxycarbonyl, C₁-C₆-alkylaminocarbonyl, C₁-C₆-alkylaminosulfonyl, optionally oxo-substituted 5- to 7-membered heterocyclyl and 5- to 10-membered heteroaryl,
or
R⁵ and R⁶ are identical, and
represent C₆-C₁₀-arylaminocarbonyl, C₆-C₁₀-arylcarbonyl, C₆-C₁₀-arylaminothiocarbonyl, C₆-C₁₀-arylthiocarbonyl, C₆-C₁₀-arylsulfonylaminocarbonyl, 5- to 10-membered heteroarylaminocarbonyl, 5- to 10-membered heteroarylcarbonyl, 5- to 10-membered heteroarylaminothiocarbonyl or 5- to 10-membered heteroarylthiocarbonyl,
whereby arylaminocarbonyl, arylcarbonyl, arylaminothiocarbonyl, arylthiocarbonyl, arylsulfonylaminocarbonyl, heteroarylaminocarbonyl, heteroarylcarbonyl, heteroarylaminothiocarbonyl and heteroarylthiocarbonyl can be substituted with 0, 1, 2 or 3 substituents selected independently of one another from the group consisting of halogen, C₁-C₆-alkyl, C₁-C₆-alkoxy, C₁-C₆-alkylamino, C₁-C₆-alkoxycarbonyl, C₁-C₆-alkylaminocarbonyl, C₁-C₆-alkylaminosulfonyl, optionally oxo-substituted 5- to 7-membered heterocyclyl and 5- to 10-membered heteroaryl,
R⁷ represents hydrogen,
or one of its salts, its solvates or the solvates of its salts.

3. Compound according to one of claims 1 or 2, **characterized in that**
R¹ represents 2-methylprop-1-yl,
R² represents hydrogen,
R³ represents 1-amino-3-methylbut-1-ylcarbonyl,
R⁴ represents hydrogen,
and
R⁵ represents hydrogen,
R⁶ represents C₆-C₁₀-arylaminocarbonyl, C₆-C₁₀-arylcarbonyl, C₆-C₁₀-arylaminothiocarbonyl, C₆-C₁₀-arylthiocarbonyl, C₆-C₁₀-arylsulfonylaminocarbonyl, 5- to 10-membered heteroarylaminocarbonyl, 5- to 10-membered heteroarylcarbonyl, 5- to 10-membered heteroarylaminothiocarbonyl or 5- to 10-membered heteroarylthiocarbonyl,
whereby arylaminocarbonyl, arylcarbonyl, arylaminothiocarbonyl, arylthiocarbonyl, arylsulfonylaminocarbonyl, heteroarylaminocarbonyl, heteroarylcarbonyl, heteroarylaminothiocarbonyl and heteroarylthiocarbonyl can be substituted with 0, 1, 2 or 3 substituents selected independently of one another from the group consisting of halogen, C₁-C₆-alkyl, C₁-C₆-alkoxy, C₁-C₆-alkylamino, C₁-C₆-alkoxycarbonyl, C₁-C₆-alkylaminocarbonyl, C₁-C₆-alkylaminosulfonyl, optionally oxo-substituted 5- to 7-membered heterocyclyl and 5- to 10-membered heteroaryl,
R⁷ represents hydrogen,
or
R⁵ represents hydrogen,
R⁶ represents hydrogen,
R⁷ represents C₆-C₁₀-arylaminocarbonyl, C₆-C₁₀-arylcarbonyl, C₆-C₁₀-arylaminothiocarbonyl, C₆-C₁₀-arylthiocarbonyl, 5- to 10-membered heteroarylaminocarbonyl, 5- to 10-membered heteroarylcarbonyl, 5- to 10-membered heteroarylaminothiocarbonyl or 5- to 10-membered heteroarylthiocarbonyl,
whereby arylaminocarbonyl, arylcarbonyl, arylaminothiocarbonyl, arylthiocarbonyl, heteroarylaminocarbonyl, heteroarylcarbonyl, heteroarylaminothiocarbonyl and heteroarylthiocarbonyl can be substituted with 0, 1, 2 or 3 substituents selected independently of one another from the group consisting of halogen, C₁-C₆-alkyl, C₁-C₆-alkoxy, C₁-C₆-alkylamino, C₁-C₆-alkoxycarbonyl, C₁-C₆-alkylaminocarbonyl, C₁-C₆-alkylaminosulfonyl, optionally oxo-substituted 5- to 7-membered heterocyclyl and 5- to 10-membered heteroaryl,
or one of its salts, its solvates or the solvates of its salts.

4. Compound according to one of claims 1 to 3, **characterized in that**
R¹ represents 2-methylprop-1-yl,
R² represents hydrogen,
R³ represents 1-amino-3-methylbut-1-ylcarbonyl,
R⁴ represents hydrogen,
and
R⁵ represents hydrogen,
R⁶ represents phenylaminocarbonyl, phenylcarbonyl, pyridylaminocarbonyl or pyridylcarbonyl,
whereby phenylaminocarbonyl, phenylcarbonyl, pyridylaminocarbonyl and pyridylcarbonyl can be substituted with 0, 1, 2 or 3 substituents selected independently of one another from the group consisting of pyrrolidinyl, piperidinyl, tetrahydropyranyl, piperazinyl, morpholinyl, 2-oxopyrrolidinyl and 2-oxopiperidinyl,
R⁷ represents hydrogen,
or
R⁵ represents hydrogen,
R⁶ represents hydrogen,
R⁷ represents phenylaminocarbonyl, phenylcarbonyl, pyridylaminocarbonyl or pyridylcarbonyl,
whereby phenylaminocarbonyl, phenylcarbonyl, pyridylaminocarbonyl and pyridylcarbonyl can be substituted with 0, 1, 2 or 3 substituents selected independently of one another from the group consisting of pyrrolidinyl, piperidinyl, tetrahydropyranyl, piperazinyl, morpholinyl, 2-oxopyrrolidinyl and 2-oxopiperidinyl,
or one of its salts, its solvates or the solvates of its salts.

5. Method for preparing a compound of formula (I) according to claim 1, **characterized in that** a compound of formula 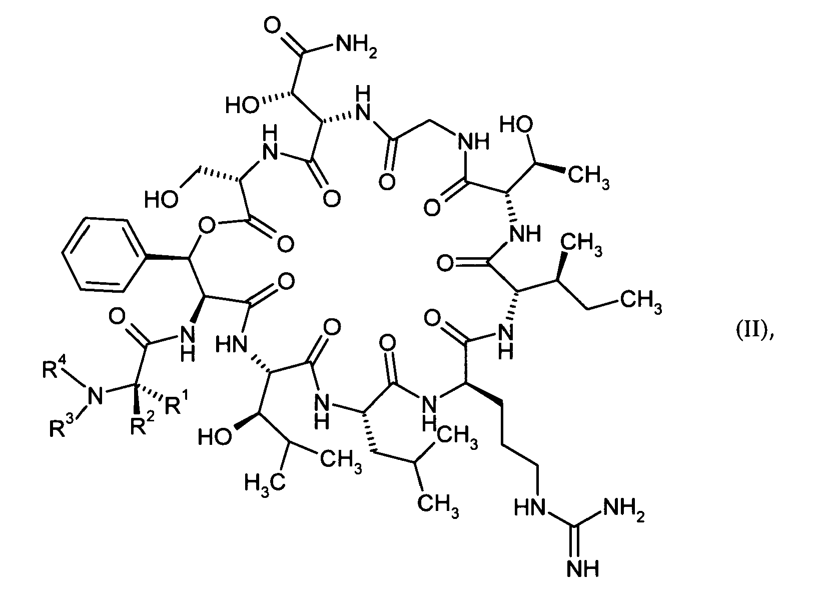 in which
R¹, R², R³ and R⁴ have the meaning indicated in claim 1,
is reacted with 1 to 10 equivalents of an aryl- or heteroarylcarbonyl chloride, of an aryl or heteroaryl isocyanate, of an aryl- or heteroarylthiocarbonyl chloride, of an aryl or heteroaryl isothiocyanate or of an arylsulfonyl isocyanate, whereby the aryl and heteroaryl radicals correspond to the aryl and heteroaryl radicals in the radicals R⁵, R⁶ and R⁷, which have the meaning indicated in claim 1,
and subsequently the resulting mixture of compounds of formula (I) is separated by chromatography into the individual compounds of formula (I).

6. Compound according to one of claims 1 to 4 for the treatment and/or prophylaxis of diseases.

7. Use of a compound according to one of claims 1 to 4 for the production of a medicament for the treatment and/or prophylaxis of bacterial infections.

8. Medicament comprising a compound according to one of claims 1 to 4 in combination with an inert, non-toxic, pharmaceutically suitable excipient.

9. Medicament according to claim 8 for the treatment and/or prophylaxis of bacterial infections.

10. Use of an antibacterially effective amount of at least one compound according to one of claims 1 to 4, of a medicament according to claim 8 or 9 or of a medicament obtained according to claim 7 for the production of a medicament for controlling bacterial infections in humans and animals.

## Revendications

1. Composé de la formule 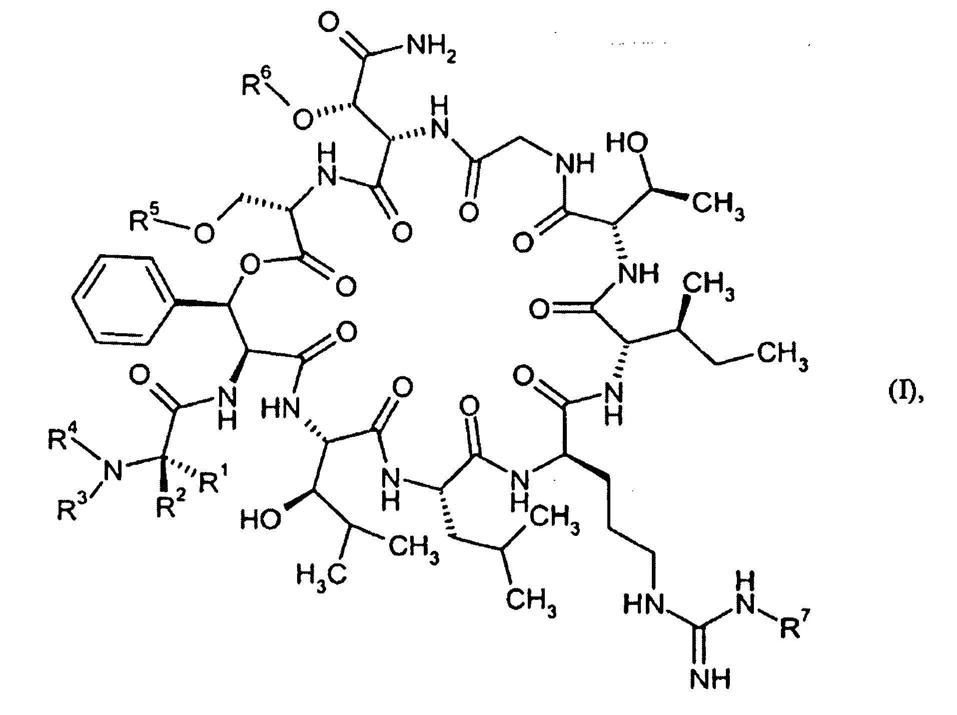 dans laquelle
R¹ signifie un hydrogène, un alkyle en C₁ à C₆, un alcényle en C₂ à C₆, un cycloalkyle en C₃ à C₆ ou un aryle en C₆ à C₁₀,
sachant que l'alkyle, l'alcényle, le cycloalkyle et l'aryle peuvent être substitués par 0, 1, 2 ou 3 substituants choisis indépendamment l'un de l'autre parmi le groupe consistant en un halogène, un hydroxyle, un amino, un cyano, un triméthylsilyle, un alkyle en C₁-C₆, un alcoxyle en C₁-C₆, un benzyloxyle, un cycloalkyle en C₃ à C₆, un aryle en C₆ à C₁₀, un hétérocyclyle à 5 à 7 atomes dans le cycle, un hétéroaryle à 5 à 10 atomes dans le cycle, un alkylamino en C₁ à C₆, un arylamino en C₆ à C₁₀, un alkylcarbonylamino en C₁ à C₆, un arylcarbonylamino en C₆ à C₁₀, un alkylcarbonyle en C₁ à C₆, un alcoxycarbonyle en C₁ à C₆, un arylcarbonyle en C₆ à C₁₀ et un benzyloxycarbonylamino,
où le cycloalkyle, l'aryle, l'hétérocyclyle et l'hétéroaryle peuvent être substitués de leur côté par 0, 1, 2 ou 3 substituants choisis indépendamment l'un de l'autre parmi le groupe consistant en un halogène, un hydroxyle, un amino, un cyano, un nitro, un trifluorométhyle, un alkyle en C₁à C₆, un alcoxyle en C₁ à C₆, un phényle et un hétérocyclyle à 5 à 7 atomes dans le cycle,
R² signifie un hydrogène ou un alkyle en C₁ à C₄,
R³ signifie un alkyle en C₁ à C₆, un cycloalkyle en C₃ à C₆, hétérocyclyle à 5 à 7 atomes dans le cycle, un aryle en C₆ à C₁₀, un hétéroaryle à 5 ou 6 atomes dans le cycle, un alkylcarbonyle en C₁ à C₆, un alcoxycarbonyle en C₁ à C₆, un cycloalkylcarbonyle en C₃ à C₆, un hétérocyclylcarbonyle à 5 à 7 atomes dans le cycle, un arylcarbonyle en C₆ à C₁₀, un hétéroarylcarbonyle à 5 ou 6 atomes dans le cycle ou un alkylaminocarbonyle en C₁ à C₆,
sachant que l'alkyle, le cycloalkyle, l'hétérocyclyle, l'aryle, l'hétéroaryle, l'alcoxycarbonyle, le cycloalkylcarbonyle, l'hétérocyclylcarbonyle, l'arylcarbonyle, l'hétéroarylcarbonyle et l'alkylaminocarbonyle peuvent être substitués par 0, 1, 2 ou 3 substituants choisis indépendamment l'un de l'autre parmi le groupe consistant en un halogène, un hydroxyle, un amino, un alkylamino en C₁ à C₆ et un phényle,
et
sachant que l'alkylcarbonyle est substitué par un substituant amino ou alkylamino en C₁ à C₆,
et
sachant que l'alkylcarbonyle peut être substitué par 0, 1 ou 2 autres substituants choisis indépendamment l'un de l'autre parmi le groupe consistant en un halogène, un hydroxyle, un triméthylsilyle, un alcoxyle en C₁ à C₆, un alkylthio en C₁ à C₆, un benzyloxyle, un cycloalkyle en C₃ à C₆, un phényle, un naphthyle, un hétéroaryle à 5 à 10 atomes dans le cycle, un alkylcarbonylamino en C₁ à C₆, un alcoxylcarbonylamino en C₁ à C₆, un arylcarbonylamino en C₆ à C₁₀, un arylcarbonyloxyle en C₆ à C₁₀, un benzyloxycarbonyle et un benzyloxycarbonylamino,
où le phényle et l'hétéroaryle peuvent être substitués de leur côté par 0, 1, 2 ou 3 substituants choisis indépendamment l'un de l'autre parmi le groupe consistant en un halogène, un hydroxyle, un nitro, un alkyle en C₁ à C₆, un alcoxyle en C₁ à C₆ et un phényle,
R⁴ signifie un hydrogène, un alkyle en C₁ à C₄, un cyclopropyle ou un cyclopropylméthyle,
et
R⁵ signifie un arylaminocarbonyle en C₆ à C₁₀, un arylcarbonyle en C₆ à C₁₀, un arylaminothiocarbonyle en C₆ à C₁₀, un arylthiocarbonyle en C₆ à C₁₀, un arylsulfonylaminocarbonyle en C₆ à C₁₀, un hétéroarylaminocarbonyle à 5 à 10 atomes dans le cycle, un hétéroarylcarbonyle à 5 à 10 atomes dans le cycle, un hétéroarylaminothiocarbonyle à 5 à 10 atomes dans le cycle ou un hétéroarylthiocarbonyle à 5 à 10 atomes dans le cycle,
sachant que l'arylaminocarbonyle, l'arylcarbonyle, l'arylaminothiocarbonyle, l'arylthiocarbonyle, l'arylsulfonylaminocarbonyle, l'hétéroarylaminocarbonyle, l'hétéroarylcarbonyle, l'hétéroarylaminothiocarbonyle et l'hétéroarylthiocarbonyle peuvent être substitués par 0, 1, 2 ou 3 substituants choisis indépendamment l'un de l'autre parmi le groupe consistant en un halogène, un alkyle en C₁ à C₆, un alcoxyle en C₁ à C₆, un alkylamino en C₁ à C₆, un alcoxycarbonyle en C₁ à C₆, un alkylaminocarbonyle en C₁ à C₆, un alkylaminosulfonyle en C₁ à C₆, un hétérocyclyle à 5 à 7 atomes dans le cycle, le cas échéant substitués par oxo, et un hétéroaryle à 5 à 10 atomes dans le cycle,
R⁶ signifie un hydrogène,
R⁷ signifie un hydrogène,
ou
R⁵ signifie un hydrogène,
R⁶ signifie un arylaminocarbonyle en C₆ à C₁₀, un arylcarbonyle en C₆ à C₁₀, un arylaminothiocarbonyle en C₆ à C₁₀, un arylthiocarbonyle en C₆ à C₁₀, un arylsulfonylaminocarbonyle en C₆ à C₁₀, un hétéroarylaminocarbonyle à 5 à 10 atomes dans le cycle, un hétéroarylcarbonyle à 5 à 10 atomes dans le cycle, un hétéroarylaminothiocarbonyle à 5 à 10 atomes dans le cycle ou un hétéroarylthiocarbonyle à 5 à 10 atomes dans le cycle,
sachant que l'arylaminocarbonyle, l'arylcarbonyle, l'arylaminothiocarbonyle, l'arylthiocarbonyle, l'arylsulfonylaminocarbonyle, l'hétéroarylaminocarbonyle, l'hétéroarylcarbonyle, l'hétéroarylaminothiocarbonyle et l'hétéroarylthiocarbonyle peuvent être substitués par 0, 1, 2 ou 3 substituants choisis indépendamment l'un de l'autre parmi le groupe consistant en un halogène, un alkyle en C₁ à C₆, un alcoxyle en C₁ à C₆, un alkylamino en C₁ à C₆, un alcoxycarbonyle en C₁ à C₆, un alkylaminocarbonyle en C₁ à C₆, un alkylaminosulfonyle en C₁ à C₆, un hétérocyclyle à 5 à 7 atomes dans le cycle, le cas échéant substitué par oxo, et un hétéroaryle à 5 à 10 atomes dans le cycle,
R⁷ signifie un hydrogène,
ou
R⁵ signifie un hydrogène,
R⁶ signifie un hydrogène,
R⁷ signifie un arylaminocarbonyle en C₆ à C₁₀, arylcarbonyle en C₆ à C₁₀, un arylaminothiocarbonyle en C₆ à C₁₀, un arylthiocarbonyle en C₆ à C₁₀, un hétéroarylaminocarbonyle à 5 à 10 atomes dans le cycle, un hétéroarylcarbonyle à 5 à 10 atomes dans le cycle, un hétéroarylaminothiocarbonyle à 5 à 10 atomes dans le cycle ou un hétéroarylthiocarbonyle à 5 à 10 atomes dans le cycle,
sachant que l'arylaminocarbonyle, l'arylcarbonyle, l'arylaminothiocarbonyle, l'arylthiocarbonyle, l'hétéroarylaminocarbonyle, l'hétéroarylcarbonyle, l'hétéroarylaminothiocarbonyle et l'hétéroarylthiocarbonyle peuvent être substitués par 0, 1, 2 ou 3 substituants choisis indépendamment l'un de l'autre parmi le groupe consistant en un halogène, un alkyle en C₁ à C₆, un alcoxyle en C₁ à C₆, un alkylamino en C₁ à C₆, un alcoxycarbonyle en C₁ à C₆, un alkylaminocarbonyle en C₁ à C₆, un alkylaminosulfonyle en C₁ à C₆, un hétérocyclyle à 5 à 7 atomes dans le cycle, le cas échéant substitué par oxo, et un hétéroaryle à 5 à 10 atomes dans le cycle,
ou
R⁵ et R⁶ sont identiques et
signifient un arylaminocarbonyle en C₆ à C₁₀, un arylcarbonyle en C₆ à C₁₀, un arylaminothiocarbonyle en C₆ à C₁₀, un arylthiocarbonyle en C₆ à C₁₀, un arylsulfonylaminocarbonyle en C₆ à C₁₀, un hétéroarylaminocarbonyle à 5 à 10 atomes dans le cycle, un hétéroarylcarbonyle à 5 à 10 atomes dans le cycle, un hétéroarylaminothiocarbonyle à 5 à 10 atomes dans le cycle ou un hétéroarylthiocarbonyle à 5 à 10 atomes dans le cycle,
sachant que l'arylaminocarbonyle, l'arylcarbonyle, l'arylaminothiocarbonyle, l'arylthiocarbonyle, l'arylsulfonylaminocarbonyle, l'hétéroarylaminocarbonyle, l'hétéroarylcarbonyle, l'hétéroarylaminothiocarbonyle et l'hétéroarylthiocarbonyle peuvent être substitués par 0, 1, 2 ou 3 substituants choisis indépendamment l'un de l'autre parmi le groupe consistant en un halogène, un alkyle en C₁ à C₆, un alcoxyle en C₁ à C₆, un alkylamino en C₁ à C₆, un alcoxycarbonyle en C₁ à C₆, un alkylaminocarbonyle en C₁ à C₆, un alkylaminosulfonyle en C₁ à C₆, un hétérocyclyle à 5 à 7 atomes dans le cycle, le cas échéant substitué par oxo, et un hétéroaryle à 5 à 10 atomes dans le cycle,
R⁷ signifie un hydrogène,
ou un de leurs sels, de leurs solvates ou des solvates de leurs sels.

2. Composé selon la revendication 1, **caractérisé en ce que**
R¹ signifie un 2-méthylprop-1-yle, un 2,2-diméthylprop-1-yle, un 2-pyridylméthyle ou un 3-pyridylméthyle,
sachant que le 2-pyridylméthyle ou le 3-pyridylméthyle peuvent être substitués par 0, 1, 2 ou 3 substituants choisis indépendamment l'un de l'autre parmi le groupe consistant en un hydroxyle, un amino, un trifluorométhyle, un méthyle, un méthoxyle et un morpholinyle,
R² signifie un hydrogène,
R³ signifie un 1-amino-3-méthylbut-1-ylcarbonyle, un 1-amino-3,3-diméthylbut-1-ylcarbonyle ou un 1-amino-2-triméthylsilyléth-1-ylcarbonyle,
R⁴ signifie un hydrogène,
et
R⁵ signifie un arylaminocarbonyle en C₆ à C₁₀, un arylcarbonyle en C₆ à C₁₀, un arylaminothiocarbonyle en C₆ à C₁₀, un arylthiocarbonyle en C₆ à C₁₀, un arylsulfonylaminocarbonyle en C₆ à C₁₀, un hétéroarylaminocarbonyle à 5 à 10 atomes dans le cycle, un hétéroarylcarbonyle à 5 à 10 atomes dans le cycle, un hétéroarylaminothiocarbonyle à 5 à 10 atomes dans le cycle ou un hétéroarylthiocarbonyle à 5 à 10 atomes dans le cycle,
sachant que l'arylaminocarbonyle, l'arylcarbonyle, l'arylaminothiocarbonyle, l'arylthiocarbonyle, l'arylsulfonylaminocarbonyle, l'hétéroarylaminocarbonyle, l'hétéroarylcarbonyle, l'hétéroarylaminothiocarbonyle et l'hétéroarylthiocarbonyle peuvent être substitués par 0, 1, 2 ou 3 substituants choisis indépendamment l'un de l'autre parmi le groupe consistant en un halogène, un alkyle en C₁ à C₆, un alcoxyle en C₁ à C₆, un alkylamino en C₁ à C₆, un alcoxycarbonyle en C₁ à C₆, un alkylaminocarbonyle en C₁ à C₆, un alkylaminosulfonyle en C₁ à C₆, un hétérocyclyle à 5 à 7 atomes dans le cycle, le cas échéant substitué par oxo, et un hétéroaryle à 5 à 10 atomes dans le cycle,
R⁶ signifie un hydrogène,
R² signifie un hydrogène,
ou
R⁵ signifie un hydrogène,
R⁶ signifie un arylaminocarbonyle en C₆ à C₁₀, un arylcarbonyle en C₆ à C₁₀, un arylaminothiocarbonyle en C₆ à C₁₀, un arylthiocarbonyle en C₆ à C₁₀, un arylsulfonylaminocarbonyle en C₆ à C₁₀, un hétéroarylaminocarbonyle à 5 à 10 atomes dans le cycle, un hétéroarylcarbonyle à 5 à 10 atomes dans le cycle, un hétéroarylaminothiocarbonyle à 5 à 10 atomes dans le cycle ou un hétéroarylthiocarbonyle à 5 à 10 atomes dans le cycle,
sachant que l'arylaminocarbonyle, l'arylcarbonyle, l'arylaminothiocarbonyle, l'arylthiocarbonyle, l'arylsulfonylaminocarbonyle, l'hétéroarylaminocarbonyle, l'hétéroarylcarbonyle, l'hétéroarylaminothiocarbonyle et l'hétéroarylthiocarbonyle peuvent être substitués par 0, 1, 2 ou 3 substituants choisis indépendamment l'un de l'autre parmi le groupe consistant en un halogène, un alkyle en C₁ à C₆, un alcoxyle en C₁ à C₆, un alkylamino en C₁ à C₆, alcoxycarbonyle en C₁ à C₆, un alkylaminocarbonyle en C₁ à C₆, un alkylaminosulfonyle en C₁ à C₆, un hétérocyclyle à 5 à 7 atomes dans le cycle, le cas échéant substitué par oxo, et un hétéroaryle à 5 à 10 atomes dans le cycle,
R⁷ signifie un hydrogène,
ou
R⁵ signifie un hydrogène,
R⁶ signifie un hydrogène,
R⁷ signifie un arylaminocarbonyle en C₆ à C₁₀, un arylcarbonyle en C₆ à C₁₀, un arylaminothiocarbonyle en C₆ à C₁₀, un arylthiocarbonyle en C₆ à C₁₀, un hétéroarylaminocarbonyle à 5 à 10 atomes dans le cycle, un hétéroarylcarbonyle à 5 à 10 atomes dans le cycle, un hétéroaryleaminothiocarbonyle à 5 à 10 atomes dans le cycle ou un hétéroarylthiocarbonyle à 5 à 10 atomes dans le cycle,
sachant que l'arylaminocarbonyle, l'arylcarbonyle, l'arylaminothiocarbonyle, l'arylthiocarbonyle, l'hétéroarylaminocarbonyle, l'hétéroarylcarbonyle, l'hétéroarylaminothiocarbonyle et l'hétéroarylthiocarbonyle peuvent être substitués par 0, 1, 2 ou 3 substituants choisis indépendamment l'un de l'autre parmi le groupe consistant en un halogène, un alkyle en C₁ à C₆, a un alcoxyle en C₁ à C₆, un alkylamino en C₁ à C₆, un alcoxylcarbonyle en C₁ à C₆, un alkylaminocarbonyle en C₁ à C₆, un alkylaminosulfonyle en C₁ à C₆, un hétérocyclyle à 5 à 7 atomes dans le cycle, le cas échéant substitué par oxo, et un hétéroaryle à 5 à 10 atomes dans le cycle,
ou
R⁵ et R⁶ sont identiques, et
signifient un arylaminocarbonyle en C₆ à C₁₀, un arylcarbonyle en C₆ à C₁₀, un arylaminothiocarbonyle en C₆ à C₁₀, un arylthiocarbonyle en C₆ à C₁₀, un arylsulfonylaminocarbonyle en C₆ à C₁₀, un hétéroarylaminocarbonyle à 5 à 10 atomes dans le cycle, un hétéroarylcarbonyle à 5 à 10 atomes dans le cycle, un hétéroarylaminothiocarbonyle à 5 à 10 atomes dans le cycle ou un hétéroarylthiocarbonyle à 5 à 10 atomes dans le cycle,
sachant que l'arylaminocarbonyle, l'arylcarbonyle, l'arylaminothiocarbonyle, l'arylthiocarbonyle, l'arylsulfonylaminocarbonyle, l'hétéroarylaminocarbonyle, l'hétéroarylcarbonyle, l'hétéroarylaminothiocarbonyle et l'hétéroarylthiocarbonyle peuvent être substitués par 0, 1, 2 ou 3 substituants choisis indépendamment l'un de l'autre parmi le groupe consistant en un halogène, un alkyle en C₁ à C₆, un alcoxyle en C₁ à C₆, un alkylamino en C₁ à C₆, un alcoxycarbonyle en C₁ à C₆, un alkylaminocarbonyle en C₁ à C₆, un alkylaminosulfonyle en C₁ à C₆, un hétérocyclyle à 5 à 7 atomes dans le cycle, le cas échéant substitué par oxo, et un hétéroaryle à 5 à 10 atomes dans le cycle,
R⁷ signifie un hydrogène,
ou un de leurs sels, de leurs solvates ou des solvates de leurs sels.

3. Composé selon l'une quelconque des revendications 1 ou 2, **caractérisé en ce que**
R¹ signifie un 2-méthylprop-1-yle,
R² signifie un hydrogène,
R³ signifie un 1-amino-3-méthylbut-1-ylcarbonyle,
R⁴ signifie un hydrogène,
et
R⁵ signifie un hydrogène,
R⁶ signifie un arylaminocarbonyle en C₆ à C₁₀, un arylcarbonyle en C₆ à C₁₀, un arylaminothiocarbonyle en C₆ à C₁₀, un arylthiocarbonyle en C₆ à C₁₀, un arylsulfonylaminocarbonyle en C₆ à C₁₀, un hétéroarylaminocarbonyle à 5 à 10 atomes dans le cycle, un hétéroarylcarbonyle à 5 à 10 atomes dans le cycle, un hétéroarylaminothiocarbonyle à 5 à 10 atomes dans le cycle ou un hétéroarylthiocarbonyle à 5 à 10 atomes dans le cycle,
sachant que l'arylaminocarbonyle, l'arylcarbonyle, l'arylaminothiocarbonyle, l'arylthiocarbonyle, l'arylsulfonylaminocarbonyle, l'hétéroarylaminocarbonyle, l'hétéroarylcarbonyle, l'hétéroarylaminothiocarbonyle et l'hétéroarylthiocarbonyle peuvent être substitués par 0, 1, 2 ou 3 substituants choisis indépendamment l'un de l'autre parmi le groupe consistant en un halogène, un alkyle en C₁ à C₆, un alcoxyle en C₁ à C₆, un alkylamino en C₁ à C₆, un alcoxycarbonyle en C₁ à C₆, un alkylaminocarbonyle en C₁ à C₆, un alkylaminosulfonyle en C₁ à C₆, un hétérocyclyle à 5 à 7 atomes dans le cycle, le cas échéant substitué par oxo, et un hétéroaryle à 5 à 10 atomes dans le cycle,
R⁷ signifie un hydrogène,
ou
R⁵ signifie un hydrogène,
R⁶ signifie un hydrogène,
R⁷ signifie un arylaminocarbonyle en C₆ à C₁₀, un arylcarbonyle en C₆ à C₁₀, un arylaminothiocarbonyle en C₆ à C₁₀, un arylthiocarbonyle en C₆ à C₁₀, un hétéroarylaminocarbonyle à 5 à 10 atomes dans le cycle, hétéroarylcarbonyle à 5 à 10 atomes dans le cycle, un hétéroarylaminothiocarbonyle à 5 à 10 atomes dans le cycle ou un hétéroarylthiocarbonyle à 5 à 10 atomes dans le cycle,
sachant que l'arylaminocarbonyle, l'arylcarbonyle, l'arylaminothiocarbonyle, l'arylthiocarbonyle, l'hétéroarylaminocarbonyle, l'hétéroarylcarbonyle, l'hétéroarylaminothiocarbonyle et l'hétéroarylthiocarbonyle peuvent être substitués par 0, 1, 2 ou 3 substituants choisis indépendamment l'un de l'autre parmi le groupe consistant en un halogène, un alkyle en C₁ à C₆, un alcoxyle en C₁ à C₆, un alkylamino en C₁ à C₆, un alcoxycarbonyle en C₁ à C₆, un alkylaminocarbonyle en C₁ à C₆, un alkylaminosulfonyle en C₁ à C₆, un hétérocyclyle à 5 à 7 atomes dans le cycle, le cas échéant substitué oxo, et un hétéroaryle à 5 à 10 atomes dans le cycle,
ou un de leurs sels, de leurs solvates ou des solvates de leurs sels.

4. Composé selon l'une quelque des revendications 1 à 3, **caractérisé en ce que**
R¹ signifie un 2-méthylprop-1-yle,
R² signifie un hydrogène,
R³ signifie un 1-amino-3-méthylbut-1-ylcarbonyle,
R⁴ signifie un hydrogène,
et
R⁵ signifie un hydrogène,
R⁶ signifie un phénylaminocarbonyle, un phénylcarbonyle, un pyridylaminocarbonyle ou un pyridylcarbonyle,
sachant que le phénylaminocarbonyle, le phénylcarbonyle, le pyridylaminocarbonyle et le pyridylcarbonyle peuvent être substitués par 0, 1, 2 ou 3 substituants choisis indépendamment l'un de l'autre parmi le groupe consistant en un pyrrolidinyle, un pipéridinyle, un tétrahydropyranyle, un pipérazinyle, un morpholinyle, un 2-oxopyrrolidinyle et un 2-oxopipéridinyle,
R⁷ signifie un hydrogène,
ou
R⁵ signifie un hydrogène,
R⁶ signifie un hydrogène,
R⁷ signifie un phénylaminocarbonyle, un phénylcarbonyle, un pyridylaminocarbonyle ou un pyridylcarbonyle,
sachant que le phénylaminocarbonyle, le phénylcarbonyle, le pyridylaminocarbonyle et le pyridylcarbonyle peuvent être substitués par 0,1,2 ou 3 substituants choisis indépendamment l'un de l'autre parmi le groupe consistant en un pyrrolidinyle, un pipéridinyle, un tétrahydropyranyle, un pipérazinyle, un morpholinyle, un 2-oxopyrrolidinyle et un 2-oxopipéridinyle,
ou un de leurs sels, de leurs solvates ou des solvates de leurs sels.

5. Procédé pour la préparation d'un composé de la formule (I) selon la revendication 1, **caractérisé en ce que** l'on fait réagir un composé de la formule 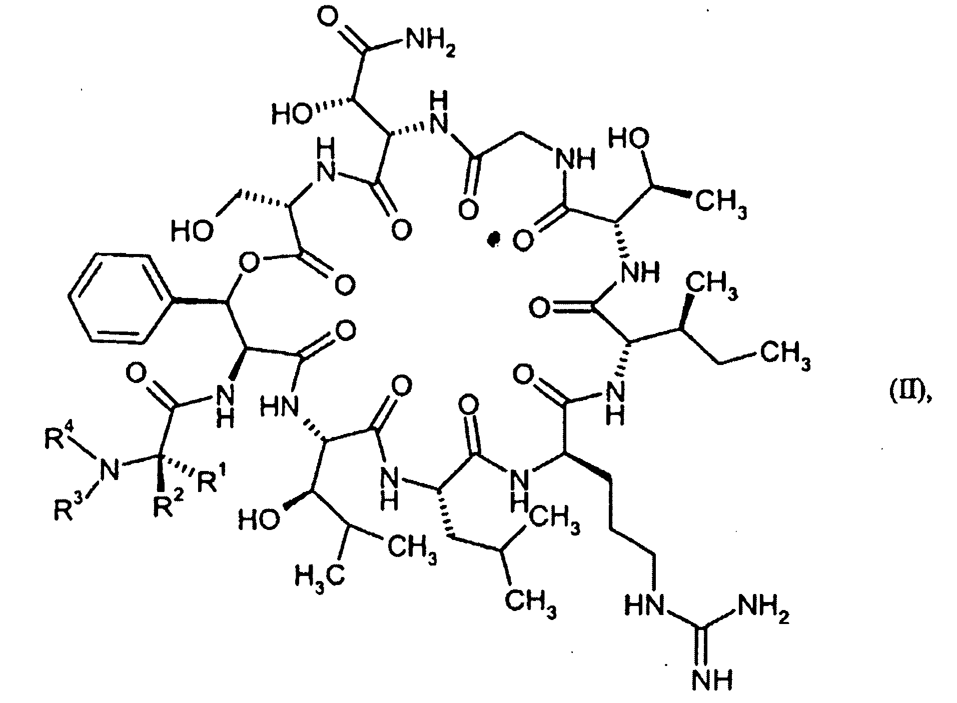 dans laquelle
R¹, R², R³ et R⁴ ont la signification spécifiée dans la revendication 1,
avec 1 à 10 équivalents d'un chlorure d'acide aryl- ou hétéroarylcarboxylique, d'un aryl- ou hétéroarylisocyanate, d'un chlorure d'acide aryl- ou hétéroarylthiocarboxylique, d'un aryl- ou hétéroarylisothiocyanate ou d'un arylsulfonylisocyanate, sachant que les radicaux aryle et hétéroaryle correspondent aux radicaux aryle et hétéroaryle dans les radicaux R⁵, R⁶ et R⁷, qui ont la signification spécifiée dans la revendication 1,
et **en ce qu'**on sépare ensuite le mélange de composés de la formule (I) par chromatographie en les composés individuels de la formule (I).

6. Composé selon l'une quelconque des revendications 1 à 4 pour le traitement et/ou la prophylaxie des maladies.

7. Utilisation un composé selon l'une quelconque des revendications 1 à 4 à la préparation d'un produit pharmaceutique pour le traitement et/ou la prophylaxie d'infections bactériennes.

8. Produit pharmaceutique contenant un composé selon l'une quelconque des revendications 1 à 4 en combinaison avec une substance inerte, non toxique, pharmaceutiquement appropriée.

9. Produit pharmaceutique selon la revendication 8 pour le traitement et/ou la prophylaxie d'infections bactériennes.

10. Utilisation d'une quantité efficace du point de vue antibactérien d'au moins un composé selon l'une quelconque des revendications 1 à 4, d'un produit pharmaceutique selon la revendication 8 ou 9 ou d'un produit pharmaceutique obtenu selon la revendication 7 à la préparation d'un médicament pour la lutte contre les infections bactériennes chez l'homme et l'animal.
